(19) Europäisches Patentamt
European Patent Office
Office européen des brevets

(11) EP 1 536 003 A1

(12) **EUROPEAN PATENT APPLICATION**
published in accordance with Art. 158(3) EPC

(43) Date of publication:
**01.06.2005 Bulletin 2005/22**

(21) Application number: **03733147.7**

(22) Date of filing: **29.05.2003**

(51) Int Cl.[7]: **C12N 15/09**, C12M 1/00, C12Q 1/68, G01N 33/15, G01N 33/50, G01N 33/53, G01N 33/566

(86) International application number:
**PCT/JP2003/006750**

(87) International publication number:
**WO 2003/102181 (11.12.2003 Gazette 2003/50)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **30.05.2002 JP 2002158237**

(71) Applicant: **Riken**
**Wako-shi, Saitama 351-0198 (JP)**

(72) Inventors:
- **NAKAMURA, Yusuke**
  **Yokohama-shi, Kanagawa 225-0011 (JP)**
- **SEKINE, Akihiro**
  **Kunitachi-shi, Tokyo 186-0001 (JP)**
- **IIDA, Aritoshi**
  **Kawasaki-shi, Kanagawa 211-0014 (JP)**
- **SAITO, Susumu**
  **Ome-shi, Tokyo 198-0042 (JP)**

(74) Representative: **Glawe, Delfs, Moll & Partner**
**Patentanwälte**
**Rothenbaumchaussee 58**
**20148 Hamburg (DE)**

(54) **METHOD OF DETECTING GENE POLYMORPHISM**

(57) A method for detecting a genetic polymorphism(s) in a gene encoding cytochrome P450 using as oligonucleotide probes and/or oligonucleotide primers at least one sequence selected from the group consisting of an at least 13 nucleotide sequence within any of the nucleotide sequences as shown in SEQ ID NOS: 1 through 215, said at least 13 nucleotide sequence containing the 21st nucleotide, or a sequence complementary to said at least 13 nucleotide sequence.

EP 1 536 003 A1

## Description

TECHNICAL FIELD

The present invention relates to information on genetic polymorphisms; a method for detecting information on genetic polymorphisms; a method for evaluating drugs using genetic polymorphisms; and a method for screening for drugs.

BACKGROUND ART

As physical appearances of human individuals vary infinitely, the human genetic code consisting of three billion (3,000,000,000) base pairs vary at a considerably large number of sites when compared among individuals. These differences in the genetic code are called genetic polymorphisms, and single nucleotide polymorphism is known as a representative polymorphism.

Single nucleotide polymorphism (SNP) means a difference in one DNA letter among individuals. As faces and shapes of human individuals vary infinitely, nucleotide sequences (i.e. genetic code) of individuals vary at a considerably large number of sites. SNPs are classified into cSNP (coding SNP) and gSNP (genome SNP) depending on their locations; cSNP is further classified into sSNP (silent SNP), rSNP (regulatory SNP) and iSNP (intron SNP).

These SNPs are useful as polymorphic markers in searching for those genes which are associated in the development or worsening of diseases; finally, these SNPs directly relates to risk diagnosis of diseases or selection and use of therapeutic drugs in the clinical field. Also, drug development on the basis of evidence obtained using causative substances as target molecules has become the trend of the world. When a drug is administered to patients with the same disease, their responsiveness is diverse. Some patients show remarkable effect; some patients show low effect; and some patients show no effect. Thus, responsiveness to a drug varies greatly depending on the patient. Even if the conditions of patients are the same and diagnosed as the same disease, the routes which have caused that disease may be different; or the rate of metabolizing drugs may vary greatly among patients. Therefore, it is desired to select an appropriate drug and develop an appropriate therapeutic method against a target disease based on genetic polymorphisms such as SNPs (i.e. the so-called tailored medicine is desired).

In addition to responsiveness to drugs, the problem of strong side effect which sometimes might be lethal is also one of the major problems that medical staffs should address. Even if there is no excessive administration caused by prescription error or the like, unexpected, lethal side effect might occur. Therefore, with respect to responsiveness to a drug, it is desired that individual difference in the metabolism of the drugs, delivery of the drug, and the strength of responsiveness or side effects by the drug's receptor should be determined taking into account genetic polymorphisms such as SNPs.

It is an object of the present invention to provide a method for detecting information on genetic polymorphism; a method for evaluating the efficacy and safety of drugs based on the information; and a method for screening for drugs.

DISCLOSURE OF THE INVENTION

As a result of extensive and intensive researches toward the solution of the above problem, the present inventors have succeeded in establishing a method which comprises detecting genetic polymorphisms in a gene encoding a drug metabolizing enzyme and evaluating with the resultant information the cause and effect relationship between the drug and the disease. Thus, the present invention has been achieved.

The present invention is as described below.

(1) A method for detecting a genetic polymorphism(s) in a gene encoding cytochrome P450 using as oligonucleotide probes and/or oligonucleotide primers at least one sequence selected from the group consisting of an at least 13 nucleotide sequence within any of the nucleotide sequences as shown in SEQ ID NOS: 1 through 215, said at least 13 nucleotide sequence containing the 21st nucleotide, or a sequence complementary to said at least 13 nucleotide sequence.

The genetic polymorphism includes a single-nucleotide polymorphism, a polymorphism caused by deletion, substitution or insertion of a plurality of nucleotides, or VNTR or microsatellite polymorphism may be enumerated. The genetic polymorphism information according to the present invention is listed in Table 1.

(2) A method for evaluating a drug, comprising evaluating the efficacy and safety of the drug metabolized by cytochrome P450 from the detection results obtained by the method of (1) mentioned above.

(3) A method for screening a drug, comprising selecting a drug to be used with reference to the evaluation obtained by the method of (2) mentioned above.

(4) A method for screening a drug, comprising comparing information about a polymorphism(s) in a gene encoding cytochrome P450 and/or a sequence complementary thereto (for example, information shown in Table 1) with information about a polymorphism(s) in a gene encoding cytochrome P450 and/or a sequence complementary thereto obtained from a subject; analyzing the efficacy and/or safety of drugs metabolized by cytochrome P450; and selecting a drug to be used from the analysis results obtained.

(5) The method of (1) to (4) mentioned above, wherein the cytochrome P450 is at least one selected from the group consisting of 2A6, 2A13, 2B6, 2E, 2S1, 5A1, 7A1 and 7B1.

(6) An oligonucleotide selected from the group consisting of the nucleotide sequences as shown in SEQ ID NOS: 1 through 215 and sequences complementary thereto.

(7) An oligonucleotide prepared so as to include a gene polymorphic site present in a gene encoding cytochrome P450 and/or a sequence complementary thereto based on the information about a polymorphism(s) in the gene and/or the complementary sequence thereto.

In this case, the oligonucleotide may be designed such that the nucleotide at the 5'-end, the 3'-end or the center of the oligonucleotide corresponds to the gene polymorphic site.

As more specific examples of the oligonucleotide containing a gene polymorphic site, a oligonucleotide which is composed of two fragments being linked to each other, one fragment being hybridizable to the gene encoding a cytochrome P450 or the sequence complementary thereto and the other fragment being not hybridizable thereto, and said polymorphic site is positioned at the 5'-end or the 3'-end of the hybridizable fragment may be given.

More specifically, the oligonucleotide consists of an at least 13 nucleotide sequence within any of the nucleotide sequences as shown in SEQ ID NOS: 1 through 215, said at least 13 nucleotide sequence containing the 21st nucleotide, or a sequence complementary to said at least 13 nucleotide nay be given.

(8) An oligonucleotide which is designed in a genomic DNA region containing a gene polymorphic site in any of the nucleotide sequences as shown in SEQ ID NOS: 1 through 215 so that it is located within 1000 bp of the polymorphic site toward the 5'-end and/or the 3'-end of the genomic DNA region, and which has a length of 13-60 nucleotides.

(9) A microarray wherein the oligonucleotide of (6) to (8) mentioned above is immobilized on a support.

(10) A genetic polymorphism detection kit comprising the oligonucleotide of (6) to (8) mentioned above and/or the microarray of (9) mentioned above.

BRIEF DESCRIPTION OF THE DRAWINGS

Fig. 1 is a diagram showing TaqMan probes.
Fig. 2 is a diagram showing an outline of TaqMan PCR.
Fig. 3 is a diagram showing probes labeled with a fluorescent dye.
Fig. 4A is a diagram showing an outline of the invader method.
Fig. 4B is a diagram showing locational relationships between an invader probe and an allele probe.
Fig. 4C is a diagram showing locational relationships between an invader probe and an allele probe.
Fig. 5 is a diagram showing a FRET probe.
Fig. 6 is a diagram showing an outline of the invader method.
Fig. 7 is a diagram showing a probe that does not match with an allele.
Fig. 8 is a diagram showing an outline of allele discrimination by ligation reaction.
Fig. 9 is a drawing showing the structure of CYP2A6 cytochrome P450 (subfamily IIA (phenobarbital-inducible) Cpolypeptide 6) gene, and the locations of SNPs.
Accession No.: NG_000008.2
Fig. 10 is a drawing showing the structure of CYP2A13 cytochrome P450 (subfamily IIA (phenobarbital-inducible) Cpolypeptide 13) gene, and the locations of SNPs.
Accession No.: AC008962.9
Fig. 11 is a drawing showing the structure CYP2B6 cytochrome P450 (subfamily IIB (phenobarbital-inducible) Cpolypeptide 6) gene, and the locations of SNPs.
Accession No.: NG_000010.1
Fig. 12 is a drawing showing the structure of CYP2E cytochrome P450 (subfamily IIE (ethanol-inducible)) gene, and the locations of SNPs.
Accession No.: AL161645.14
Fig. 13 is a drawing showing the structure of CYP2S1 cytochrome P450 (subfamily IIS, polypeptide 1) gene, and the locations of SNPs.
Accession No.: NG_000011.1
Fig. 14 is a drawing showing the structure TBXAS1 thromboxane A synthase 1 (platelet, cytochrome P450, subfamily V) (CYP5A1) gene, and the locations of SNPs.

Accession No.: AC006021.2, AC004914.1, and AC004961.2

Fig. 15 is a drawing showing the structure of CYP7A1 cytochrome P450 (subfamily VIIA (cholesterol 7 alphamonooxydase), polypeptide 1) gene, and the locations of SNPs.

Accession No.: AC020782.10

Fig. 16 is a drawing showing the structure of CYP7B1 cytochrome P450 (subfamily VIIB (Oxysterol 7 alpha-hydroxylase), polypeptide 1) gene, and the locations of SNPs.

Accession No.: AF127089.1, AF176800.1, and AF215845.2

Fig. 17 shows a result of typing the subjects from the different two groups by invader assay.

The present invention is explained in detail as follows.

The present invention relates to a method for detecting genetic polymorphisms in a subject using information on genetic polymorphism in a gene encoding drug metabolizing enzyme, especially, a gene encoding cytochrome P450 and/or a sequence complementary thereto. The present invention is also characterized by analyzing the absence/presence or intensity of the efficacy and safety of the drug; based on the analysis result, the relation between the disease and the drug is evaluated. Even when a plurality of patients suffer from the same disease, it is quite often that genetic polymorphism information on drug metabolizing enzyme gene varies by individual patient. Therefore, difference in drug response is drawn out from genetic polymorphism information different among individuals; then, the efficacy or safety of the drug (i.e. the efficacy of the specific drug is recognized or not recognized when a patient has such and such genetic polymorphism information, or side effect occurs frequently or seldom when a patient has such and such genetic polymorphism information) is evaluated. From these results, it becomes possible to determine what drug should be used for a specific disease, and to administer a drug suitable for an individual patient (tailored medicine) based on his/her genetic polymorphism information.

1. Definition

Now, the terms used in this specification will be explained below.

The term "drug metabolizing enzyme" is a general term for enzymes catalyzing in-vivo structural conversion of an exogenous substance including a medicament. Enzymes that are involved in metabolism of some endogenous substances are also included in the drug metabolizing enzyme as long as they are administered for a therapeutic purpose.

The term "efficacy" refers to a property of a drug that has a beneficial effect (efficacy) on a subject when the drug is administered to the subject. The term "safety" is a property of a drug that has an adverse effect or a side effect on a subject when the drug is administered to the subject.

The term "genetic polymorphism information" includes both polymorphism information in a gene and polymorphism information in its complementary sequence.

The term "therapy" means that at least one symptom of a disorder or a disease is cured or mitigated.

The term "screening" means a step of selecting a drug candidate and refers to a step of narrowing down a drug candidate by screening numerous test substances.

The term "gene" refers to RNA having no coding function (e.g., ribosome RNA or transfer RNA), a regulatory sequence required for producing a polypeptide or a precursor, and a DNA sequence having a coding sequence. The RNA or polypeptide may be encoded by a full-length coding sequence or a part of the coding sequence as long as it can be provided with a desired activity or function.

The term "wild type" means a gene or gene product isolated from a naturally occurring source and having a function inherent to the gene or gene product. The wild type gene is one that is most frequently observed in a population, and more specifically, means a "normal type" or "wild type" of gene. On the other hand, the "mutation type" means a gene or gene product having a change in sequence and/or functional characteristics in comparison with a wild-type gene or gene product, and sometimes called as a "modification type" or a "polymorphism type". Examples of such a mutation type include (1) a gene having a modified characteristic compared to a wild-type gene or gene product and (2) a gene having the same function although it differs in gene sequence. The wild type structural gene and its mutation type, these two type of structural genes, have sequence variation caused by substitution and/or deletion of a single nucleotide, or insertion of not less than one nucleotide.

The term "oligonucleotide" is defined as a molecule having not less than two deoxyribonucleotides or ribonucleotides, preferably at least 5 nucleotides, more preferably at least about 10 to 15 nucleotides, and further preferably, at least about 15 to 30 nucleotides. The accurate size of the oligonucleotide may be changed depending upon its function and use. The oligonucleotide can be produced by a known method in the art, for example, chemical synthesis, DNA replication, reverse transcription, PCR, or a combination thereof.

The oligonucleotide is prepared by binding the 5' phosphate group of its pentose ring to the 3' oxygen of the pentose ring of an adjacent mononucleotide via a phosphodiester bond in a single orientation. When the 5' phosphate group is not bound to the 3' oxygen of a mononucleotide pentose ring, the end of the oligonucleotide is called the "5'-end", whereas the 3' oxygen is not bound to the 5' phosphate group of a mononucleotide pentose, the end of the oligonucletode is called the "3'-end". Thus, a polynucleotide has the 5'-end and the 3'-end. In the case where a polynucleotide chain has a first region and a second region and the 3'-end of the first region lies anterior to the 5'-end of the second region, the first region is present upstream of the second region.

The term "polynucleotide sequence" means (1) an oligonucleotide, nucleotide, or polynucleotide, (2) a fragment or a part of them, or (3) a genome or synthesized DNA or RNA. The polynucleotide sequence may be a single strand chain or a double strand chain, or a sense chain or an antisense chain. Note that a nucleotide that is rarely found in a naturally occurring polynucleotide may be included in a polynucleotide used in the present invention. Examples of such a nucleotide include inosine and 7-deazaguanine.

The term "nucleotide analogue" refers to a modified nucleotide or a non-naturally occurring nucleotide, for example, 7-deazapurine (i.e., 7-deaza-dATP and 7-deaza-dGTP). Examples of such a nucleotide analogue include a base analogue as well as a modified deoxyribonucleotide and a modified ribonucleotide.

The term "peptide nucleic acid" (PNA) includes a base or a base analogue as found in a naturally occurring polynucleotide and refers to a nucleic acid molecule bound not to the sugarphosphate skeleton of a typical polynucleotide but to a peptide skeleton. Since a nucleotide is bound to a peptide, the nucleotide can be paired with a complementary nucleotide of a polynucleotide similarly to the case of an oligonucleotide. The aforementioned small molecule is also called as an antigene agent, which binds to a complementary polynucleotide chain, thereby stopping the transcriptional extension (Nielsen, et al., Anticancer Drug Des. 8:53 63 (1993)). In the present invention, in place of a polynucleotide, the nucleotide analogue and a peptide nucleic acid mentioned above may be used.

The terms "protein", "polypeptide" and "peptide" are defined to have the same meaning when they are used regarding a gene product. Note that a polypeptide produced by recombinant DNA technology is particularly referred to as a "recombinant protein". Such a recombinant protein can be obtained through recombinant DNA technology by inserting DNA encoding a polypeptide into an appropriate vector, thereby transforming a host cell, and expressing a heterogeneous protein.

The term "purified" means that a polynucleotide molecule, peptide, polypeptide or protein molecule is taken out from a natural environment, or isolated or separated by recombinant DNA technology, and that it contains, in a natural state, other components in an amount of 40% or less, preferably 25% or less, and most preferably 10% or less. Therefore, the term "isolated polynucleotide" or "isolated oligonucleotide" is a substantially purified polynucleotide.

The term "polymorphism" refers to the fact that one or more types of morphology simultaneously exist with respect to a gene or a part of the gene. The portion of a gene presenting at least two different morphologies, that is, having at least different nucleotide sequences, is called a "polymorphic region of a gene". The polymorphic region includes, for example, a genetic polymorphism such as a single nucleotide polymorphism as described later. The polymorphic region is different depending upon various alleles. There are some polymorphic regions having a several-nucleotide length.

The term "polymorphic gene" refers to a gene having at least one polymorphic region. Note that the term "polymorphic gene locus" is a locus present in a population but varies between members of the population. The most common allele is present with a frequency of less than 0.95. On the other hand, the term "mono morphic gene locus" is a locus that is rarely different between members of a population or completely the same between them. In general, the most common allele is considered to be a gene locus whose frequency does not exceed 0.95 in a gene pool of a population.

The term "information" refers to accumulation of fact or data. The information stored or processed by use of a computer system means data stored in accordance with an arbitrary format. Examples of such a computer system include, but not particularly limited to, the Internet and the like. Examples of such a format include analog, digital and optical formats. The term "information on a subject" means that fact or data regarding the subject. The term "genomic information" refers to information regarding a genome and include, but not limited to, a nucleotide sequence, gene, allele frequency, RNA expression level, protein expression, and the correlation between a genotype and a phenotype. The term "allele frequency information" refers to fact or data regarding an allele frequency and include, but not limited to, allele identification, statistical correlation between the presence of an allele and a trait of a subject, the presence or absence of an allele in an individual or a population, a probability (%) of the presence of an allele in an individual showing at least one specific trait.

The term "complementary" or "complementarity" is a concept used in mentioning the relationship between polynucleotides having nucleotides that can be paired with each other (i.e., nucleotide sequences of an oligonucleotide or a target polynucleotide). To explain more specifically, the sequence "5'-A-G-T-3'" is complementary to the sequence "3'-T-C-A-5'". The "complementarity" may be established "partly", that is, a certain number of nucleotides of a polynucleotide complementarily can make a pair of nucleotides or established completely, that is, all nucleotides can make a pair. The extent of the complementarity has a significant effect upon the efficiency and intensity of hybridization. This is particularly important in a detection method by use of an amplification reaction and coupling between polynucleotides. A nucleotide analogue to be used to make a pair with another nucleotide analogue (e.g., IsoC/IsoG nucleotide pair) or a naturally occurring nucleotide is also included in the definition range of the "complementary" or "complementarity" and may be said that they are complementary to the partner to be paired.

The complementarity of a nucleotide sequence means that when nucleotide sequences are laid side by side such that the 5'-end of one of the sequences faces the 3'-end of the other, oligonucleotide sequences are in "inverse-parallel relationship". The sequences may not be necessary to be completely complementary. The double helix thus is stably formed even if it includes a mismatched nucleotide pair or an uncoupled nucleotide. It can be easy for one skilled in the art to empirically determine the stability of a double helix in consideration of length of the oligonucleotide, the nucleotide composition, nucleotide sequence of the oligonucleotide, ionic strength and the presence of a mismatch nucleotide pair of oligonucleotides.

The term "hybridization" is the concept used in pairing of complementary polynucleotides with each other. The hybridization and hybridization intensity (in other words, polynucleotide-polynucleotide binding strength) is influenced by the complementarity between polynucleotides, the stringency of the conditions to be employed, and the $T_m$ of the hybrid to be formed. In the hybridization method, a polynucleotide is annealed with a complementary polynucleotide (polynucleotide having a complementary nucleotide sequence).

The symbol "$T_m$" means a "melting temperature", which refers to a temperature at which a double stranded polynucleotide is denatured with heat to convert into single strands. To cause a priming reaction during which a template DNA and a primer form into a double strand, it is necessary to set the annealing temperature to be equal to or lower than the $T_m$ of the primer. Some calculation formulas for obtaining $T_m$ of a polynucleotide are known in the art. As is described in a general reference, when a polynucleotide is dissolved in a 1 M aqueous NaCl solution, the $T_m$ value can be simply estimated, based on the following formula:

$$T_m = 81.5 + 0.41\ (\%\ G + C)$$

(See, for example, Anderson and Young, Quantitative Filter Hybridization, Nucleic Acid hybridization (1985)). Also, another reference describes a method of calculating $T_m$ more precisely in consideration of structural, environmental characteristics and sequential characteristics (see, for example, Allawi, H.T. & SantaLucia, J., Jr. Thermodynamics and NMR of internal G. T mismatches in DNA. Biochemistry 36, 10581-94 (1997)).

The term "stringency" is a concept used in mentioning conditions of hybridization including temperature, ionic strength and the presence of other compounds. Under "high stringency conditions", nucleotide paring (base pairing) takes place only between polynucleotide fragments constituted of sequences having complementary nucleotides with a high frequency. Therefore, "poor" or "low" stringency conditions are used when hybridization is performed between a polynucleotide that is not completely complementary to a polynucleotide to be coupled by annealing.

As the "high stringency conditions", in the case where a probe having about 500 nucleotide length is used, mention may be made of the conditions in which hybridization is performed in a solution containing 5 × SSPE (43.8 g/l NaCl, 6.9 g/l $NaH_2PO_4$ $H_2O$ and 1.85 g/l EDTA, pH is adjusted to 7.4 with Na-OH), 0.5% SDS, 5 × Denhalt agent, and 100 μg/ml denatured sermon sperm DNA, at 42°C, followed by washing in a solution containing 0.1 × SSPE and 1.0% SDS at 42°C. 500 ml of the 50 × Denhalt solution contains 5 g Ficoll (Type 400, Pharamcia), and 5 g BSA (fraction V; Sigma).

As the "medium stringency conditions", when a probe having about 500 nucleotide length is used , mention may be made of the conditions in which hybridization is performed in a solution containing 5 × SSPE (43.8 g/l NaCl, 6.9 g/l $NaH_2PO_4$ $H_2O$ and 1.85 g/l EDTA, pH is adjusted to 7.4 with NaOH), 0.5% SDS, 5 × Denhalt agent, and 100 μg/ml denatured sermon sperm DNA, at 42°C, followed by washing in a solution containing 1.0 × SSPE and 1.0% SDS at 42°C.

As the "low stringency conditions", when a probe having about 500 nucleotide length is used, mention may be made of the conditions in which hybridization is performed in a solution containing 5 × SSPE (43.8 g/l NaCl, 6.9 g/l $NaH_2PO_4$ $H_2O$ and 1.85 g/l EDTA, pH is adjusted to 7.4 with NaOH), 0.1% SDS, 5 × Denhalt agent and 100 μg/ml denatured sermon sperm DNA, at 42°C , followed by washing in a solution containing 5 × SSPE and 0.1% SDS at 42°C.

As to the complementarity, it is important to determine whether hybridization is completely performed or partially performed, depending upon its usage. For example, for the purpose of simply detecting the presence or absence of an exogenous DNA sequence, it is important to choose conditions under which hybridization takes place without fail as long as a detection-target, DNA sequence is present, by either a partially complementary probe or a completely complementary probe. On the other hand, there are some occasions requiring a hybridization method that can distinguish partially complementarity from the complete complementarity.

The term "primer" means an oligonucleotide capable of starting synthesis when it is placed under conditions capable of starting primer extension. An oligonucleotide primer may be naturally occurring one, purified one through restriction digestion, or synthetically produced. The primer is designed so as to be complementary to a specific sequence of a template. A primer has to be sufficiently complementary to a template sequence such that it hybridizes with a template so as to cause primer extension, thereby forming a template-primer complex to obtain a primer extension product. Note that a primer sequence may not be always completely complementary to a template as long as it can couple with the template due to the presence of a partially complementary sequence.

The "label" refers to any atom or molecule as long as it can be used in detection (preferably quantification) and bind to a polynucleotide or a protein. Examples of such a label include, but not limited to, a dye such as a radioactive label (e.g., 32P), a binding group (e.g., biotin), a hapten (e.g., digoxigenin), a luminescence group emitting phosphorous light or fluorescent light, and a fluorescent dye. These labels may be used singly or in combination with a group capable of suppressing or shifting an emission spectrum by fluorescent resonance energy transfer (FRET). The label is specified as one capable of generating a detectable signal based on fluorescence, radioactivity, colorimetric determination, weight determination, X-ray diffraction or absorption, magnetic or enzymatic activity. The label used herein may be a charged group (positively or negatively charged group) or a group electrically neutral. When a polynucleotide or a protein sequence contains a detectable label, the label may contain or be composed of such a charged sequence as mentioned above.

The term "signal" means a physicochemically detectable signal generated by a label or an assay reaction.

The "detector" refers to a system or a structural element of a system, more specifically, a device or a reactive medium of informing the presence of a signal to a user or another structural element of the system (e.g., computer or controller). Examples of such a device include, but not limited to, a camera, fluorescence detector, a charge coupled device, and scintillation counter. Examples of such a reactive medium include, but not limited to, X-ray, photographic film, and pH indicator.

Examples of such a detector include a light analysis system capable of detecting ultraviolet rays, visible rays or infrared rays (e.g., fluorescent or chemical luminescence), a spectrophotometric system, a radiation detection system, spectrometry (such as magnetic nuclear resonance spectrometry, mass spectrometry, surface-induced Raman spectrophotometry), a system such as gel electrophoresis, capillary electrophoresis, or gel exclusion chromatography, other detection systems known in the art, and a combination of these systems.

The "sample" refers to a biological specimen and a synthetic specimen. Examples of such a biological specimen include an animal specimen (e.g., human) such as a liquid, solid (fecal matter) or tissue sample, food in a liquid or solid form, feed and feed staff (daily product, vegetable, meat, meat products), and other miscellaneous wastes. The biological sample may be taken from a domestic animal or wild animal, which includes, but not limited to, an ungulate animal, bear, fish, and rodent.

A detection-target, a polynucleotide, is supplied from a sample containing or conceivably containing a polynucleotide (RNA or DNA). A particularly preferable source of such a target polynucleotide is a biological sample, which includes, but not limited to, blood, saliva, cerebrospinal fluid, pleural fluid, milk, lymph, sputum and semen.

The term of a "cleaved structure" of a nucleotide refers to a structure formed by interacting at least one probe oligonucleotide with a target polynucleotide to form a double stranded structure and cleaving the double stranded structure by a cleaving agent. Examples of such a cleaving agent include, but not limited to, enzymes (such as a restriction enzyme). The structure to be cleaved is a substrate for specific cleavage by a cleaving means and thus differs from a polynucleotide molecule, which is a substrate for non-specific cleavage. Examples of such a cleavage means include an agent such as phosphodiesterase cleaving a polynucleotide molecule regardless of its secondary structure.

The term "kit" refers to a supply system for supplying a substance. When it is used in a reaction assay, a system for storing, transporting or supplying a reaction reagent and/or auxiliary substance is included in such a supply system. Examples of such a reaction reagent include, but not limited to, an oligonucleotide and an enzyme contained in a container. Examples of such an auxiliary substance include, but not limited to, a buffer and an instruction leaflet. Examples of such a kit include at least one type of accommodation unit (e.g., box) containing a relevant reaction reagent and/or auxiliary substance, and the like. The term "partial kit" refers to a supply system containing a part of all components of the kit, that is, no smaller than two discrete containers. The containers are supplied together or separately to a user. For example, a first container contains an enzyme to be used for assay and a second container contains an oligonucleotide. Alternatively, the kit may be supplied as a "composite kit". The composite kit refers to a supply system containing all ingredients for a reaction assay in a single container. For example, a composite kit may contain necessary ingredients in a single accommodation case. Both the partial kit and the composite kit as mentioned above are included in the "kit" according to the present invention.

The term "subject" means a target living organism from which genetic polymorphism information is obtained by the present invention and in which a drug is evaluated and screened. Examples of such a subject include, but not limited to, mammals (such as a rodent, a primate including a human, sheep, goat, horse, dog, and cow), birds, amphibians, and reptiles. A subject is preferably selected from primates including human and rodents including rats and mice, most preferably, a human. Genetically manipulated animals, namely, transgenic animals, and knockout animals are also useful for evaluating and screening of a drug having an effect upon various processes (embryogenesis and histogenesis).

2. Genetic Polymorphism

Genetic polymorphism includes single nucleotide polymorphism, insertion/deletion type polymorphism, and polymorphism caused by difference in the number of repetition of a nucleotide sequence. Generally, single nucleotide polymorphism (SNP) means a polymorphism caused by substitution of one specific nucleotide with other nucleotide in a gene or its complementary strand (complementary sequence) region. In the present invention, however, the term SNP also includes a polymorphism caused by deletion of the nucleotide and a polymorphism caused by addition of one more nucleotide to the nucleotide. Insertion/deletion type polymorphism means a polymorphism caused by deletion or insertion of a plurality of nucleotides (e.g. two to several ten nucleotides). Sometimes, several hundred to several thousand nucleotides may be deleted or inserted. The polymorphism caused by difference in the number of repetition of a nucleotide sequence has repetition of a sequence of two to several ten nucleotides, and the number of this repetition varies among individuals. Those polymorphisms where the repeat unit consists of several to several ten nucleotides are called VNTR (variable number of tandem repeat) polymorphism, and those polymorphisms where the repeat unit consists of about two to four nucleotides are called microsatellite polymorphism. In VNTR or microsatellite polymorphisms, the number of such repetition is different among individuals' alleles, which results in acquisition of variation.

3. Drug metabolizing enzyme

The drug metabolizing enzyme is involved in absorption, metabolism, and excretion of drugs. A polymorphism of the enzyme changes an expression (transcription and translation) amount and the activity of the enzyme, with the result that blood concentrations of an unchanged drug or its metabolites are changed.
In the present invention, examples of a drug metabolizing enzyme expressed by a target gene of gene polymorphism analysis include cytochrome P450 (CYP) and the like.
CYP (cytochrome P450), which is responsible for a first-phase drug metabolism, introduces an oxygen atom into a drug. Examples of CYP include CYP 2A6, CYP 2A13, CYP 2B6, CYP 2E, CYP 2S1, CYP 5A1, CYP 7A1 and CYP 7B1. A single CYP or a combination of them is appropriately selected.

4. Detection and identification of genetic polymorphism of CYP gene

Information on genetic polymorphisms may be obtained by conventional methods for detecting genetic polymorphisms. For example, the sequencing method, the PCR method, Fragment length polymorphism assay, hybridization methods using an allele-specific oligonucleotide as a template (e.g. TaqMan PCR method, the invader method, the DNA chip method), methods using primer extension reaction, MALDI-TOF/MS method, the DNA chip method, and the like may be employed. The PCR method and the direct sequencing method may be used for detecting any type of genetic polymorphism. The other methods may be used mainly for detecting SNPs.
Hereinafter, the outline of each method for detecting genetic polymorphism sequence is explained.

(1) Direct sequencing assay

A gene polymorphism sequence can be detected by use of a direct sequencing method. In this assay, a DNA sample is first taken from a subject by an appropriate method. A target detection region is cloned into an appropriate vector and amplified through proliferation of a host cell (e.g., bacterial cell). The target detection region refers to a region containing, for example, an SNP or a mutation. Alternatively, DNA within the target detection region may be amplified by use of PCR. After the amplification, DNA within the target detection region is subjected to sequencing by an appropriate method. Examples of such a sequencing method include, but not limited to, a manual sequencing method or an automatic sequencing method. Examples of such a manual sequencing method include a method of using a radioactive marker nucleotide and the like. Examples of such an automatic sequencing method include a method using a Dye Terminator and the like. The sequencing results are shown by an appropriate display method. Thereafter, the presence or absence of a predetermined SNP or mutation is determined.

(2) PCR assay

In the present invention, a gene polymorphism sequence can be detected by use of an assay based on PCR. The PCR assay uses an oligonucleotide primer forming a hybrid only within a mutation type or wild type allele (e.g., polymorphic or mutation region). A DNA sample is amplified by use of a primer set consisting of primers for a mutation type and a wild type. When only the mutation-type primer generates a PCR product, it is demonstrated that the subject has a mutation allele. When only the wild-type primer generates a PCR product, it is demonstrated that the subject has a wild-type allele.

(3) Fragment length polymorphism assay

In the present invention, a gene polymorphism sequence can be detected by fragment length polymorphism assay. In the fragment length polymorphism assay, a specific DNA band pattern is obtained based on a series of enzymatic DNA cleavage sites. Examples of such an enzyme include restriction enzymes and Cleavase I (Third Wave Technologies, Inc.). The DNA fragment derived from a sample and having a SNP or a mutation exhibits a different band pattern from that of a wild type.

(3-a) RFLP assay

In the present invention, Restriction Fragment Length Polymorphism (RFLP) assay is also used to detect a gene polymorphism sequence. First, a target detection region is amplified by PCR. Subsequently, the resultant PCR product is cleaved with a restriction enzyme(s) known to produce a fragment of a specific length inherent to a predetermined polymorphism. The PCR product digested into pieces with the restriction enzyme(s) is generally separated by gel electrophoresis and visualized by staining with ethidium bromide. The lengths of fragments are compared to those of a molecular marker and wild type and mutation-type controls.

(3-b) CFLP assay

In the present invention, Cleavase Fragment Length Polymorphism (CFLP) can be also used to detect a gene polymorphism sequence (see Third Wave Technologies Inc., USP Nos. 5,843,654, 5,843,669, 5,719,208 and 5,888,780). Cleavase I enzyme is a structure-specific heat resistant nuclease which recognizes and cleaves the junction between a single stranded region and a double-stranded region of DNA.

In the first place, the DNA of a target detection region is isolated, for example, by PCR. In this step, one or two stands are preferably labeled. Subsequently, the DNA strands are separated by heating. The resultant product is annealed to form a secondary structure within a strand. Subsequently, the PCR product is treated with Cleavase I enzyme to generate a series of fragments inherent to a predetermined SNP or mutation. The PCR product treated with Cleavase enzyme is separated and detected by denatured gel electrophoresis, and subsequently visualized by autoradiography, fluorescent imaging or staining. The length of fragments is compared to those of a molecular maker and wild type and mutation-type controls.

(4) Hybridization assay

In the present invention, a gene polymorphism sequence can be detected by hybridization assay. The hybridization assay is a method of determining the presence or absence of a predetermined SNP or mutation based on the ability of the DNA derived from a sample to hybridize with a complementary DNA molecule (e.g., oligonucleotide probe). Hybridization assay is performed by various hybridization techniques and detection techniques. Which assay should be selected from them will be explained below.

(4-a) Direct detection of hybridization

Whether or not a probe hybridizes with a target detection sequence (e.g., SNP or mutation) can be directly detected by visualizing a hybridized probe. This method is known as Northern or Southern assay (see. e.g., Ausabel et al.(edited), Current Protocols in Molecular Biology, John Wiley & Sons, NY (1991)). In Southern assay, genomic DNA is isolated from a subject and in Northern assay, RNA is isolated from a subject. Subsequently, isolated DNA or RNA is cleaved with a series of restriction enzymes that infrequently cleave sites except for the region near a marker in the genome. Thereafter, DNA or RNA is separated and transferred onto a film. The separation operation can be carried out, for example, on an agarose gel. The transferring onto the film is performed by transferring a labeled probe or a probe specific to a detection target, an SNP or mutation, onto the film under low, medium or high stringency conditions. Labeling is performed by introducing, for example, a radioactive nucleotide. After an unbound probe is removed, a labeled probe is visualized, thereby detecting the presence of the binding.

(4-b) Detection of hybridization using a DNA chip assay

In the present invention, a gene polymorphism sequence can be detected also by DNA chip hybridization assay. In this assay, a series of oligonucleotide probes are adhered onto a solid support. The oligonucleotide probes are designed so as to have a specificity to a predetermined SNP or mutation. A test DNA sample is brought into contact with the DNA chip thus formed to detect hybridization.

(4-c) Detection of hybridization with enzyme

In the present invention, hybridization can be also detected by using the cleavage of a specific structure with an enzyme (Invader assay; Third Wave Technologies Inc., see, e.g., USP Nos. 5,846,717, 6,090,543, 6,001,567, 5,985,557 and 5,994,069). Invader assay detects specific DNA and RNA sequences by forming an overlapped portion by an oligonucleotide probe and cleaving the overlapped portion with a structure-specific enzyme.

Alternatively, hybridized structure with a probe can be detected by TAQMAN assay (PE Biosystems; see, e.g., USP Nos. 5,962,233 and 5,538,848). This assay is usually performed during the process of PCR. TAQMAN assay uses the 5'-3' exonuclease activity of a DNA polymerase (e.g., AMPLITAQ DNA polymerase). A probe specific to a predetermined allele or mutation is introduced in PCR. The probe is formed of an oligonucleotide having a 5' reporter dye (e.g., fluorescent dye) and 3' quencher dye. During the PCR process, when a probe is bound to a target sequence, a probe is cleaved at a site between the reporter dye and the quencher dye by the 5'-3' polynucleotide decomposition activity of AMPLITAQ polymerase. The intensity of fluorescence increases by separating the reporter dye from the quencher dye. The resultant signal is accumulated in each cycle of PCR, so that the signal can be detected by a fluorescence detector.

Furthermore, in the present invention, a polymorphism can be detected by SNP-IT primer extension assay (Orchid Biosciences Inc.; see, e.g., USP Nos. 5,952,174 and 5,919,626). In this assay, an SNP is specified by selectively extending DNA from a nucleotide positioned at a putative SNP site by use of a DNA primer specifically synthesized and a DNA polymerase. First, DNA within a target detection region is amplified and denatured. Then, a polymerase reaction is performed by a micro system called a microfluidics. Detection is performed by attaching a label to a nucleotide probably positioned at an SNP or a mutation site. The label introduced into DNA can be detected by an appropriate method arbitrarily chosen. For example, when a nucleotide contains a biotin label, it can be detected by a fluorescence-labeled antibody specific to biotin.

(5) Method of using a primer extension reaction

In the present invention, a gene polymorphism sequence can be detected also by use of a primer extension reaction. As a method of using a primer extension reaction, for example, a SniPer method may be employed. The SniPer method, which is fundamentally based on a procedure called rolling circle amplification (RCA), is one in which a DNA polymerase continuously synthesizes a complementary DNA while moving on a single stranded circular DNA serving as a template (see, e.g., USP Nos. 6,210,884 and 6,183,960). According to this method, an SNP can be determined by determining the presence or absence of a color reaction generated when DNA amplification takes place (Lizardi, P. M. et al., Nature Genet., 19, 225-232 (1998); Piated, A.S. et al., Nature Biotech., 16, 359-363 (1998)).

(6) Mass spectrometry

In the present invention, a gene polymorphism sequence can be detected by using MassARRAY system (Sequenom Inc.; see, e.g., USP Nos. 6,043,031, 5,777,324 and 5,605,798). First, DNA is isolated from a sample by a standard procedure. Subsequently, a specific DNA region containing a detection target, a mutation or an SNP, is amplified by PCR. For example, the region may have a length of 200-nucleotides. Subsequently, one of the strands thus amplified is bound and fixed to a surface of a solid phase, and then, the other chain unbound thereto is removed by denaturing generally performed or washing. Using the remaining and fixed single strand as a template in an automatic enzyme reaction, a product to be detected (detection product) having specificity to genotype is produced.

An extremely small amount of enzyme product (generally, 5-10 nano-liter) is transferred on SpectroCHIP array and then subjected to automatic analysis using an SpectroREADER mass spectrometer. To each spot, the detection product and a light absorbing crystal forming a matrix, are introduced in advance. The MassARRAY system uses MALDI-TOF(Matrix Assisted Laser Desorption Ionization-Time of Fight) mass spectrometry. The MALDI-TOF method is one performing SNP genotyping based on fundamental difference of single nucleotides in mass by using a mass spectrometer. Examples of such a method include a method of using PCR amplification and a method of using a multiplex (Haff, L.A., Smirnov, I. P., Genome Res.,7, 378-(1997); Little, D.P. et al., Eur. J. Clinica. Chem, Clin. Biochem., 35, 545- (1997); Ross, P., et al., Nat Biotechnol., 16, 1347- (1998)).

In the MALDI-TOF, a matrix collides against a pulse derived from a laser beam in a process known as an elimination process. The energy is transferred from the laser beam to the matrix to vaporize the matrix, with the result that a small amount of detection product is excluded into a flight tube. When an electric field pulse is applied to the flight tube, the detection product is electrically charged. Because of the presence of the detection product, the flight tube proceeds toward a detector. The time between the application of the electric field pulse and collision of the detection product onto the detector is expressed as flight time. Since the mass of a molecule is directly correlated to the flight time in the method, the molecular weight of the detection product can be quite accurately determined. More specifically, the smaller the molecule of the detection product, the shorter the flight time. The larger the molecule of the product, the longer the flight time. Since the assay is completed within 1/1000 seconds, a sample can be analyzed within 3-5 seconds in total including time for obtaining repetitive data. Subsequently, a genotype can be computationally obtained, recorded, compared and reported at a rate of 3 seconds per sample in accordance with SpectroTYPER soft ware.

(7) Other detection assays

Examples of other detection assays available in the method of the present invention include, but not limited to, an enzyme-mismatch cleavage method (see, e.g., Variagenics Inc.; USP Nos. 6,110,684, 5,958,692, and 5,851,770), polymerase chain reaction, branched chain DNA hybridization method (see, e.g., Chiron Corporation; USP Nos. 5,849,481, 5,710,264, 5,124, 246, and 5, 624,802), NASBA method (see, e.g., USP No.5,409,818), molecular beacon technique (see, e.g., USP No. 6,150,097), E-sensor technique (see Motorola; USP Nos. 6,248,229, 6,221,583, 6,013,170, and 6,063,573), cycling probe technique (see, e.g., USP Nos. 5,403,711, 5,011,769, and 5,660,988), Dade Behring signal amplification method (see, e.g., USP Nos. 6,121,001, 6,110,677, 5,914,230, 5,882,867, and 5,792,614), ligase chain reaction (Barnay Proc., Natl. Acad. Sci USA 88, 189-93 (1991)), and sandwich hybridization method (see, e.g., USP No. 5,288,609).

Gene polymorphism information of a CYP gene obtained by the detection methods mentioned above is listed in Table 1.

Table 1

| Designation of Gene | No. | Location | Sequence | SEQ ID NO |
|---|---|---|---|---|
| CYP2A6 | 1 | exon 1 + 31 | tgctggcctcagggatgctt C/T tggtggccttgctggtctgc | 1 |
| CYP2A6 | 2 | exon 1 + 153 | ttcattggaaactacctgca G/A ctgaacacagagcagatgta | 2 |
| CYP2A6 | 3 | intron 1 + 29 | aggcagggagatgggtggca C/T ggggtgggggctgcctagtt | 3 |
| CYP2A6 | 4 | intron 1 + 98 | cagtgtggaccagagtctta G/A gaaatggagttttggagttt | 4 |
| CYP2A6 | 5 | intron 1 + 153 | caggatcttgggatgtccag C/T tccctgactgtgagaacctg | 5 |
| CYP2A6 | 6 | intron 2 + 1011 | agctgctccgctcctgcccc C/T gccgcccctggcctgtctc | 6 |
| CYP2A6 | 7 | intron 3 + 23 | gagcaggggaccccgagtgc G/T ggggcaggagaaggaaaaca | 7 |
| CYP2A6 | 8 | intron 3 + 77 | acccgcgcgcgttctgcctg G/C ggatggggactaggtgggga | 8 |
| CYP2A6 | 9 | intron 6 + 323 | ttaacaggatcctactccaa C/A aatgcgaatggctgatgtct | 9 |
| CYP2A6 | 10 | intron 7 + 203 | ctccagactctttgtgtcag G/A agaatcaaacacatgttccc | 10 |
| CYP2A6 | 11 | intron 7 + 440 | cttccccttacctggggcac A/C ctagttccccctccagcccc | 11 |
| CYP2A6 | 12 | exon 8 + 84 | caggacttcaatccccagca C/T ttcctgaatgagaaggggca | 12 |
| CYP2A6 | 13 | exon 8 + 96 | ccccagcacttcctgaatga G/C aaggggcagtttaagaagag | 13 |
| CYP2A6 | 14 | intron 8 + 27 | gaccactgtttgctgccagg C/T cacggctcacaccagcaggg | 14 |
| CYP2A6 | 15 | intron 8 + 47 | ccacggctcacaccagcagg G/C gcctccctcaccctcctccc | 15 |
| CYP2A6 | 16 | exon 9 + 333 | gggaagagaagaaacagaag C/G ggctcagttcaccttgataa | 16 |
| CYP2A6 | 17 | exon 9 + 383 | gagctgggatgagaggaagg A/G aacccttacattatgctatg | 17 |
| CYP2A6 | 18 | exon 9 + 386 | ctgggatgagaggaaggaaa C/A ccttacattatgctatgaag | 18 |
| CYP2A13 | 1 | 5'flanking - 251 | tccacagtttatctgttgcc C/T gctcctaaatccacagccct | 19 |
| CYP2A13 | 2 | 5'flanking - 112 | agctgtgtgggattcagggt T/C ggggtgtagttgggaggtga | 20 |
| CYP2A13 | 3 | exon 1 + 83 | ggtcttgatgtcagtctggc G/A gcagaggaagagcaggggga | 21 |
| CYP2A13 | 4 | exon 2 + 121 | aggctgaggagttcagcggg C/T gaggcgagcaggccaccttc | 22 |
| CYP2A13 | 5 | intron 4 + 118 | cagtgtcccctcaaaatcag T/C ccccgatattggacaactgg | 23 |
| CYP2A13 | 6 | intron 4 + 500 | tctacttcacccacttaaat G/A cctgaaaacatggacaggtg | 24 |
| CYP2A13 | 7 | intron 4 + 673 | cacctcaacaggtatcccct C/G cacttcaacatcttcaccag | 25 |
| CYP2A13 | 8 | intron 4 + 712 | agccccactttaatacctga A/G cacctgaacaaaagcccca | 26 |
| CYP2A13 | 9 | exon 5 + 115 | agaaggtggagcacaaccag C/T gcacgctggatcccaattcc | 27 |
| CYP2A13 | 10 | exon 9 + 283 | gggctaagaatgggggcagt G/C ggggaaggaaggggagaggt | 28 |
| CYP2A13 | 11 | 3'flanking + 145 | gaaagttgtctctatctgat T/G tctcacaaaacgtaagtgcc | 29 |
| CYP2A13 | 12 | 3'flanking + 157 | tatctgatttctcacaaaac G/A taagtgcccagaaaatcttt | 30 |

| Designation of Gene | No. | Location | Sequence | SEQ ID NO |
|---|---|---|---|---|
| CYP2B6 | 1 | 5'flanking - 1179 | caccagttctgcatctcttg C/G tcctccttctgtttctccga | 31 |
| CYP2B6 | 2 | exon 1 + 71 | tcttgctactcctggttcag C/T gccaccctaacacccatgac | 32 |
| CYP2B6 | 3 | intron 1 + 2663 | aacagaaaccaacttatctt C/G gtcgattttgaggttgatag | 33 |
| CYP2B6 | 4 | intron 1 + 2676 | ttatcttcgtcgattttgag G/A ttgatagtaatttcagttat | 34 |
| CYP2B6 | 5 | intron 1 + 2832 | agagtaagcaaacctcaaga T/C actcaaggtaggcactcgtg | 35 |
| CYP2B6 | 6 | intron 1 + 2919 | tagatttgtttacccataag T/C ctgcatagctcctgaacaag | 36 |
| CYP2B6 | 7 | intron 1 + 3212 | ccacacccccataggtagtc C/T ccaggtcttgcatacgggat | 37 |
| CYP2B6 | 8 | exon 2 + 45 | ttcacggtacacctgggacc G/C aggcccgtggtcatgctgtg | 38 |
| CYP2B6 | 9 | intron 2 + 111 | ccaacttcttctacaaccaa C/T ccacacctcccctgcacccc | 39 |
| CYP2B6 | 10 | intron 3 + 2124 | ggggcctgagaggaggtgca G/T agtgagaaccggctgcatgg | 40 |
| CYP2B6 | 11 | intron 3 + 2441 | acttcagtctgtgtccttga C/T ctgctgcttcttcctagggg | 41 |
| CYP2B6 | 12 | exon 4 + 15 | tcctaggggccctcatggac C/G ccaccttcctcttccagtcc | 42 |
| CYP2B6 | 13 | exon 4 + 32 | gaccccaccttcctcttcca G/T tccattaccgccaacatcat | 43 |
| CYP2B6 | 14 | intron 4 + 1850 | agaaagacaaataaacaggc T/C gaggtagacaatgggtgaca | 44 |
| CYP2B6 | 15 | intron 4 + 2048 | gttcagaggcagaggggagt G/A gggaagtggggttcccatgg | 45 |
| CYP2B6 | 16 | intron 5 + 183 | agaaagatgaggtgaaagga G/A ggagaaaatagggaggagga | 46 |
| CYP2B6 | 17 | intron 5 + 488 | agaaaagcaaactgggccag A/G tagtgtcaaagacctttagg | 47 |
| CYP2B6 | 18 | intron 5 + 514 | tcaaagacctttaggccaac G/A gagggcagccagggagatgg | 48 |
| CYP2B6 | 19 | intron 8 + 53 | ccagagggcaggtactatcc C/T caacttgagaaaaacaacga | 49 |
| CYP2B6 | 20 | exon 9 + 165 | tacccccaacataccagatc C/T gcttcctgccccgctgaagg | 50 |
| CYP2E | 1 | 5'flanking - 1621 | tctcaccccaccaaagccaa G/C gcttcaatttcagtctgtgg | 51 |
| CYP2E | 2 | 5'flanking - 1480 | cccacacaggacaacagggt T/G caggggtctggacagctgtt | 52 |
| CYP2E | 3 | intron 2 + 15 | acaggggtgagtccgcgtcc C/T tggcacggagcggggggtgc | 53 |
| CYP2E | 4 | intron 2 + 344 | ttcccctcctgttcaaccgc C/A ggggtacaggtggcttcgtc | 54 |
| CYP2E | 5 | intron 2 + 439 | ttcagggctttgctcagctg C/T agctggtgacctccagagag | 55 |
| CYP2E | 6 | intron 2 + 500 | ggggtggatgtcctgagacc G/A ggaagggggaagagacccac | 56 |
| CYP2E | 7 | intron 2 + 1023 | tgagctgataaagaacgccg T/G cagcacagagcagacgctgg | 57 |
| CYP2E | 8 | intron 2 + 2567 | cttttcaagaatgttgtcga T/C agataggaaagaggtgggag | 58 |
| CYP2E | 9 | intron 4 + 186 | ggggccccacgcaccccctt T/G cctaacgtcaggatgtgtat | 59 |
| CYP2E | 10 | intron 6 + 326 | gacagagaagataatgtccc A/G gttcccccaagtaagacct | 60 |
| CYP2E | 11 | intron 6 + 474 | ctacagctgccctggaccct T/C gttccttccacagggctcct | 61 |

| Designation of Gene | No. | Location | Sequence | SEQ ID NO |
|---|---|---|---|---|
| CYP2E | 12 | intron 6 + 625 | gaggtgatgtgagggcaccc G/A catgcaaacaggccagtcag | 62 |
| CYP2E | 13 | intron 6 + 786 | agttcacagcctgagtggtg T/C gtgccgccctcctcctgaag | 63 |
| CYP2E | 14 | intron 7 + 117 | ctttggcaggggtcactgag T/G ggaagggctggccccactcc | 64 |
| CYP2E | 15 | intron 8 + (217-219) | tcctgggaatgaagtgttgt TGT/Δ aggtggattttttttttccc | 65 |
| CYP2E | 16 | intron 8 + (227-236) | gaagtgttgttgtaggtgga (T)9-11 cccaaagactagacatttta | 66 |
| CYP2E | 17 | 3'flanking + 1501 | agttgacagctttcccaaaa C/T ggttcacggagacttcattt | 67 |
| CYP2E | 18 | 3'flanking + 1505 | gacagctttcccaaaacggt G/T cacggagacttcatttgact | 68 |
| CYP2E | 19 | 3'flanking + 1631 | caaaccaaacatcctctaag G/T tctactgtgcagagtatagc | 69 |
| CYP2S1 | 1 | 5'flanking - 177 | ttcccgacatcaggcggcgg C/T ggtggtccgggagaaacccg | 70 |
| CYP2S1 | 2 | 5'flanking - (22-24) | ccgcgcggagcgcctgggag AGG/Δ agaaggagccgacctgccga | 71 |
| CYP2S1 | 3 | intron 1 + 540 | tgggtggagctcagttggga G/C ttcctgggactggggaggaa | 72 |
| CYP2S1 | 4 | intron 2 + (2991-3001) | acagagcgagattccgtctc (A)10-11 gaaagaaaggaagaaggggg | 73 |
| CYP2S1 | 5 | exon 5 + 35 | ctcctggttcctgcggcccc C/T gccaggcccccacaagcagc | 74 |
| CYP2S1 | 6 | intron 5 + (245-248) | catttgtgccgggctgtctg TCTC/Δ tccagcatctctcctctttc | 75 |
| CYP2S1 | 7 | intron 6 + (332-343) | acagagcgagacactgtttc (A)11-12 gtgagaattctagagggaag | 76 |
| CYP2S1 | 8 | intron 6 + 337 | gcgagacactgtttcaaaaa A/G aaaaaagtgagaattctaga | 77 |
| CYP2S1 | 9 | intron 8 + (157-172) | gaggaatactgactcagccc (TC)7-8 ctcaccagggaagcgtgtct | 78 |
| CYP2S1 | 10 | exon 9 + 757 | ctctcccagcctgtgaccac C/A gatgtccacacacccccaac | 79 |
| CYP5A1 | 1 | intron 1 + (1060-1069) | tttctttttaactggtttaa (T)10-13 gaactttttatctcattgt | 80 |
| CYP5A1 | 2 | intron 1 + 8545 | tccacaaccatcagccccgc C/T ccaggactcactgtgggtct | 81 |
| CYP5A1 | 3 | intron 1 + 8592 | ccttcagcgggccccctgtc G/A ctctctcccccacaaaccct | 82 |
| CYP5A1 | 4 | intron 1 + 8815 | caaaggcactgaggagcccc C/G tctcagcattgcttttatcc | 83 |
| CYP5A1 | 5 | intron 1 + 9827 | aactgctcccttctagtggt A/G aaggggaaagcagtttcatg | 84 |
| CYP5A1 | 6 | intron 1 - (30739-30725) | gagggatacatctatttccc (T)11-13 atctttttgtatttttgtct | 85 |
| CYP5A1 | 7 | intron 1 - 29832 | atgattgaatgaactcttcc C/T ctaaccctagtgccataata | 86 |
| CYP5A1 | 8 | intron 1 - 28130 | gaaattgaagagcaagactg C/T aaaaggaggggatgatggaa | 87 |
| CYP5A1 | 9 | intron 1 - 27482 | catccgtacacagagagtga C/G caaaattggtagcatcatct | 88 |
| CYP5A1 | 10 | intron 1 - 27360 | acagtgtagggacagacaca T/C ctctttccctgggtgtcctt | 89 |
| CYP5A1 | 11 | intron 1 - 27182 | cctaatgttaaccctccaca G/T tctggccagtcttgactccc | 90 |
| CYP5A1 | 12 | intron 1 - 25419 | taagctgaagtgtcttcggg G/A gtgttggggatggtcaccaa | 91 |
| CYP5A1 | 13 | intron 1 - 25391 | ggatggtcaccaatggtgtg G/A tggggacagagccatagtgc | 92 |

| Designation of Gene | No. | Location | Sequence | SEQ ID NO |
|---|---|---|---|---|
| CYP5A1 | 14 | intron 1 - 22669 | tcagtgtaggggccaggttg G/C tcctgggccgaacctgggga | 93 |
| CYP5A1 | 15 | intron 1 - 21974 | ttgcaatccagaatcagcca C/T aaaatcccatttcctttttt | 94 |
| CYP5A1 | 16 | intron 1 - 7228 | aaatactcactattataaat T/C tgtaaatgaacagtctcgtt | 95 |
| CYP5A1 | 17 | intron 1 - 1368 | gggaggccaaggcaggagga C/T cgcttgaggccaggagttaa | 96 |
| CYP5A1 | 18 | intron 1 - 883 | gctgatctctgcgtgatgct A/T aacaaccagtttgactcatt | 97 |
| CYP5A1 | 19 | intron 2 + 508 | tggcaagaatccatctctta T/C cactatcaatatgtattgag | 98 |
| CYP5A1 | 20 | intron 3 + 3741 | agctattagtcgtggtggta C/T gtgataaaaaactctcctga | 99 |
| CYP5A1 | 21 | intron 3 + 6106 | gtcccatactgaaaaaaaaa A/Δ tcaatttagcaaataaataa | 100 |
| CYP5A1 | 22 | intron 3 + (6688-6689) | tggctgaggatagggtaggg TG/Δ gaaactattgctgatttgtc | 101 |
| CYP5A1 | 23 | intron 3 + 6894 | actttaggtttttgctgctg A/G taaaagtgcaagcctcagta | 102 |
| CYP5A1 | 24 | intron 3 + 7924 | tccctcttctttgggtagaa G/A catccaacagggcatgatag | 103 |
| CYP5A1 | 25 | intron 3 + 9360 | tggagaggcaccgagtctca T/C gatgtgtggctggaggttgt | 104 |
| CYP5A1 | 26 | intron 3 + (10750-10774) | aggcactctttgcagatgaa (T)22-25 ggcctcggcttaaagaggaa | 105 |
| CYP5A1 | 27 | intron 3 + 11020 | tccctggcttgtgccacctc C/T atcttttccgtggctaatgt | 106 |
| CYP5A1 | 28 | intron 3 + 12740 | tactgacttctgcttccttg T/C tccctgggtctacactgttc | 107 |
| CYP5A1 | 29 | intron 3 + 13193 | acaaccttggcagtggcaga T/C gagagagaacctcaggactg | 108 |
| CYP5A1 | 30 | intron 3 + 18241 | caatagagtagggaggttta T/A cattacaggtttctttcaga | 109 |
| CYP5A1 | 31 | intron 3 + 18569 | actaccgtgccaccacccac G/A caccacaccattgctgcaga | 110 |
| CYP5A1 | 32 | intron 3 + 20654 | acaaggagcgaagggtccat G/A aagccttcacaaagaggggc | 111 |
| CYP5A1 | 33 | intron 3 + (23375-23386) | gtgacaaattctccctctcc (T)10-12 cattgcagaagtcacatgcg | 112 |
| CYP5A1 | 34 | intron 3 + 23471 | aggacagtgccccataattt C/T ctgctccagatctatgccct | 113 |
| CYP5A1 | 35 | intron 3 + 23793 | tctacttaagttcccagaag G/A cggctgtgaactattttggt | 114 |
| CYP5A1 | 36 | intron 3 + (25690-25704) | agcattagcaatattactac (A)12-16 gaagcaggaagactcccaag | 115 |
| CYP5A1 | 37 | intron 3 + (34024-34026) | tttccatagttaatgagaag GAG/Δ aagaatttaaaacaattaat | 116 |
| CYP5A1 | 38 | intron 4 + 2446 | attttcgtcctcttccactc A/G gcacaggattaagccttcag | 117 |
| CYP5A1 | 39 | intron 4 + 4399 | gggggtgatgcaacagggag C/T tccctgacatacaacaacat | 118 |
| CYP5A1 | 40 | intron 4 + (5554-5555) | tgaggagagaggggcccttg (C) gcagacttgccataaaaccg | 119 |
| CYP5A1 | 40 | intron 4 + (5554-5555) | tgaggagagaggggcccttg gcagacttgccataaaaccg | 120 |
| CYP5A1 | 41 | intron 4 + 7453 | tgtttttagccaccacatgc C/T cctgagctgacccctcgagc | 121 |
| CYP5A1 | 42 | intron 4 + (7645-7654) | tactgttttcatttttggta (T)9-10 acattttaatttttttatac | 122 |
| CYP5A1 | 43 | intron 4 + 7756 | ggttcaaatctttgtacagc C/T agatagcaatagatataaat | 123 |

| Designation of Gene | No. | Location | Sequence | SEQ ID NO |
|---|---|---|---|---|
| CYP5A1 | 44 | intron 4 + 7770 | tacagccagatagcaataga T/C ataaattgacaatggcctca | 124 |
| CYP5A1 | 45 | intron 4 + 17600 | ccagtcaacagggcttccct T/C aggagatgttctctaaactg | 125 |
| CYP5A1 | 46 | intron 4 + (22506-22542) | acatgtggttctgagactgc TTGGTCCCCCAACCGCCTGT CTCCCCACACCCCGCAA/AGGCTGCAGTCC cctgcaggctggggacactc | 126 |
| CYP5A1 | 47 | intron 4 + 24582 | cctgctcagtagggggactg C/A agaacacacagaagccattg | 127 |
| CYP5A1 | 48 | exon 5 + 37 | tcaagtcggtagccgacagc G/A ttctgtttttacgtgacaaa | 128 |
| CYP5A1 | 49 | intron 5 + (5127-5138) | ttgtatttaaattaaaattg (T)9-12 ggcctatgtcataccaggga | 129 |
| CYP5A1 | 50 | intron 5 + (5140-5141) | aaattgtttttttttttttgg (T) cctatgtcataccagggata | 130 |
| CYP5A1 | 50 | intron 5 + (5140-5141) | aaattgtttttttttttttgg cctatgtcataccagggata | 131 |
| CYP5A1 | 51 | intron 5 + 7570 | ggcattaattttttcatttt T/A aaaaaaaacccagaaatacg | 132 |
| CYP5A1 | 52 | intron 6 + 1001 | ttttcactctagcagggaaa T/C gtgtaatcagttgtagaaac | 133 |
| CYP5A1 | 53 | intron 7 + 1203 | gggcaggtggctcagttgtg C/T catcccttctatttctcctc | 134 |
| CYP5A1 | 54 | intron 7 + 1815 | attaattgcacagatgttta T/C tgagtgcttactatgtgcca | 135 |
| CYP5A1 | 55 | intron 7 + 1922 | gggagggcaggactccaaaa C/A ggctccgattccaggcccgc | 136 |
| CYP5A1 | 56 | intron 8 + (1245-1274) | gccaccttttgagagtgttc (AC)12-13 gcatccctagcctggctgtg | 137 |
| CYP5A1 | 57 | intron 9 + 139 | tgaaatagggtcattgcttg G/C cttctcctgctctccgggtt | 138 |
| CYP5A1 | 58 | intron 9 + 5862 | caataattcagattctctac T/C gttagcagttctaaacccct | 139 |
| CYP5A1 | 59 | intron 9 + 5978 | catctctcatcccatacagc C/T ttgactgcattctcctttca | 140 |
| CYP5A1 | 60 | intron 9 + (7360-7369) | caaatctatcctcactatgg (A)9-10 tgtcttcccatgtggatctc | 141 |
| CYP5A1 | 61 | intron 9 + (7562-7563) | ttcagtcccccagccacaca (A) ggggtcagaggtgctgctga | 142 |
| CYP5A1 | 61 | intron 9 + (7562-7563) | ttcagtcccccagccacaca ggggtcagaggtgctgctga | 143 |
| CYP5A1 | 62 | intron 9 + 9085 | accctatcttggaagttaag A/T tttgtggtcactgctaccaa | 144 |
| CYP5A1 | 63 | intron 9 + 9441 | gcccatgcccacagtaaccc G/T tagggaaactgacccgctct | 145 |
| CYP5A1 | 64 | intron 9 + (11323-11338) | ggtttgtttggtgagctttc (T)13-16 aggttgtattttataactga | 146 |
| CYP5A1 | 65 | intron 9 + 13259 | accaagtggatcaatggtgg C/Δ tctggcatttctataaaggg | 147 |
| CYP5A1 | 66 | intron 9 + (17698-17707) | catgtaacattgggttttgc (T)9-10 cctcacgtaaaatccacata | 148 |
| CYP5A1 | 67 | intron 9 + (18709-18719) | caagcagtattccacagatg (A)10-12 ttataaaagcgaaaccaaaa | 149 |
| CYP5A1 | 68 | intron 9 + 20380 | aaatggaacaaaaagtttta G/T gaaacatgtcaccttctatg | 150 |
| CYP5A1 | 69 | intron 9 + 20607 | tacaaacgaggaaatgtatc G/A ggttccttaagagcctttcc | 151 |
| CYP5A1 | 70 | intron 9 + (21300-21308) | agccgcccactccgcacaca (T)8-9 ctagttgtcataaacaaatt | 152 |
| CYP5A1 | 71 | intron 9 + 22829 | tgtccctctggcttctgcag C/A ttttcctgatgtggcagggt | 153 |

| Designation of Gene | No. | Location | Sequence | SEQ ID NO |
|---|---|---|---|---|
| CYP5A1 | 72 | intron 9 + 22919 | tgtccctctagagggtgaca T/C gccttgaatggcagcgaacc | 154 |
| CYP5A1 | 73 | intron 9 + 23463 | ccccatagtgtgtggaccat T/C gggggcctagaactgaggtc | 155 |
| CYP5A1 | 74 | intron 9 + 25072 | ggtttaactgcattttactc T/G ttccagcctgtctggccatt | 156 |
| CYP5A1 | 75 | intron 9 + 25641 | ttgcagtccagcccaaagag G/A cctgtcccagcaggacgcca | 157 |
| CYP5A1 | 76 | intron 9 + 25763 | tgccaaacttttaaactcag G/T ggaattccgcaaaaatctag | 158 |
| CYP5A1 | 77 | intron 9 + 26532 | gagaggcaggttcacccagc T/C gtctgtgcccgggcagggca | 159 |
| CYP5A1 | 78 | intron 9 + 26542 | ttcacccagctgtctgtgcc C/T gggcagggcaactcccgaag | 160 |
| CYP5A1 | 79 | intron 9 + 26792 | ttggcctggggacagttttt C/G agcgactgctctttgagaag | 161 |
| CYP5A1 | 80 | intron 9 + 28687 | cacagagagcgtgcgctcca A/T taaaacggcactaaatggca | 162 |
| CYP5A1 | 81 | intron 9 + 28989 | ccagggactgtgcatcaccc G/A tctccaccccaccccagaac | 163 |
| CYP5A1 | 82 | intron 9 + 29606 | tggaatggcctttctctccc C/A caacgcccagccaagggagg | 164 |
| CYP5A1 | 83 | intron 9 + 32268 | tctgagcccagacaatggct T/C atttatttcagacaatattc | 165 |
| CYP5A1 | 84 | intron 9 + 32429 | ggccttattaataaatccat C/T ggtggtctgctgaaaatgct | 166 |
| CYP5A1 | 85 | intron 9 + 32912 | tcctacagcaaatcatcaac A/G agatgcacacgaagatgagg | 167 |
| CYP5A1 | 86 | intron 9 + 33183 | ccttttcagatgctccataa C/A ctcgtgtgaaaagccccggt | 168 |
| CYP5A1 | 87 | intron 9 + 34811 | ggatctgtggggtgagtgaa C/T aaaagcattttctctgcaaa | 169 |
| CYP5A1 | 88 | intron 9 + 36495 | gggttctgtccccttctaaa G/A caggtataaatagatgccat | 170 |
| CYP5A1 | 89 | intron 9 + 38683 | aggccaaggtcaaggggagg C/G gctgcaggggggcttcctc | 171 |
| CYP5A1 | 90 | intron 9 + 43855 | atcccagtgcgcccacaccc T/C gctcaagagctcatgggcac | 172 |
| CYP5A1 | 91 | exon 10 + 25 | cccctgagttctgcagcctc G/A aggaaggcctgccctatctg | 173 |
| CYP5A1 | 92 | intron 10 + 3166 | gctgacacaacttggactta T/C tcataaaagaaaatcagaat | 174 |
| CYP5A1 | 93 | exon 12 + 138 | ccacgtgctgcacaagttcc G/A gttccaagcctgccctgaga | 175 |
| CYP5A1 | 94 | intron 12 + 1073 | gaaacgggggtcctcaccca T/G atcctgcccactcccaagct | 176 |
| CYP5A1 | 95 | intron 12 + 1806 | actcagctccagaggaaacc C/G aactcctcgagtaattcttt | 177 |
| CYP5A1 | 96 | intron 12 + 2033 | tggggaggatcagcagggac G/A gctggagtcactgaaagata | 178 |
| CYP5A1 | 97 | 3'flanking + 1402 | tcctgtggccctccccacac C/T cttgttgccaccacctgccc | 179 |
| CYP7A1 | 1 | 5'flanking - 469 | gaaacatgaagcagcagaaa C/T gtttttcccagttctctttc | 180 |
| CYP7A1 | 2 | 5'flanking - 203 | gtcaacatatatttgagaga C/A cttcaacttatcaagtattg | 181 |
| CYP7A1 | 3 | 5'flanking - (114-105) | tcaaggccagttactaccac (T)9-10 ctaatagaatgaacaaatgg | 182 |
| CYP7A1 | 4 | intron 1 + 512 | ttctattactgtttttaaat C/A aatgttaatcaactgtggtg | 183 |
| CYP7A1 | 5 | intron 1 + 607 | gttcaagaatgcaagaaaaa C/T aaatacagtcagatccagaa | 184 |

| Designation of Gene | No. | Location | Sequence | SEQ ID NO |
|---|---|---|---|---|
| CYP7A1 | 6 | intron 1 + 611 | aagaatgcaagaaaaacaaa T/C acagtcagatccagaaccat | 185 |
| CYP7A1 | 7 | intron 1 + 1184 | gaaaaatgatgcttagcaaa G/A tgataaacactagaatgtag | 186 |
| CYP7A1 | 8 | intron 2 + 770 | aaaacattatcaattagttt A/G tgtgcaatagctgtaaataa | 187 |
| CYP7A1 | 9 | intron 2 + 782 | attagtttatgtgcaatagc T/C gtaaataagtgcagtagcat | 188 |
| CYP7A1 | 10 | exon 3 + 377 | catgttcaggactgcgcaca A/G tgcccgggagaaactggcag | 189 |
| CYP7A1 | 11 | exon 4 + 131 | tgaatgacctgccagtatta G/A gtgggtatacttgttatgtt | 190 |
| CYP7A1 | 12 | intron 5 + (249-257) | catgataataaacctgccac (T)8-9 cttaaaaagcacctcctccc | 191 |
| CYP7A1 | 13 | intron 5 + 471 | agtatttgctaaatcactaa T/C ggggtagaattaaaaagaaa | 192 |
| CYP7A1 | 14 | intron 5 + 566 | ttaaaggcggttttctttgt A/G tttttattgcagacttttaa | 193 |
| CYP7A1 | 15 | exon 6 + 758 | cttagtgagatcccgtctcc G/A aagaaaagatatgtattcta | 194 |
| CYP7B1 | 1 | 5'flanking - 595 | ccttattctttctgagtaca A/G cctgtagtacttgaaccact | 195 |
| CYP7B1 | 2 | intron 1 + 19 | aggtaagcgcctcggccgcc A/T cgacgcatgcgccctgggcc | 196 |
| CYP7B1 | 3 | intron 1 - (19-9) | ttttcttttgttcttttatc (T)9-12 ccttgtaggagacccggtga | 197 |
| CYP7B1 | 4 | intron 2 + (8107-8117) | gaactcatattttctctctc (T)10-11 ctaggaaagtacataacatt | 198 |
| CYP7B1 | 5 | intron 3 + (158-171) | ataaccctctataattgata (T)12-14 gctgaggtggtaaaacaaga | 199 |
| CYP7B1 | 6 | intron 4 + 2144 | gcatgaagtatatgtacaaa G/A gcaagacttacacagttaaa | 200 |
| CYP7B1 | 7 | intron 4 + 2280 | aaaaagtttaaacttttatc A/C ttataagaggaaaaaagatt | 201 |
| CYP7B1 | 8 | intron 4 + 2626 | tcttgttaagattctgctta T/C gacctcagtaaaacatttta | 202 |
| CYP7B1 | 9 | intron 4 + 2841 | tcagaaaaaaatctggggtg G/T aggtaggatttagaactgcg | 203 |
| CYP7B1 | 10 | intron 4 + (7610-7627) | atttataagtatatggaaag (A)16-19 aattctagaacgaattgaga | 204 |
| CYP7B1 | 11 | intron 4 + (7846-7851) | cctcatgtgtgagaaggggg (A)6-7 tgagggtcttgttcatttgt | 205 |
| CYP7B1 | 12 | intron 4 + 8004 | ggaagtcctcccagggccca G/T ctggtctgtcagtggcactg | 206 |
| CYP7B1 | 13 | intron 4 + 8217 | ccattgttgataataacgca A/G ctcattacttttgtcgtcta | 207 |
| CYP7B1 | 14 | intron 4 + 8267 | ggcatgatggctcagaacga G/C agccaacctcatctcgcaca | 208 |
| CYP7B1 | 15 | intron 4 + 8417 | aattgtcatttgtgatactg T/C ggtgggcgcaagctgccatg | 209 |
| CYP7B1 | 16 | intron 4 + 9226 | ccatttgttatcatgtgaag G/C gggagaaaactccatttggg | 210 |
| CYP7B1 | 17 | intron 5 + 6213 | aagctcacttagactgattt G/T ggttaggtagatagaggacc | 211 |
| CYP7B1 | 18 | intron 5 + 6224 | gactgatttgggttaggtag A/G tagaggaccagtctcctcag | 212 |
| CYP7B1 | 19 | intron 5 + 6629 | aaatgaggacaaacctaata G/C ttttaaaggaaatggttcac | 213 |
| CYP7B1 | 20 | intron 5 + 7545 | aagtcatacagcttttcaga T/C ttcactttgatatgggagaa | 214 |
| CYP7B1 | 21 | exon 6 + 337 | atcaaaattaccctaaacat C/T ctaagctcatctatttcttt | 215 |

In Table 1, the "Designation of Gene" column shows the designations of the genes encoding drug metabolizing enzymes (CYP). The nucleotides expressed with capital letters in the "Sequence" column are the polymorphic information. The two nucleotides on both sides of the mark "/" represent a homozygous or heterozygous SNP. For example, "A/G" means that the allele is A/A or G/G homozygote or A/G heterozygote. The sequences in this Table basically represent 20 nucleotides each before and after the SNP. However, the nucleotide in parentheses [e.g. (C) in No. 40 of CYP5A1: SEQ ID NO: 119] represents a polymorphism caused by insertion. The mark "?" (e.g. see No. 21 of CYP5A1: SEQ ID NO: 100) means a polymorphism caused by deletion of one or more nucleotides. The nucleotide in parentheses provided with a number means that the nucleotide is repeated that number of times. For example, "(T) 9-11" appearing in SEQ ID NO: 66 (Table 1, No. 16 of CYP2E) means a sequence where T is repeated from 9 to 11 times. It should be noted that the term "21st (position 21)" used in explaining locational relations in the nucleotide sequence described in the "Sequence" column in Table 1 means the location of a genetic polymorphism site. Therefore, in the case of deletion SNP (?), the deleted, imaginary nucleotide is the "21st". In the case where a plurality of nucleotides are present at the polymorphic site, a group of those nucleotides is the "21st". For example, in the case of No. 15 of CYP2E (SEQ ID NO: 65), the polymorphic site "TGT" is the 21st; in the case of No. 16 of CYP2E (SEQ ID NO: 66), the polymorphic site "(T) 9-11" is the 21st.

The "Location" in the Table shows the location of SNP in the genome. The locations of SNPs in 5' flanking region, intron regions and 3' flanking region are expressed taking the nucleotide located 1 bp upstream of the 5' utmost end nucleotide of exon 1 as -1 position. Then, nucleotides are numbered -2, -3, -4, ... toward the 5' end of the gene. An intron region means a region spanning from a nucleotide positioned 1 bp downstream of the 3' end of an exon to the subsequent exon. The number of an intron is the same as the number of the exon located 5' to the relevant intron. For example, an intron which exists between exon 3 and exon 4 (i.e. the intron located 3' to exon 3) is called intron 3. The locations of SNPs in an intron region are counted taking the first nucleotide located immediately 3' to the exon/intron junction located 5' to the relevant intron as position 1 of the nucleotide sequence of the intron. Numbers with "+" mark or without any mark in the "Location" column mean that they are counted toward the 3' end of the gene; numbers with "-" mark mean that they are counted toward the 5' end of the gene. For example, if the third nucleotide counted from exon 3/intron 3 junction toward the 3' end of the gene is polymorphic, the location is expressed as "intron 3, +3". Similarly, if the fifth nucleotide counted from intron 3/exon 4 junction toward the 5' end of the gene is polymorphic, the location is expressed as "intron 3, -5". The region located 3' to the final exon is designated 3' flanking region. The locations of SNPs in this region are counted taking the nucleotide located 1 bp downstream of the 3' end nucleotide of the final exon as position 1.

The numbers appearing in the "No." column in Table 1 correspond to the numbers appearing in respective gene maps (Figs. 9-16) which show the locations of SNPs. In Figs. 9-16, accession numbers of polymorphisms in public genome databases are also provided. By using information given in Table 1, Figures and databases, sequences adjacent to polymorphisms can be specified. Such information is useful, for example, in preparing PCR primers adjacent to a polymorphism.

Examples of the above-mentioned genome databases include, but are not limited to, the list of services provided by IMS-JST JSNP database website (Laboratory for Genotyping, SNP Research Center, RIKEN, Japan).

5. Preparation of Oligonucleotide Probes or Oligonucleotide Primers

Oligonucleotides which are used in the detection method of the present invention as primers and/or probes may be prepared based on the nucleotide sequences described in Table 1 (SEQ ID NOS: 1-215). For example, when SNPs are to be detected, these sequences per se may be synthesized, or primers and/or probes may be designed and synthesized so that they contain a part of these sequences. However, it should be noted here that the nucleotide sequences of such primers or probes must contain an genetic polymorphism (the portion indicated in capital letters in the "Sequence" column in Table 1) The present invention also includes complementary strands to such sequences.

Taking SNP as an example for the purpose of illustration, a primer or probe is designed so that an SNP site is located at the 3' or 5' end of the nucleotide sequence of the primer or probe; or a primer or probe is designed so that an SNP site is located at the 3' or 5' end of the sequence complementary to its nucleotide sequence; or a primer or probe is designed so that an SNP site is located within four nucleotides, preferably two nucleotides, from the 3' or 5' end of its nucleotide sequence or the sequence complementary thereto. Alternatively, a primer or probe is designed so that an SNP site is located at the center of the full-length sequence of the oligonucleotide. The "center" refers to a central region where the number of nucleotides counted from there toward the 3' end and the number of nucleotides counted from there toward the 5' end are almost equal. If the number of nucleotides of the oligonucleotide is an odd number, the "center" is the central five nucleotides, preferably the central three nucleotides, more preferably the single nucleotide at the very center. For example, if the oligonucleotide consists of 41 nucleotides, the "center" is from position 19 to position 23 nucleotides, preferably from position 20 to position 22 nucleotides, more preferably the nucleotide at position 21. If the number of nucleotides of the oligonucleotide is an even number, the "center" refers to the central four nucleotides, preferably the central two nucleotides. In the nucleotide sequences shown in the "Sequence" column in Table 1, if the polymorphism is deletion polymorphism, the actual length of such sequences is 40, an even number. Therefore, if an oligonucleotide consisting of 40 nucleotides is designed based on such sequences, the "center" is from position 19 to position 22 nucleotides, preferably the nucleotide at position 20.

When a polymorphic site is composed of a plurality of nucleotides, a probe or primer is designed and prepared so that the entire or a partial sequence of the polymorphic site or a sequence complementary thereto is contained in the nucleotide sequence of the probe or primer. When the thus prepared oligonucleotide is used as a probe, it is possible to determine alleles using the presence or absence of hybridization or difference of hybridization. Hereinbelow, those nucleotides in a probe or primer DNA which form a complementary strand with the polymorphic site or its peripheral site are called "corresponding nucleotides". A probe or primer may be designed so that corresponding nucleotides are located on any nucleotide(s) of the sequence constituting a polymorphism. The "peripheral site" means a region one to three nucleotides outside (5' side) of the 5' utmost end of the sequence constituting a polymorphism, or a region one to three nucleotides outside (3' side) of the 3' utmost end of the sequence constituting a polymorphism. In particular, the corresponding nucleotides in a probe or primer can be designed so that the 5' or 3' end nucleotide when forming a complementary strand is located on the 5' terminal side, 3' terminal side, or the center of the sequence constituting a polymorphism. In the present invention, it is preferred that the above-mentioned 5' or 3' end nucleotide is located on the center of the sequence constituting a polymorphism. It is also possible to design corresponding nucleotides so that they are located on a peripheral region of the sequence constituting a polymorphism.

For example, when an invader probe and allele probes are prepared to detect the genetic polymorphism (TGT) as shown in No. 15 of CYP2E (SEQ ID NO: 65) in Table 1 by the invader method described later, allele probes are designed so that nucleotides complementary to the polymorphic site TGT (i.e. A, C or A in the 5' to 3' direction) are the corresponding nucleotides of the allele probes and hybridize (see panels a to c in Fig. 4B). For example, in panel (a) of Fig. 4B, the 5' utmost, corresponding nucleotide "C" forming a complementary strand is located on the center of the nucleotides (TGT) constituting a polymorphism; in panel (b) of Fig. 4B, the 5' utmost, corresponding nucleotide "A" forming a complementary strand is located on "T" of the nucleotides (TGT) constituting a polymorphism. In panel (d) of Fig. 4B, an allele probe is shown which is designed so that its corresponding nucleotides are located on the peripheral region (three nucleotides) of the polymorphic site (the underlined portion).

An invader probe is designed so that the position of its 3' end nucleotide "N" (any one of A, T, C or G) corresponds to the position of any of the nucleotides of the polymorphic site (TGT) when hybridized. However, it is most effective to design an invader probe so that there is an overlap of one nucleotide between the invader probe and the allele probe (Fig. 4C). On the other hand, in order to detect another polymorphism (i.e. deletion of TGT), the invader probe and allele probe may be designed so that a nucleotide located one to three nucleotides upstream or downstream of the deletion site will be the overlapping nucleotide taking into consideration of the deletion of TGT (i.e. deleting from the sequence). Panel (e) of Fig. 4B shows an allele probe which is designed so that the two nucleotides of the both sides of the deletion site hybridize thereto. With respect to TaqMan probes, they may be designed so that any of these nucleotides TGT or the deletion site is located at the center of the probe.

The length of the nucleotide sequence of the oligonucleotide is at least 13 nucleotides, preferably 13 to 60 nucleotides, more preferably 15 to 40 nucleotides, and most preferably 18-30 nucleotides. This oligonucleotide sequence may be used as a probe for detecting a target gene, and it may be used as either a forward (sense) primer or a reverse (antisense) primer.

The oligonucleotide used in the invention may be an oligonucleotide composed of two regions connected in tandem, one region being hybridizable to the genomic DNA and the other region being not hybridizable thereto. The order of connection is not particularly limited; either region may be located upstream or downstream. The hybridizable region of this oligonucleotide is designed based on the information on SNP-containing sequences described in Table 1. The oligonucleotide is prepared so that the sequence located at the 5' or 3' utmost end of the region hybridizable to the genomic DNA includes the SNP. The region of the above oligonucleotide not hybridizable to the genomic DNA is designed at random so that it does not hybridize to the SNP-containing sequence described in Table 1. This oligonucleotide may be used as a probe mainly for detecting SNPs in the invader method.

Further, the primer used in the present invention is designed so that a nucleotide sequence given in Table 1 contains an SNP when amplified by PCR for the purposes of examining functional changes resulted from the SNP, judging the efficiency or non-efficiency of drugs, and examining the occurrence of side effect. The length of the primer is designed so that at least 15 nucleotides, preferably 15 to 30 nucleotides, more preferably 18 to 24 nucleotides are contained in the primer. The primer sequence is appropriately selected from the template DNA so that the amplified fragment has a length of 1000 bp or less, preferably within 500 bp (e.g. 120 to 500 bp), more preferably within 200 bp (120 to 200 bp).

For example, primers are designed so that at least one of the sense or the antisense primer is contained in a region within 1000 bp, preferably 200 bp, more preferably 100 bp counted from the nucleotide immediately adjacent to 5' or 3' to the SNP toward the 5' or 3' end of the gene, respectively.

The thus designed oligonucleotide primers or probes may be synthesized chemically according to known techniques. Usually, such primers or probes are synthesized with a commercial chemical synthesizer.

It is also possible to label probes with fluorescent substances (e.g. FAM, VIC, Redmond Dye, etc.) in advance to thereby automate detection procedures.

6. Kit

The above-described oligonucleotide may be included in a genetic polymorphism detection kit. The genetic polymorphism detection kit of the present invention comprises one or more components necessary for practicing the present invention. For example, the detection kit of the present invention comprises components to preserve or supply enzymes and/or reaction components necessary for performing cleavage assay (e.g. invader assay). The detection kit of the present invention comprises any and all components enzymes or components necessary (suitable) for an intended assay. Examples of such components include, but are not limited to, oligonucleotides, polymerases (e.g. Taq polymerase), buffers (e.g. Tris buffer), dNTPs, control reagents (e.g. tissue samples, target oligonucleotides for positive and negative controls, etc.), labeling and/or detection reagents (fluorescent dyes such as VIC, FAM), solid supports, manual, illustrative diagrams and/or product information, inhibitors, and packing environment adjusting agents (e.g. ice, desiccating agents). The detection kit of the present invention may be a partial kit which comprises only a part of the necessary components. In this case, users may provide the remaining components. The detection kit of the present invention may comprise two or more separate containers, each containing a part of the components to be used. For example, the kit may comprise a first container containing an enzyme and a second container containing an oligonucleotide. Specific examples of the enzyme include a structure-specific cleaving enzyme contained in an appropriate storage buffer or a container. Specific examples of the oligonucleotide include invader oligonucleotides, probe oligonucleotides, target oligonucleotides for use as control, and the like. Alternatively, ore or more reaction components may be provided in such a manner that they are pre-divided into portions of a specific amount. Since such a kit contains components which have already been quantitatively determined for use in one step of the method of the present invention, it is not necessary to remeasure or re-divide. Selected reaction components may also be mixed and divided into portions of a specific amount. It is preferred that reaction components should be pre-divided into portions and contained in a reactor. Specific examples of the reactor include, but are not limited to, reaction tubes or wells, or microtiter plates. It is especially preferable that the pre-divided reaction component should be kept dry in a reactor by means of, for example, dehydration or freeze drying.

7. Detection

A gene encoding a drug metabolizing enzyme using DNA polymerase (template DNA) is amplified using the oligonucleotide prepared as above as a primer. Alternatively, the probe prepared as above is hybridized with a template DNA to detect a target DNA with a gene polymorphism. The template DNA can be prepared by a known technique, for example, cesium chloride density-gradient ultracentrifugation, SDS dissolution, or phenol-chloroform extraction.

(1) Detection Using PCR

Amplification may be performed by polymerase chain reaction (PCR). Specific examples of useful DNA polymerase include LA Taq DNA polymerase (Takara), Ex Taq polymerase (Takara), Gold Taq polymerase (Perkin Elmer), AmpliTaq (Perkin Elmer), Pfu DNA polymerase (Stratagene) and the like.

Amplification conditions are as follows. Denaturation step at 85-105°C for 10-40 seconds, preferably at 94°C for 20-30 seconds; annealing step at 50-72°C for 20 seconds to 1 minute, preferably at 60°C for 20 seconds to 1 minutes; and extension step at 65-75°C for 1-4 minutes, preferably at 72°C for 2-3 minutes constitute one cycle, and 30 to 40 cycles are performed. However, in order to denature the template DNA and the primers sufficiently, a denaturation step of at 95°C for 1-5 minutes [if Gold Taq polymerase (Perkin Elmer) is used, at least 8-15 minutes, preferably 10-12 minutes] may be added before the start of the above-described amplification cycles. Also, in order to extend the amplified DNA completely, an extension step of at 72°C for 1-5 minutes for 1-10 minutes may be added after the above amplification cycles. If the detection of the amplified product is not performed immediately, it is desirable to add a step of storing the amplified product at 4°C to avoid unspecific amplification. Thus, a gene encoding drug metabolizing enzyme (CYP) can be amplified.

Subsequently, the amplified product is subjected to agarose gel electrophoresis, followed by staining with ethidium bromide, SYBR Green solution or the like to thereby detect the amplified product as a band or two to three bands (DNA fragments). Thus, a part of a gene encoding drug metabolizing enzyme, containing a genetic polymorphism can be detected as a DNA fragment. Instead of agarose gel electrophoresis, polyacrylamide gel electrophoresis or capillary electrophoresis may be performed. It is also possible to perform PCR using primers labeled with a substance such as fluorescent dye and to detect the amplified product. A detection method which does not require electrophoresis may also be employed; in such a method, the amplified product is bound to a solid support such as a microplate, and a DNA fragment of interest is detected by means of fluorescence, enzyme reaction, or the like.

(2) Detection by TaqMan PCR

TaqMan PCR is a method using PCR reaction with fluorescently labeled allele-specific oligos and Taq DNA polymerase. The allele-specific oligo used in TaqMan PCR (called "TaqMan probe") may be designed based on the SNP information described above. The 5' end of TaqMan probe is labeled with fluorescence reporter dye R (e.g. FAM or VIC), and at the same time, the 3' end thereof is labeled with quencher Q (quenching substance) (Fig. 1). Under these conditions, fluorescence is not detectable since the quencher absorbs fluorescence energy. Since the 3' end of TaqMan probe is phosphorylated, no extension reaction occurs from TaqMan probe during PCR Fig. 1). However, when PCR is performed using this TaqMan probe together with Taq DNA polymerase and primers designed so that an SNP-containing region is amplified, the reaction described below occurs.

First, a TaqMan probe hybridizes to a specific sequence in the template DNA (Fig. 2a), and at the same time, an extension reaction occurs from a PCR primer (Fig. 2b). At this time, Taq DNA polymerase having 5' nuclease activity cleaves the hybridized TaqMan probe as the extension reaction proceeds. When the TaqMan probe has been cleaved, the fluorescent dye becomes free from the influence of the quencher. Then, fluorescence can be detected (Fig. 2c).

For example, as shown in Fig. 3, two alleles are supposed: one allele has A at the SNP site (allele 1) and the other allele has G at the SNP site (allele 2). A TaqMan probe specific to allele 1 is labeled with FAM and another TaqMan probe specific to allele 2 is labeled with VIC (Fig. 3). These two allele specific oligos are added to PCR reagents, and then TaqMan PCR is performed with a template DNA whose SNP is to be detected. Subsequently, fluorescence intensities of FAM and VIC are determined with a fluorescence detector. When the SNP site of the allele is complementary to the site within TaqMan probe corresponding to the SNP, the probe hybridizes to the allele; and Taq polymerase cleaves the fluorescent dye of the probe, which becomes free form the influence of the quencher. As a result, fluorescence intensity is detected.

If the template is a homozygote of allele 1, strong fluorescence intensity of FAM is recognized but the fluorescence of VIC is hardly recognized. If the template is a heterozygote of allele 1 and allele 2, fluorescence of both FAM and VIC can be detected.

(3) SNP Detection by the Invader Method

The invader method is a method for detecting SNPs by hybridizing allele-specific oligos to the template. In the invader method, two unlabeled oligos and one fluorescently labeled oligo are used. One of the two unlabeled oligos is called an "allele probe". The allele probe is composed of a region which hybridizes to the genomic DNA (template DNA) to form a complementary double strand, and a region (called "flap") which has a sequence entirely unrelated to the sequence of the template DNA and thus does not hybridize to the genomic DNA. A nucleotide located at the 5' or 3' utmost end of the hybridizable region corresponds to the SNP (panel (a) in Fig. 4A). The above-described flap sequence is an oligonucleotide having a sequence complementary to a FRET probe described later. The other unlabeled oligo is called an "invader probe". This oligo is designed so that it hybridizes complementarily to the template DNA from the SNP site toward the 3' end of the genomic DNA (panel (b) in Fig. 4A). However, the nucleotide corresponding to the SNP ("N" in panel (b) in Fig. 4A) may be any nucleotide. Thus, when the genomic DNA (the template) is hybridized to the above-described two probes, one nucleotide (N) of the invader probe invades into the SNP site (panel (c) in Fig. 4A). As a result, a triple strand is formed at the SNP site.

On the other hand, the fluorescently labeled oligo has a sequence completely unrelated to the allele. This sequence is common regardless of the types of SNPs. This probe is called a "FRET" probe (fluorescence resonance energy transfer probe) (Fig. 5). The nucleotide at the 5' end of FRET probe (reporter) is labeled with fluorescent dye R, while quencher Q is linked upstream of the reporter. Therefore, under these conditions, the quencher absorbs the fluorescent dye and no fluorescence is detectable. A certain region of the FRET probe starting from the 5' end reporter nucleotide (designated "region 1") is designed so that it is complementary to a certain region of the probe located 3' to region 1 (designated "region 2") when region 1 and region 2 are faced with each other. Therefore, region 1 and region 2 form a complementary strand within the FRET probe (Fig. 5). Also, the region located downstream of this complementary strand forming region is designed so that it hybridizes to the flap of the allele probe to thereby form a complementary strand_(Fig. 5).

In the invader method, an enzyme called cleavase is used which is one of 5' nucleotidases having a unique endonuclease activity of cleaving upon recognition of a special structure of DNA. Cleavase is an enzyme which cleaves the allele probe at a point immediately 3' to the SNP site when the genomic DNA, the allele probe and the invader probe form a triple strand at the SNP site. Therefore, when three nucleotides form a triple strand as shown in panel (c) in Fig. 4A, cleavase recognizes the 5' flap and cuts off this flap. As a result, the structure of this SNP site is recognized by cleavage (panel (a) in Fig. 6), and the allele probe is cut at the site of its flap to liberate the flap (panel (b) in Fig. 6). Subsequently, the flap liberated from the allele probe complementarily binds to the FRET probe since it has a sequence complementary to the FRET probe (panel (c) in Fig. 6). At this time, the SNP site of the flap invades into the portion of the FRET probe which has already formed a double strand. Cleavase again recognizes this structure and cuts off the nucleotide labeled with the fluorescent dye. The thus cleaved fluorescent dye becomes free from the influence of the quencher and emits fluorescence (panel (d) in Fig. 6). When the SNP in the genomic DNA does not match the nucleotide corresponding to the SNP in the allele probe, a specific DNA structure recognizable by cleavase is not formed as seen in Fig. 7. Thus, the allele probe is not cleaved and no fluorescence is detected.

For example, when an SNP to be detected is T/C, an invader probe and an allele probe for T, and a FRET probe with a FAM-linked reporter corresponding to the SNP are prepared. Separately, an invader probe and an allele probe for C, and a FRET probe with a VIC-linked reporter corresponding to the SNP are prepared are also prepared. Then, all of them are mixed to carry out SNP detection. As a result, if the SNP of a subject is T/T homozygous, the fluorescence of FAM is emitted; if the SNP of a subject is C/C homozygous, the fluorescence of VIC is emitted; and if the SNP of a subject is T/C heterozygous, the fluorescence of both FAM and VIC is emitted. Since FAM and VIC have different fluorescent wavelengths, they can be discriminated. Products labeled with fluorescent dyes can be detected with a fluorescent plate reader or an apparatus for collecting fluorescence data generated during reaction (real-time fluorescence detector). Specific examples of real-time fluorescence detector include ABI 7700 sequence detection system (Applied Biosystems).

(4) Detection by SniPer Method

In order to detect SNPs by SniPer method, it is possible to discriminate alleles by examining the presence or absence of amplification by RCA. Briefly, the genomic DNA to be used as a template is linearized. Then, a probe is hybridized to this genomic DNA. When the probe sequence and the sequence of the genomic DNA are complementary to each other and form a double strand, the genomic DNA can be converted into a circular DNA through ligation reaction. As a result, RCA of the circular DNA proceeds. On the other hand, when the ends of the probe do not match with the genomic DNA, the DNA is not ligated to become a circular DNA. Thus, RCA reaction does not proceed. Therefore, in Sniper method, a single-stranded probe which anneals with the genomic DNA and is circularizable is designed. This single-stranded probe is called a padlock probe. The sequences of the two ends of this padlock probe are designed so that they correspond to the SNP to be detected. Then, this padlock probe and the genomic DNA are mixed for ligation. If the two ends of the padlock probe and the SNP site of the genomic DNA are complementary to each other, the two ends of the padlock probe are joined by ligation, yielding a circular probe. If the two ends of the padlock probe and the SNP site of the genomic DNA are not complementary to each other, the probe does not become circular. Therefore, only those padlock probes which are complementary to the SNP to be detected become circular and are amplified by DNA polymerase. By detecting the presence or absence of this amplification, SNP may be detected. For the detection, synthetic oligonucleotides which have a fluorescent dye and a quencher at their respective ends and also have a hairpin structure are used.

(5) Detection by MALDI-TOF/MS Method

MALDI-TOF/MS (Matrix Assisted Laser Desorption-Time of Flight/Mass Spectrometry) is a method using a mass spectrometer in SNP typing. This method is composed of the following steps.

(i) PCR Amplification and Purification of SNP-Containing DNA Fragments

PCR primers are designed so that there is no overlapping between them and the nucleotides of SNP site. Then, DNA fragments are amplified. The amplified fragments are purified from the amplification reaction product by treatment with exonuclease, alkaline phosphatase, etc. to remove primers, dNTPs, etc.

(ii) Primer Extension (Thermal Cycling) and Purification

Ten-fold or more primers are added to the template of the target region (which is the PCR product), and primer extension is performed by thermal cycling. The primers used here are designed so that their 3' ends are adjacent to the nucleotide of the SNP site. The length of the primer is 15 to 30 nucleotides, preferably 20 to 25 nucleotides. When multiplex reaction is performed, a sequence not complementary to the template is added to the 5' end. Thermal cycling is performed between the two temperatures of at 85-105°C (preferably 94°C) and at 35-40°C (preferably 37°C) for 20 to 30 cycles (preferably 25 cycles). The resultant reaction products are purified with a purification kit or the like to make them fit for mass spectrometer.

(iii) Mass Spectrometry of DNA with Mass Spectrometer

The purified extension reaction product is applied to a mass spectrometer to determine the mass of the purified product. Briefly, the purified product is mixed with a matrix, and 0.5-1.0 il of the mixture is spotted on MALDI plate. After drying the plate, laser light is applied to the sample to prepare spectrograms.

(6) Detection by DNA Sequencing Method

In the present invention, polymorphisms may be detected by using single nucleotide extension reactions. Briefly, four types of dideoxynucleotides labeled with different fluorescent compounds are added to a reaction system containing a gene of interest. Then, single nucleotide extension reactions are performed. In this case, the nucleotide to be extended is the polymorphic site. Also, two reactions of DNA synthesis termination and the fluorescent labeling of the 3' end of DNA molecules are operated. Four types of reaction solutions are subjected to electrophoresis on the same lane of a sequencing gel or on capillary. Difference in the fluorescent dyes used for labeling is detected with a fluorescence detector to thereby sequence the DNA band. Alternatively, the one-nucleotide extended oligonucleotide is examined with a fluorescence detection system or a mass spectrometry system to thereby determine which nucleotide was extended using the difference in the fluorescent dyes. Instead of fluorescently labeled dideoxynucleotides, primers may be fluorescently labeled and used with unlabeled dideoxynucleotides.

(7) Detection in DNA Microarray

DNA microarrays are solid supports onto which nucleotide probes are immobilized, and they include DNA chips, gene chips, microchips, beads arrays, and the like. As a specific example of DNA microarray (e.g. DNA chip) assay, GeneChip assay (Affymetrix; US Patent Nos. 6,045,996; 5,925,525; and 5,858,659) may be given. GeneChip technology uses small sized, high density microarrays of oligonucleotide probes affixed to chips. Probe arrays are manufactured, for example, by the light irradiation chemical synthesis method (Affymetrix) which is a combination of solid chemical synthesis and photolithography production technology used in the semiconductor industry. High density arrays can be constructed by using photolithography masks in order to make the boundary of the chemical reaction site of chips definite and by performing a specific chemical synthesis step. Multi-probe arrays are synthesized simultaneously on a large glass baseboard. Subsequently, this baseboard is dried, and individual probe arrays are packed in injection-molded plastic cartridges. This cartridge protects the array from the outer environment and also serves as a hybridization chamber.

First, a polynucleotide to be analyzed is isolated, amplified by PCR, and labeled with a fluorescent reporter group. Then, the labeled DNA is incubated with an array using a fluid station. This array is inserted into a scanner to detect a hybridization pattern. Hybridization data are collected as luminescence from fluorescent reporter group bound to the probe array (i.e. taken into the target sequence). Generally, probes which completely matched with the target sequence generate stronger signal than those probes which have portions not matching with the target sequence. Since the sequences and locations of individual probes on the array are known, it is possible to determine the sequence of the target polynucleotide reacted with the probe array on the basis of complementation.

In the present invention, it is also possible to use DNA microchips with electrically captured probes (Nanogen; see, for example, US Patents Nos. 6,017,696; 6,068,818; and 6,051,380). By using microelectronics, the technology of Nanogen is capable of transferring charged molecules to and from specific test sites on semidonductor microchips and concentrating them. DNA capturing type probes specific to certain SNPs or variations are arranged on specific sites on microchips or assigned addresses. Since DNA is strongly negatively charged, it is capable of moving electronically to a positively charged area.

First, the test site or a row of the test site on a microchip is electronically activated with positive charge. Then, a solution containing DNA probes is introduced onto the microchip. Since negatively charged probes quickly moves to a positively charged site, probes are concentrated at this site on the microchip and chemically bind thereto. This microchip is washed, and another DNA probe solution is added to the microchip to allow specific binding of DNA probes to the chip.

Subsequently, whether the target DNA molecule is present in a test sample or not is analyzed. This analysis is performed by judging the type of the DNA capturing probe which has hybridized to a complementary DNA in a test sample. As a test sample, a gene of interest which has been amplified by PCR may be given. By using electric charge, target molecules may be moved to one or more test sites on the microchip and concentrated. As a result of electronic concentration of the sample DNA at each test site, the hybridization between the sample DNA and a capturing probe complementary thereto is performed quickly. For example, as a result of these operations, hybridization occurs in several minutes. In order to remove unbound DNA or non-specifically bound DNA from each test site, the polarity or electric charge of the site is converted to negative charge to thereby return the unbound DNA or non-specifically bound DNA into the solution. In this method, specific binding can be detected, for example, with a fluorescence scanner utilizing laser.

Further, in the present invention, it is also possible to use an array technology utilizing fluid separation phenomenon on a plane surface (chip) because of difference in surface tensions (ProtoGene; see, for example. US Patents Nos. 6,001,311; 5,985,551; and 5,474,796). The technology of ProtoGene is based on a fact that fluids are separated from each other on a plane surface because of difference in surface tensions given by chemical coating. Since oligonucleotide probes may be separated based on the above-mentioned principle, it is possible to synthesize probes directly on a chip by the ink-jet printing of a reagent containing probes. An array having reaction sites defined by surface tension is mounted on X/Y movable stage located under one set (4) of piezoelectric nozzles. Each piezoelectric nozzle contains four standard nucleotides. This movable stage moves along each row of the array to supply an appropriate reagent (e.g. amidite) to each reaction site. The entire surface of the array is soaked in a reagent common to the test sites in the array and then in a washing solution. Subsequently, the array is rotated to remove these solutions.

DNA probes specific to the SNPs or variations to be detected are affixed to a chip using the technology of Protogene. Then, the chip is contacted with PCR-amplified gene of interest. After hybridization, unbound DNA is removed, followed by detection of hybridization using an appropriate method.

Further, it is possible to detect polymorphisms using "bead arrays" (Illumina Inc.; see, for example, PCT International Publication Nos. WO 99/67641 and WO 00/39587). Illumina Inc. utilizes Bead Array technology which uses a combination of optical fiber bundles and beads that undergo self-association with the array. Each optical fiber bundle has several millions of fibers depending on the diameter of the bundle. Beads are coated with oligonucleotides specific for the detection of certain SNPs or variations. Various types of beads are mixed in specific amounts to allow the formation of an array-specific pool. For assay, a bead array is contacted with a sample prepared from a subject. Then, hybridization is detected by an appropriate method.

8. Evaluation of Drugs

In the present invention, it is possible to evaluate the efficacy and safety of a drug metabolized by the drug metabolizing enzyme, from the results of detection of genetic polymorphisms such as SNP obtained as described above.

Evaluation of drugs may be performed by typing system. Briefly, according to any one of the detection methods described above, allele frequencies between toxicity (side effect) occurrence group and non-occurrence group are compared. A polymorphism which brings about difference in allele frequencies between the two groups is selected as a marker for recognizing the occurrence of toxicity. As a statistical test, usually $\div^2$ test is carried out, but other statistical processing such as Fisher test may also be used. Further, it is possible to reflect this result on the concentration of active body of drugs (or unchanged body or metabolite of drugs) in blood or tissue. With respect to all genetic polymorphisms, the relation of cause and effect with the toxicity is examined. Then, only those genetic polymorphism sites that show correlation with the toxicity are selected. Allele pattern can be examined by preparing in advance all probes or primers for analyzing the genetic polymorphisms and reagents necessary for each technique in reaction plates, cards, glass baseboards or the like, and adding thereto the genomic DNA of a human subject for reaction. When the subject has a genetic polymorphism which has correlation with the toxicity, it is possible to predict whether the drug exhibits effect or toxicity in that subject. The efficacy of a drug may be evaluated in a similar manner. Genetic polymorphisms which correlate with side effect or efficacy vary depending on drugs. Therefore, by conducting typing using the genetic polymorphisms, it becomes possible to predict the efficacy or side effect of the drug.

Using this, the frequency of the genetic polymorphism is compared with efficacy/non-efficacy or presence/absence of side effect. When there is difference in allele frequency, a judgment on the drug can be made.

For example, if the results of analysis of an SNP in persons who showed toxicity (side effect) upon administration of drug A have revealed statistically that 90% of those persons have T/T (e.g. fluorescence intensity of FAM was detected), and if the results of analysis of the SNP in persons who did not show toxicity (side effect) have revealed that only 10% of those persons have T/T and 90% of them have C/C, drug A can be evaluated that it should not be administered to persons with T/T.

9. Screening for Drugs.

The genetic polymorphism information obtained as described above in the present invention may be compared with genetic polymorphism information in a gene of a subject encoding corresponding drug metabolizing enzyme. By doing so, the above information may be used as an indicator for analyzing the efficacy and safety of a drug metabolized by the drug metabolizing enzyme. Therefore, the genetic polymorphism information obtained in the present invention serves as an information source for selecting most effective drug to treat a disease.

As a method, the evaluation method described in "8. Evaluation of Drugs" may be used. Briefly, it can be said that the genetic polymorphisms which were recognized to be correlating with side effect or efficacy in the preceding sub-section give influence upon the activity, the transcription, and the translation of the enzyme. Also, it can be said that such genetic polymorphisms have some indirect relation with a mechanism of the occurrence of side effect or efficacy. The metabolism of a drug is examined and confirmed by a pharmaceutical manufacturer or the like in preclinical test or clinical test. Therefore, if the drug is an enzyme and a polymorphism which correlates with severe side effect exists among the polymorphisms located in the gene encoding the enzyme, it is possible to delete the relevant polymorphism or to use the drug conditionally. With respect to efficacy, a similar evaluation can be made. From such information on side effect and efficacy, drug screening becomes possible.

Further, by conducting genetic polymorphism frequency analysis on cases with side effect occurrence and cases without side effect occurrence in clinical tests (from phase I to phase III tests), it becomes possible to detect new genetic polymorphisms other than the above-mentioned polymorphism which correlate with side effect or efficacy. By examining such polymorphisms in the same manner as described above, drug screening becomes possible.

## BEST MODE FOR CARRYING OUT THE INVENTION

Hereinbelow, the present invention will be described more specifically with reference to the following Example. However, the technical scope of the present invention is not limited to the Example.

### Example 1: Acquisition of SNP information

#### (1) Extraction of DNA

Blood was collected in the presence of EDTA from 48 unrelated individuals. Extraction of DNA was carried out as described below according to the method described in "Genomic Analysis Laboratory Manual (Genomu Kaiseki Rabo Manyuaru)" (Yusuke Nakamura ed., Springer-Verlag Tokyo).

Ten ml of blood was placed in a 50-ml Falcon tube and centrifuged at 3000 rpm at room temperature for 5 minutes. The supernatant (serum) was collected with a pipette, followed by addition of 30 ml of RBC lysing buffer (10 mM $NH_4HCO_3$, 144 mM NH3Cl). After mixing until the sediment was loosened, the mixture was left at room temperature for 20 minutes. This was then centrifuged at 3000 rpm at room temperature for 5 minutes, and the supernatant (serum) was discarded using a pipette to obtain leukocyte pellets. Thirty ml of RBC lysing buffer was added to the pellets and the same procedures were further repeated 2 times. To the leukocyte pellets 4 ml of Proteinase K buffer (50 mM Tris-HCl (pH 7.4), 100 mM NaCl, 1 mM EDTA (pH 8.0)), 200 µl of 10% SDS, 200 µl of 10 mg/ml Proteinase K were added, and the mixture was mixed with inversion, and left overnight at 37°C. Four ml of phenol was added and slowly mixed with inversion using a rotator (Rotator T-50, Taitec). This mixture was centrifuged at 3000 rpm at room temperature for 10 minutes, and the top layer was collected into a new tube. Four ml of phenol-chloroform-isoamyl alcohol (ratio by volume 25:24:1) was added, mixed with inversion for 2 hours as above and centrifuged. The top layer was collected into a new tube, and 4 ml of chloroform-isoamyl alcohol (ratio by volume 24:1) was added, and mixed with inversion for 30 minutes as above and centrifuged. The top layer was collected into a new tube, and 400 µl of 8 M ammonium acetate and 4 ml of isopropanol were added, and then mixed with inversion. Stringy white precipitate (DNA) was collected into a 2-ml tube, and 1 ml of 70% ethanol was added and mixed with inversion. After DNA was collected into a new 2-ml tube and air-dried, 500 µl of TE solution (10 mM Tris-HCl (pH 7.4) and 1 mM EDTA (pH 7.4)) were added to dissolve the DNA to obtain genomic DNA sample.

#### (2) PCR

The genomic sequence was obtained from GenBank DNA database. The repeat sequence was removed using RepMask computer program, and PCR primers were designed so that PCR products would be approximately 1 kb. The genomic DNA used was DNA from 48 unrelated individuals prepared to be at the same concentration. Equal amounts of each DNA from 3 individuals were placed in one tube and mixed, and 60 ng out of the mixture was used for PCR. PCR was performed using Ex-Taq (2.5 U; TaKaRa) on GeneAmp PCR System 9700 (PE Applied Biosystems). One cycle was composed of reaction at 94°C for 2 minutes followed by denaturation at 94°C for 30 seconds, annealing at 60°C or 55°C for 30 seconds, and extension at 72°C for 1 minute, and 35 cycles were completed.

#### (3) Sequencing

PCR products were purified using Arraylt (Telechem), and underwent sequence reaction with BigDye Terminator RR Mix (PE Applied Biosystems). One cycle was composed of reaction at 96°C for 2 minutes, followed by denaturation at 96°C for 20 seconds, annealing at 50°C for 30 seconds, and extension at 60°C for 4 minutes using GeneAmp PCR System 9700 (PE Applied Biosystems), and 25 cycles were completed. After the sequence reafction, sequence analysis was carried out on ABI PRISM 3700 DNA Analyzer.

(4) Detection of SNP

PolyPhred computer program (Nickerson et al., 1997, Nucleic Acids Res., 25, 2745-2751) was used to carry out analysis for SNP detection.

(5) Results

The results as shown in Table 1 were obtained for SNP. In addition, the names and abbreviations of drug metabolizing enzymes analyzed, ACCESSION number from database (GenBank), and genetic structure and positions of SNPs of drug metabolizing enzyme are shown in Figures 9 to 16. In Figures 9 to 16, exons are shown as white boxes or black lines across genes which are shown as horizontal lines. Positions of SNPs are shown with solid lines above the genes and numbered.

Example 2

Polymorphism of CYP genes obtained from 2 different groups of subjects was determined with typing by the Invader method. The results are shown in Figure 17. In Figure 17, the horizontal (Allele 1) and vertical (Allele 2) axes represent fluorescence intensity of VIC corresponding to G and FAM corresponding to A, respectively. Each area circled in with broken lines shows SNP patterns for dark rectangles **(■)** , dark circles (●), and dark triangles (▲) are G/G, A/A, and G/A, respectively. White rectangles (□) mean background values. It can be seen that the subject group shown in the graph of panel A (top) frequently has a SNP pattern of G/G, whereas the subject group shown in the graph of panel B (bottom) frequently has a SNP pattern of A/A.

Example 3: Detection of SNP

Using as samples genomic DNA collected from 5 unrelated individuals as described in Example 1, detection of SNP in 2 types of CYP genes (CYP2E, CYP2S) was performed by the Invader method. Invader probes and allele probes designed based on the respective sequences of Nos. 6 and 13 (SEQ ID Nos. 56 and 63) for CYP2E and Nos. 3 and 10 (SEQ ID Nos. 72 and 79) for CYP2S were used. Locations of each SNP are shown in Table 1.

Results are shown in Table 2.

Table 2

| Drug metabolizing enzyme gene | CYP2E | | CYP2S | |
|---|---|---|---|---|
| | No. 6 | No. 13 | No. 3 | No. 10 |
| | SEQ ID No. 56 | SEQ ID No. 63 | SEQ ID No. 72 | SEQ ID No. 79 |
| SNP | (G/A) | (T/C) | (G/C) | (C/A) |
| Subject I | G/G | T/C | C/C | C/C |
| Subject II | G/A | T/C | G/C | A/A |
| Subject III | G/G | T/T | G/C | C/C |
| Subject IV | G/A | T/T | G/C | C/A |
| Subject V | G/A | T/C | G/G | C/A |
| Subject VI | A/A | C/C | G/C | C/A |

It can be seen from the result shown in Table 2 that the method of the present invention can detect SNP and determine their patterns in drug metabolizing enzyme genes of each subject.

All publications, patents, and patent applications cited herein are incorporated herein by reference in their entirety.

Industrial Applicability

According to the present invention, methods for analyzing SNPs are provided. According to the methods of the invention, it becomes possible to select appropriate drugs for target diseases. Thus, the methods of the invention are extremely useful.

Sequence Listing Free Text

SEQ ID NO 65 : n represents tgt or deletion (Location 21).
SEQ ID NO 66 : n represents 9 to 11 repeats of t (Location 21).
SEQ ID NO 71 : n represents agg or deletion (Location 21).
SEQ ID NO 73 : n represents 10 to 11 repeats of a (Location 21).
SEQ ID NO 75 : n represents tctc or deletion (Location 21).
SEQ ID NO 76 : n represents 11 to 12 repeats of a (Location 21).
SEQ ID NO 78 : n represents 7 to 8 repeats of tc (Location 21).
SEQ ID NO 80 : n represents 10 to 13 repeats of t (Location 21).
SEQ ID NO 85 : n represents 11 to 13 repeats of t (Location 21).
SEQ ID NO 100 : n represents a or deletion (Location 21).
SEQ ID NO 101 : n represents tg or deletion (Location 21).
SEQ ID NO 105 : n represents 12 to 25 repeats of t (Location 21).
SEQ ID NO 112 : n represents 10 to 12 repeats of t (Location 21).
SEQ ID NO 115 : n represents 12 to 16 repeats of a (Location 21).
SEQ ID NO 116 : n represents gag or deletion (Location 21).
SEQ ID NO 122 : n represents 9 to 10 repeats of t (Location 21).
SEQ ID NO 126 : n represents "ttggtcccccaaccgcctgtctccccacaccccgcaa" or " aggctgcagtcc" (Location 21).
SEQ ID NO 129 : n represents 9 to 12 repeats of t (Location 21).
SEQ ID NO 137 : n represents 12 to 13 repeats of ac (Location 21).
SEQ ID NO 141 : n represents 9 to 10 repeats of a (Location 21).
SEQ ID NO 146 : n represents 13 to 16 repeats of t (Location 21).
SEQ ID NO 147 : n represents c or deletion (Location 21).
SEQ ID NO 148 : n represents 9 to 10 repeats of t (Location 21).
SEQ ID NO 149 : n represents 10 to 12 repeats of a (Location 21).
SEQ ID NO 152 : n represents 8 to 9 repeats of t (Location 21).
SEQ ID NO 182 : n represents 9 to 10 repeats of t (Location 21).
SEQ ID NO 191 : n represents 8 to 9 repeats of t (Location 21).
SEQ ID NO 197 : n represents 9 to 12 repeats of t (Location 21).
SEQ ID NO 198 : n represents 10 to 11 repeats of t (Location 21).
SEQ ID NO 199 : n represents 12 to 14 repeats of t (Location 21).
SEQ ID NO 204 : n represents 16 to 19 repeats of a (Location 21).
SEQ ID NO 205 : n represents 6 to 7 repeats of a (Location 21).

## Claims

1. A method for detecting a genetic polymorphism(s) in a gene encoding cytochrome P450 using as oligonucleotide probes and/or oligonucleotide primers at least one sequence selected from the group consisting of an at least 13 nucleotide sequence within any of the nucleotide sequences as shown in SEQ ID NOS: 1 through 215, said at least 13 nucleotide sequence containing the 21st nucleotide, or a sequence complementary to said at least 13 nucleotide sequence.

2. The method according to claim 1, wherein the polymorphism is a single-nucleotide polymorphism, a polymorphism caused by deletion, substitution or insertion of a plurality of nucleotides, or a VNTR or microsatellite polymorphism.

3. A method for evaluating a drug, comprising evaluating the efficacy and safety of the drug metabolized by cytochrome P450 from the detection results obtained by the method according to claim 1 or 2.

4. A method for screening a drug, comprising selecting a drug to be used with reference to the evaluation obtained by the method according to claim 3.

5. A method for screening a drug, comprising comparing information about a polymorphism(s) in a gene encoding cytochrome P450 and/or a sequence complementary thereto with information about a polymorphism(s) in a gene encoding cytochrome P450 and/or a sequence complementary thereto obtained from a subject; analyzing the efficacy and/or safety of drugs metabolized by cytochrome P450; and selecting a drug to be used from the analysis results obtained.

**6.** The method according to any one of claims 1 to 5, wherein the cytochrome P450 is at least one selected from the group consisting of 2A6, 2AI3, 2B6, 2E, 2S1, 5A1, 7A1 and 7B1.

**7.** An oligonucleotide selected from the group consisting of the nucleotide sequences as shown in SEQ ID NOS: 1 through 215 and sequences complementary thereto.

**8.** An oligonucleotide prepared so as to include a gene polymorphic site present in a gene encoding cytochrome P450 and/or a sequence complementary thereto based on the information about a polymorphism(s) in the gene and/or the complementary sequence thereto.

**9.** The oligonucleotide according to claim 8, wherein the oligonucleotide is designed such that the nucleotide at the 5'-end, the 3'-end or the center of the oligonucleotide corresponds to the gene polymorphic site.

**10.** The oligonucleotide according to claim 9, wherein the oligonucleotide containing a gene polymorphic site is composed of two fragments being linked to each other, one fragment being hybridizable to the gene encoding a cytochrome P450 or the sequence complementary thereto and the other fragment being not hybridizable thereto, and said polymorphic site is positioned at the 5'-end or the 3'-end of the hybridizable fragment.

**11.** The oligonucleotide according to claim 9 or 10, wherein the oligonucleotide consists of an at least 13 nucleotide sequence within any of the nucleotide sequences as shown in SEQ ID NOS: 1 through 215, said at least 13 nucleotide sequence containing the 21st nucleotide, or a sequence complementary to said at least 13 nucleotide sequence.

**12.** An oligonucleotide which is designed in a genomic DNA region containing a gene polymorphic site in any of the nucleotide sequences as shown in SEQ ID NOS: 1 through 215 so that it is located within 1000 bp of the polymorphic site toward the 5' -end and/or the 3' -end of the genomic DNA region, and which has a length of 13-60 nucleotides.

**13.** A microarray wherein the oligonucleotide according to any one of claims 7 to 12 is immobilized on a support.

**14.** A genetic polymorphism detection kit comprising the oligonucleotide according to any one of claims 7 to 12 and/or the microarray according to claim 13.

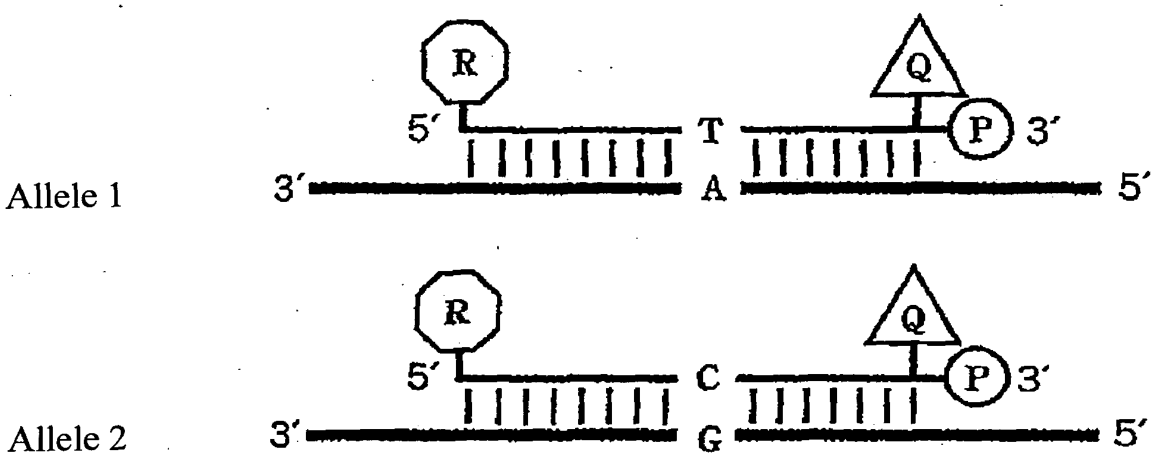
R
5′
T
Q
P
3′
Allele 1
3′
A
5′
R
5′
C
Q
P
3′
Allele 2
3′
G
5′

a. Hybridization

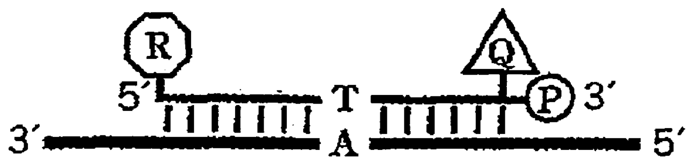


b. PCR reaction

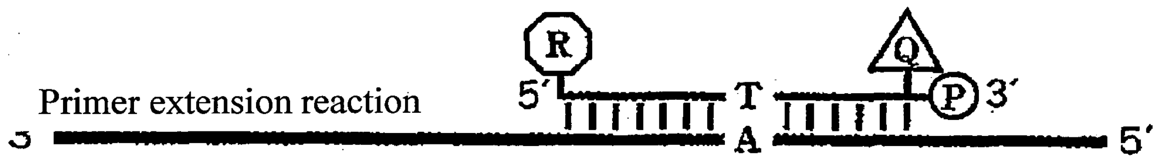


c. 5'- nuclease activity

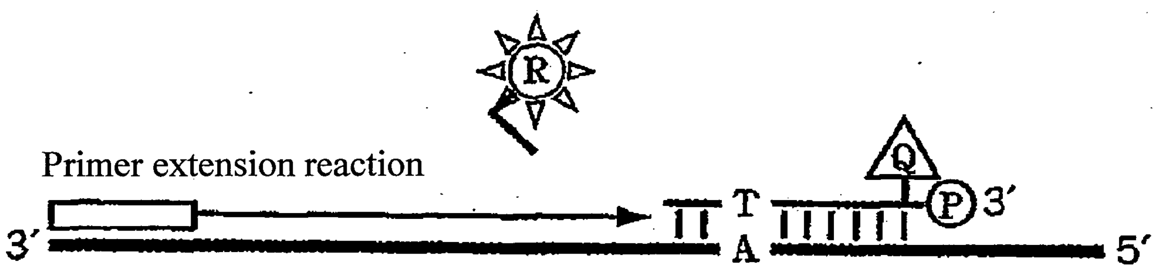

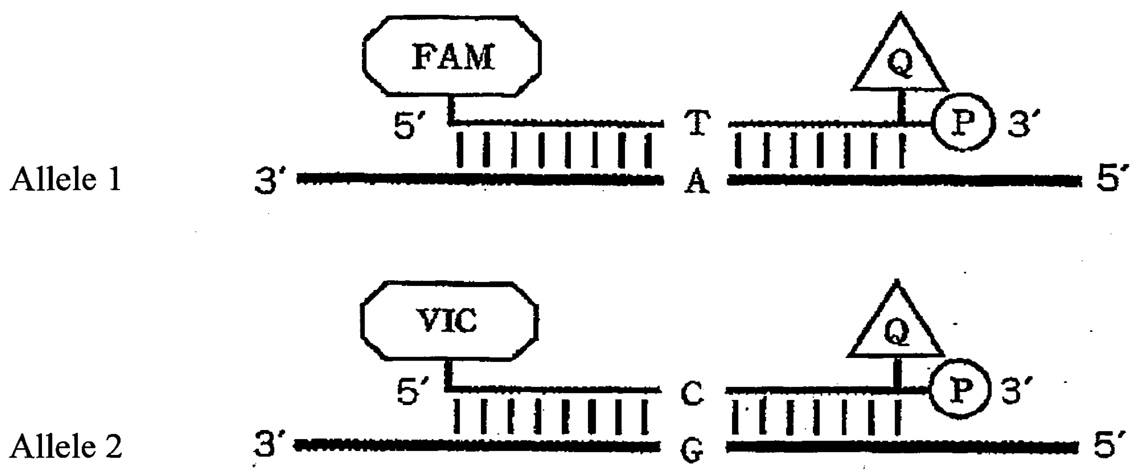
Allele 1
FAM
5′
T
Q
P
3′
3′
A
5′
Allele 2
VIC
5′
C
Q
P
3′
3′
G
5′

a. allele probe

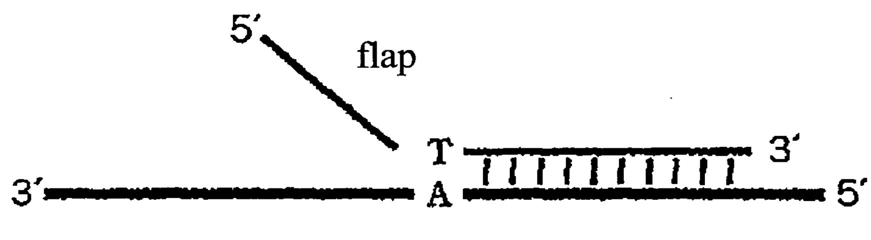


b. invader probe

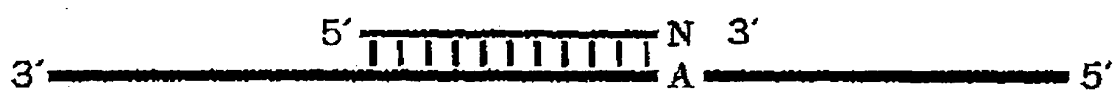


c. 5’- nuclease activity

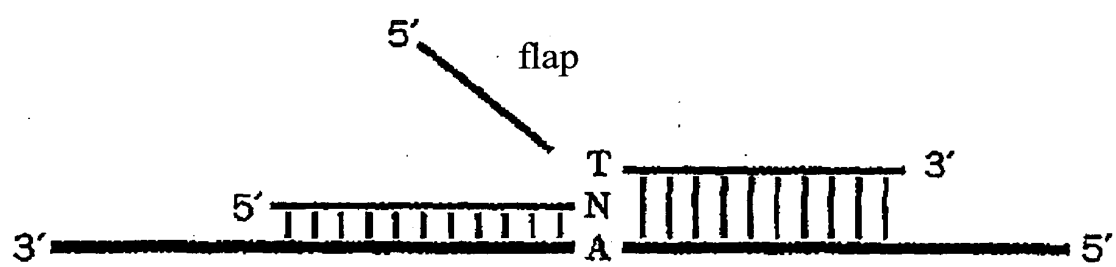

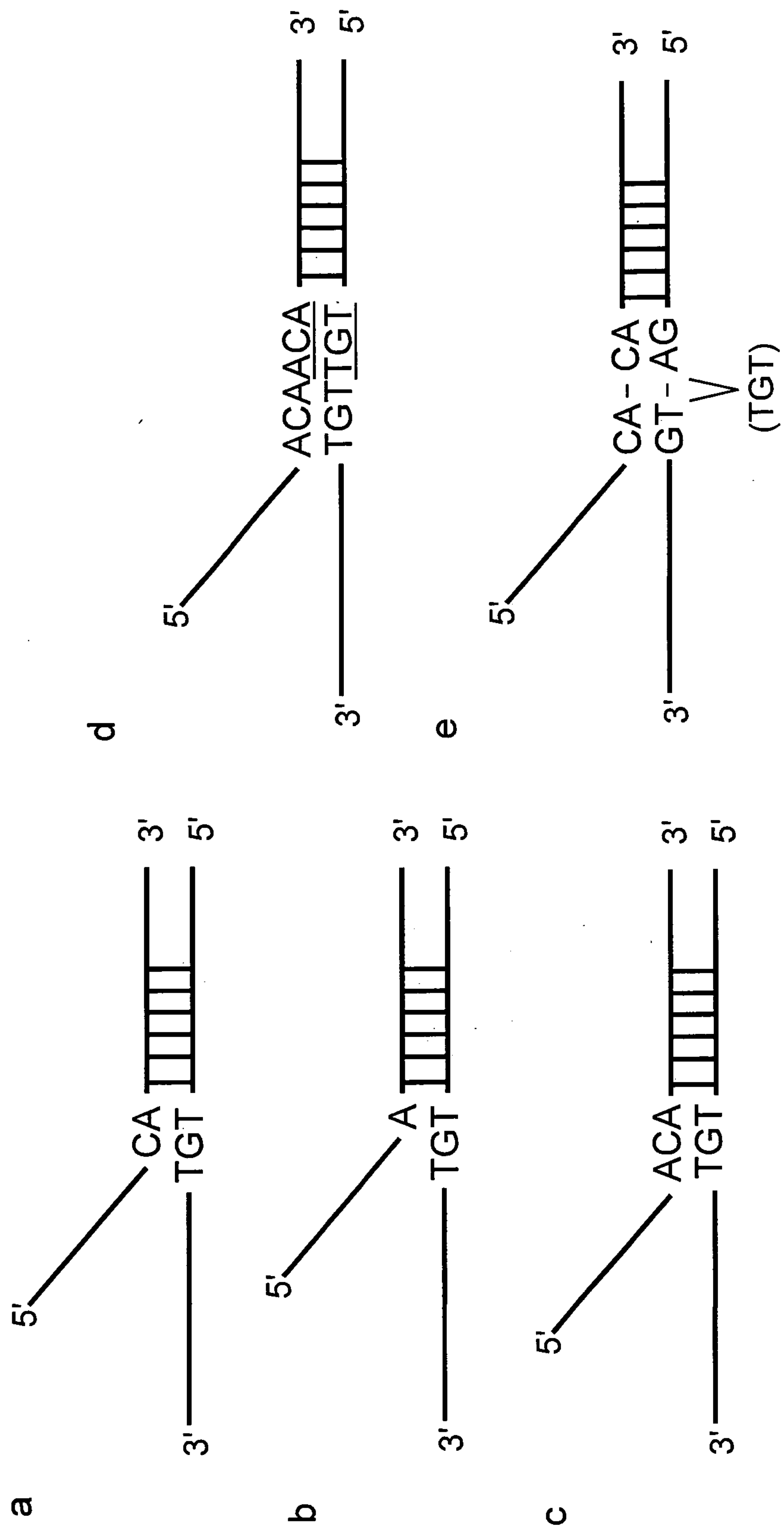
a
5'
3'
CA
TGT
3'
5'
b
5'
3'
A
TGT
3'
5'
c
5'
3'
ACA
TGT
3'
5'
d
5'
3'
ACAACA
TGTTGT
3'
5'
e
5'
3'
CA - CA
GT - AG
(TGT)
3'
5'

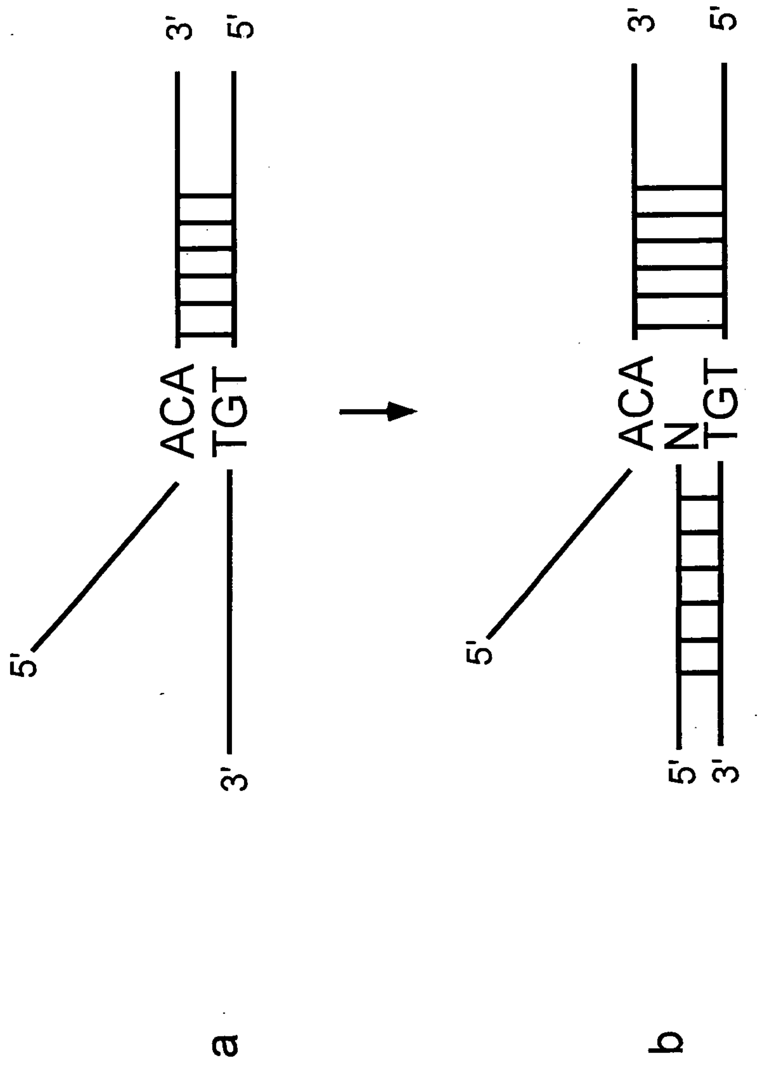
a
5'
3'
ACA
TGT
3'
5'
b
5'
5'
3'
ACA
N
TGT
3'
5'

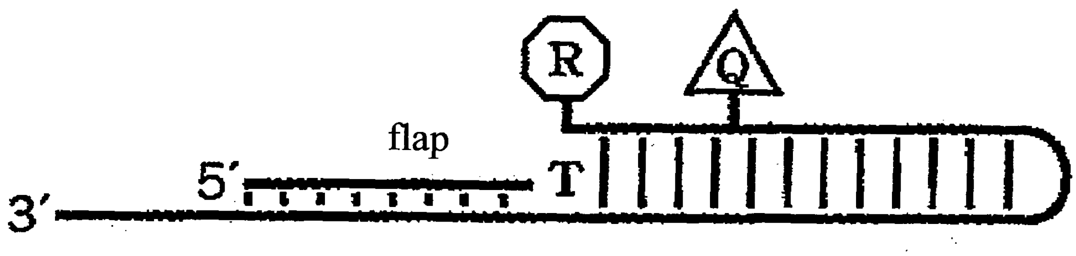
R
Q
flap
5′
T
3′

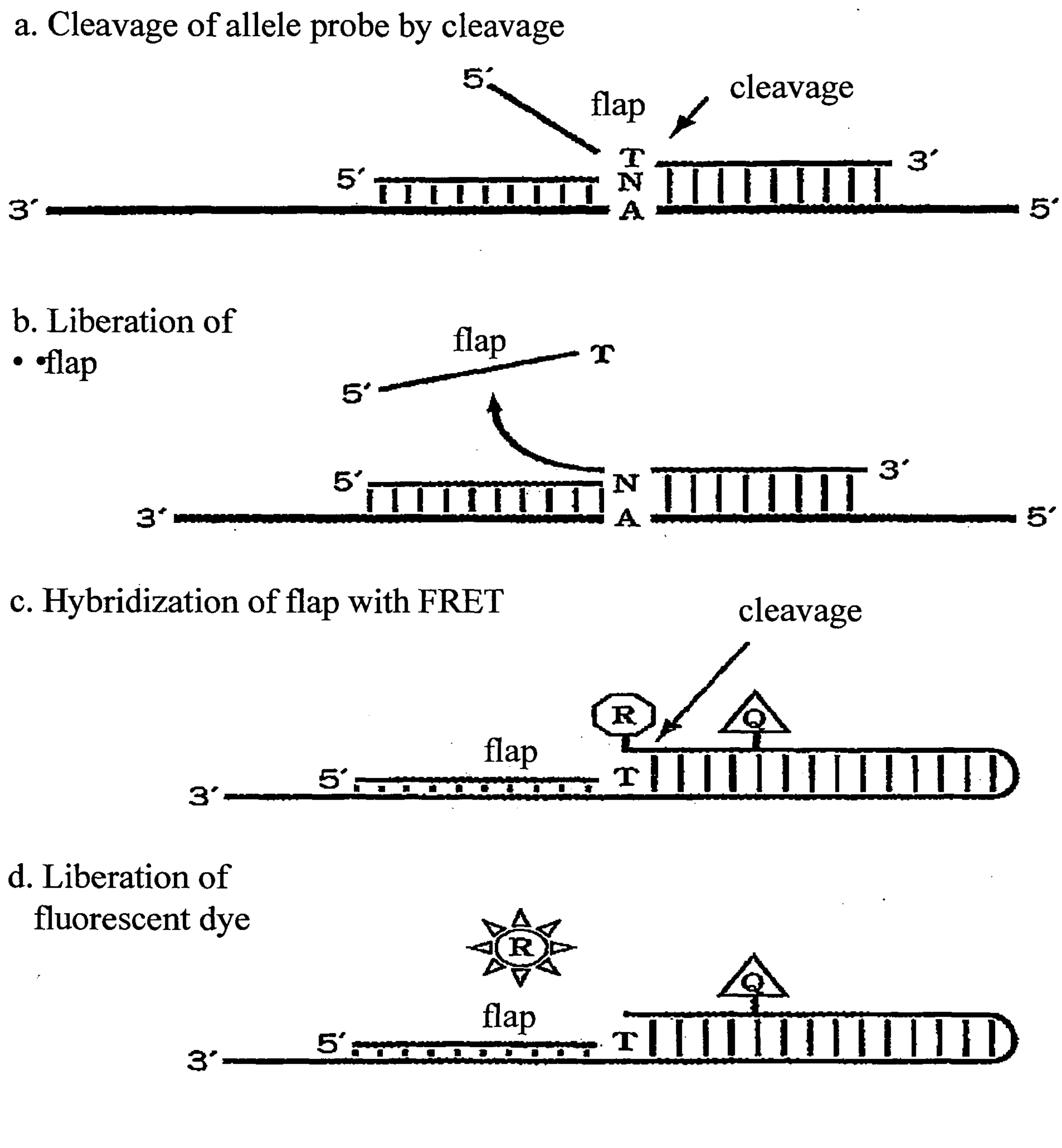
a. Cleavage of allele probe by cleavage
5'
flap
cleavage
T
N
A
5'
3'
3'
5'
b. Liberation of
• •flap
flap
T
5'
5'
N
A
3'
3'
5'
c. Hybridization of flap with FRET
cleavage
R
Q
flap
5'
T
3'
d. Liberation of
fluorescent dye
R
Q
flap
5'
T
3'

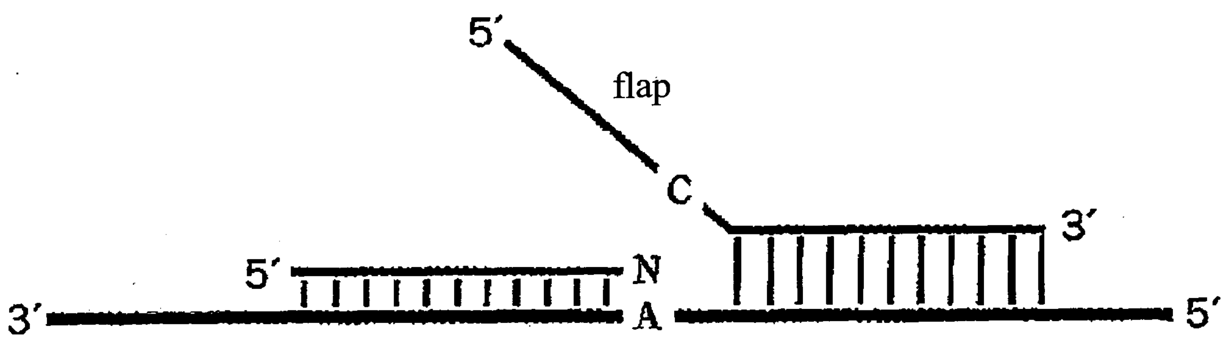
5′
flap
C
3′
5′
N
3′
A
5′

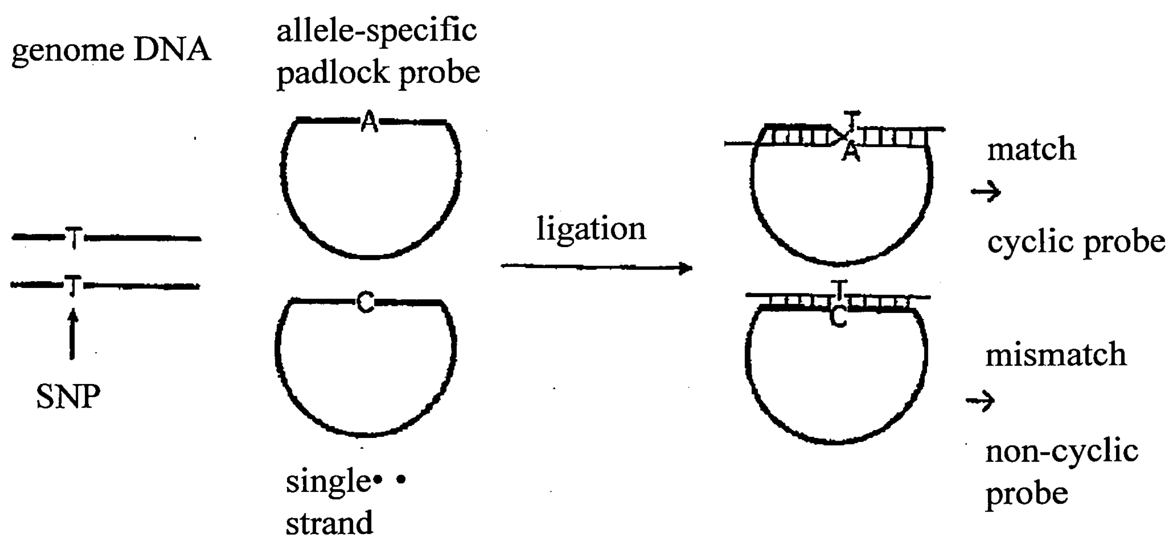
genome DNA
allele-specific
padlock probe
A
T
T
SNP
C
single• •
strand
ligation
T
A
match
→
cyclic probe
T
C
mismatch
→
non-cyclic
probe

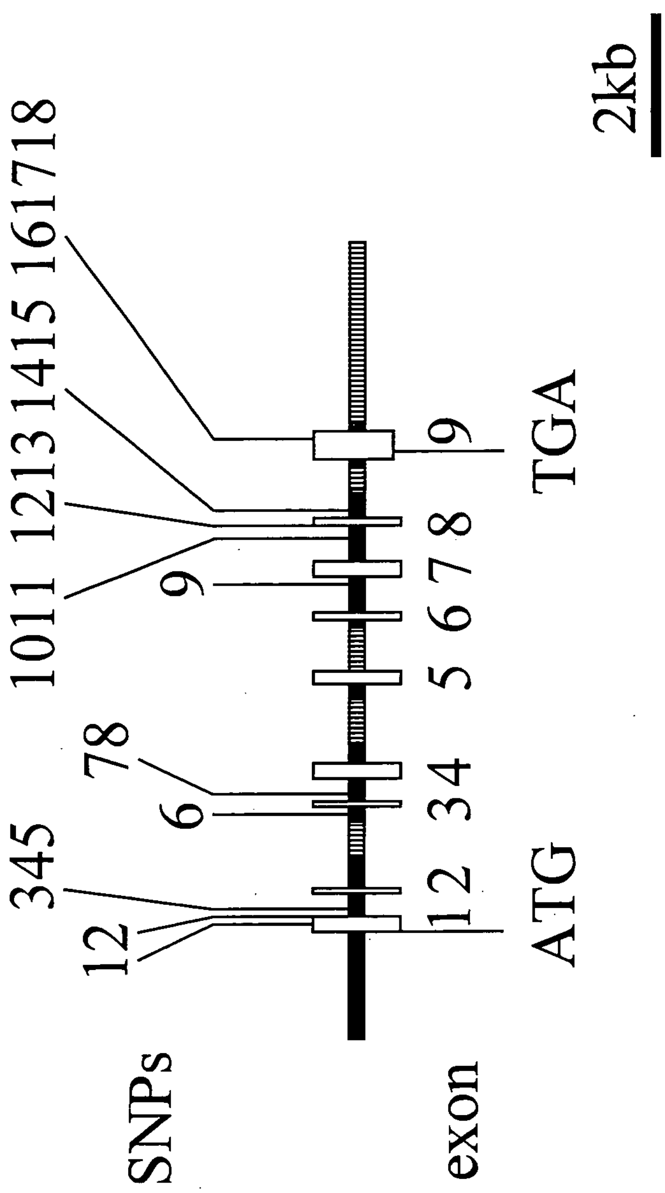
SNPs
exon
12
345
6
78
9
1011
1213
1415
161718
1 2 3 4 5 6 7 8 9
ATG
TGA
2kb

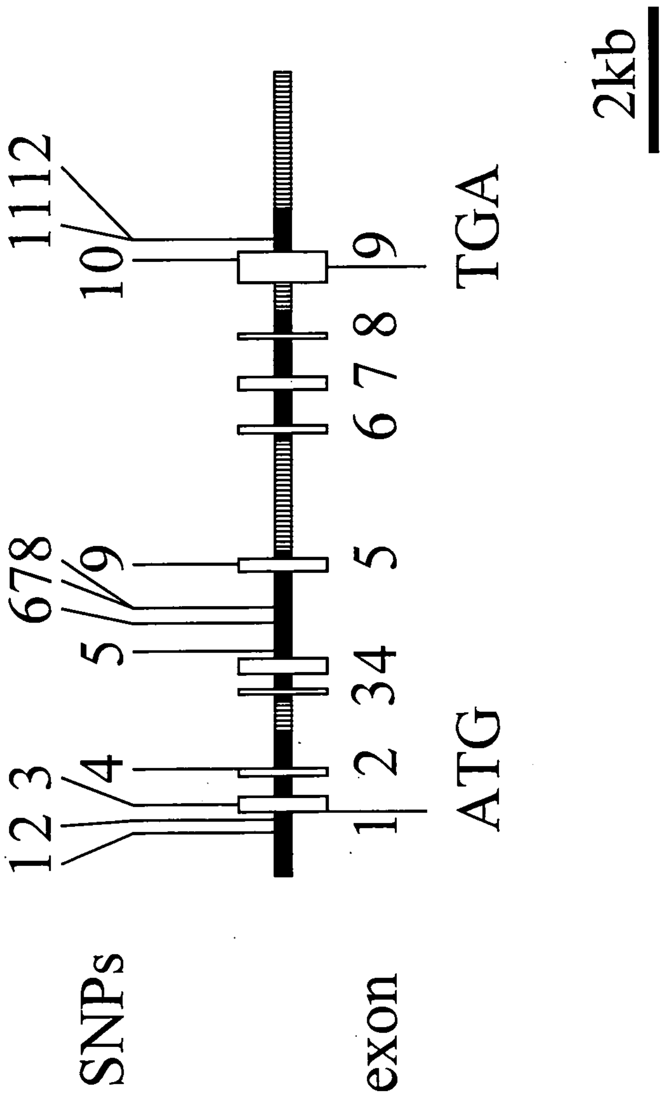
SNPs
1 2 3 4 5 6 7 8 9 10 11 12
exon
1 2 3 4 5 6 7 8 9
ATG
TGA
2kb

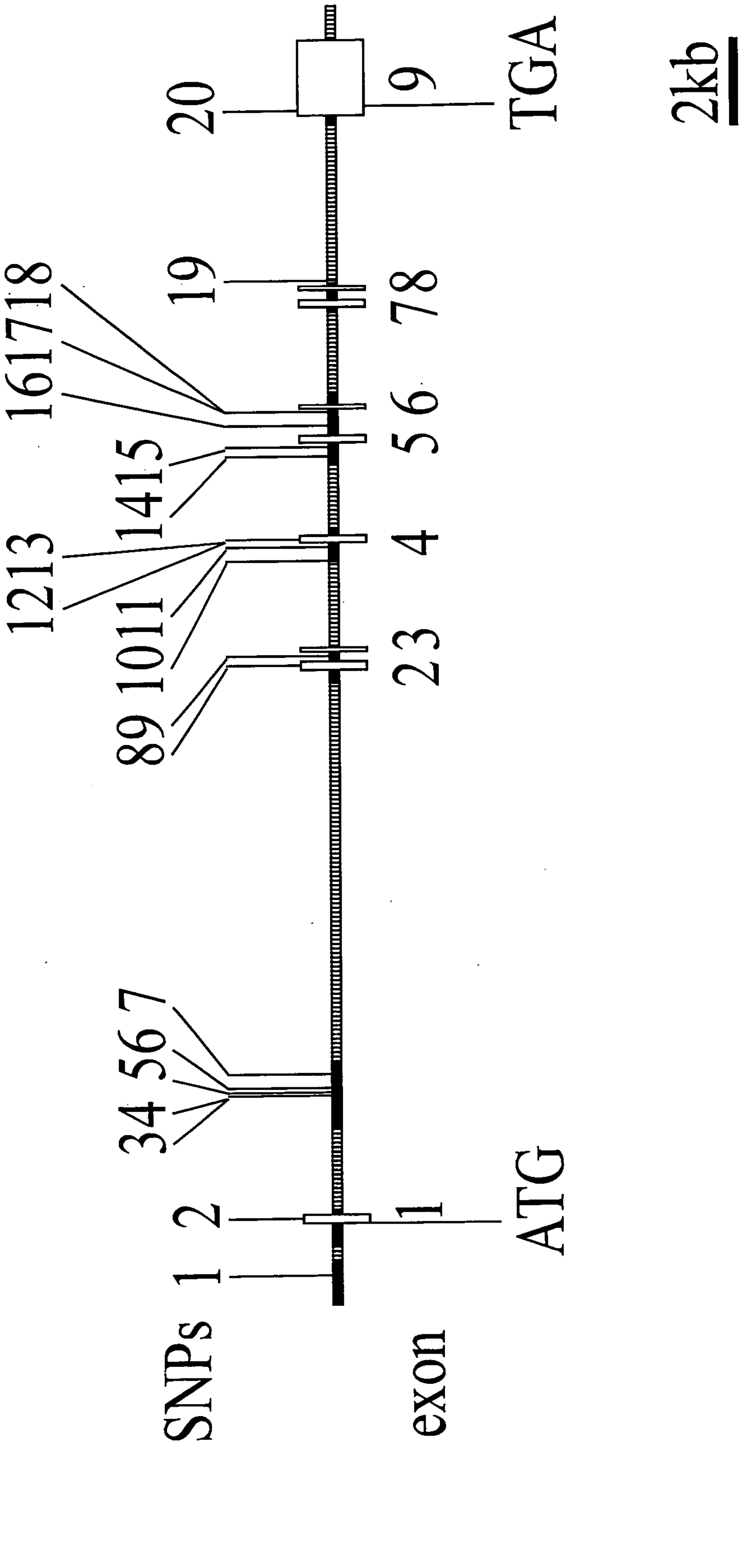
SNPs
exon
1
2
34
56
7
89
1011
1213
1415
161718
19
20
ATG
1
23
4
56
78
9
TGA
2kb

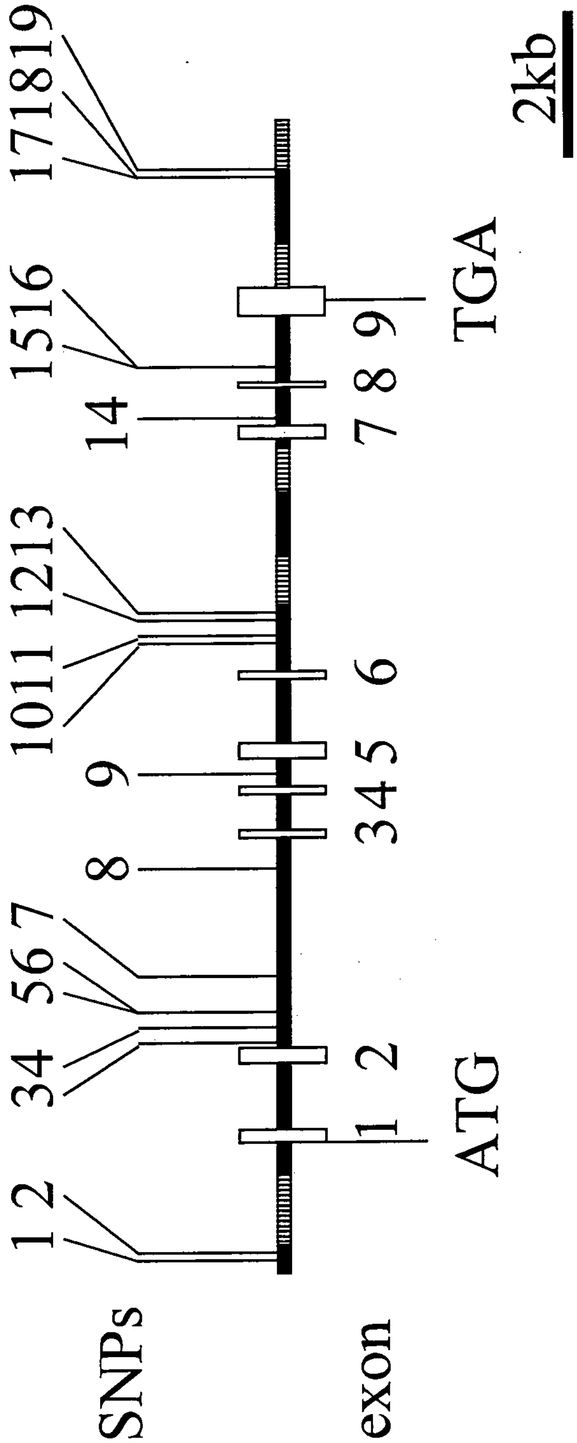
SNPs
1 2
3 4
5 6
7
8
9
10 11
12 13
14
15 16
17 18 19
exon
1 2
3 4 5
6
7 8 9
ATG
TGA
2kb

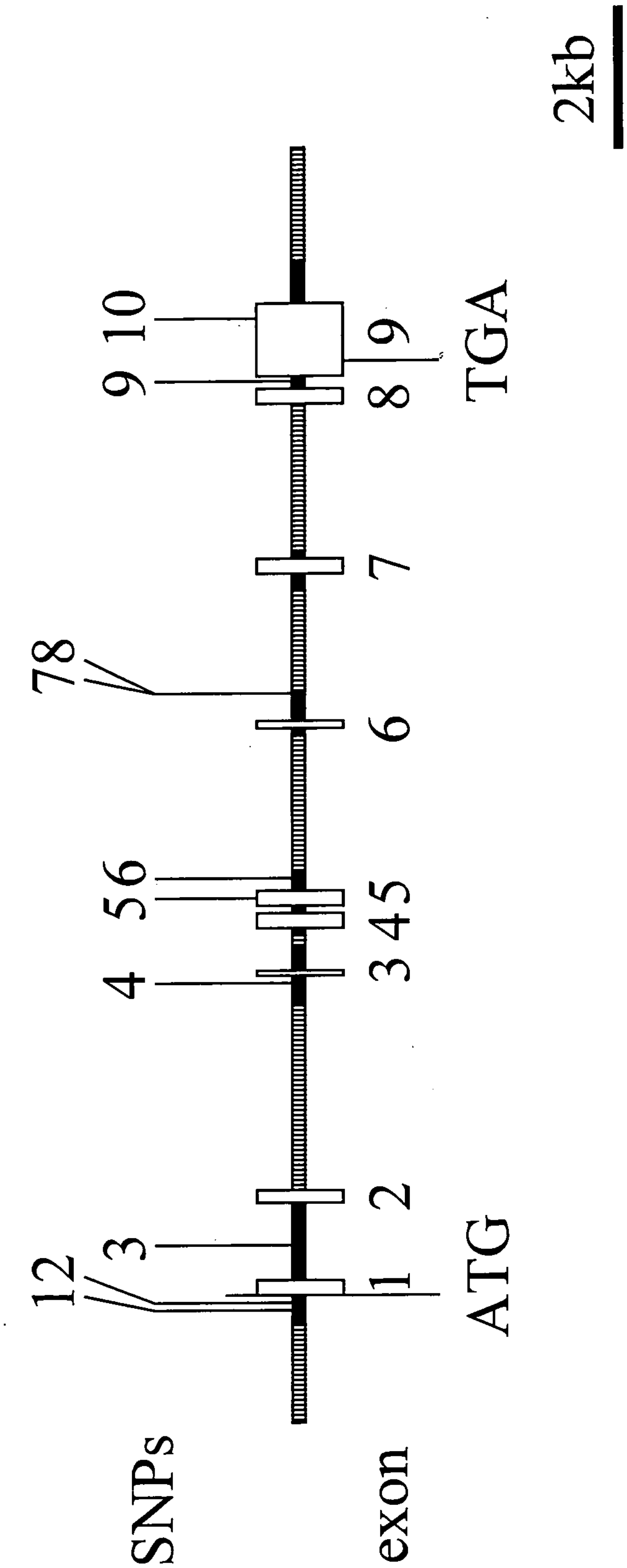
SNPs
exon
1 2
3
4
5 6
7 8
9
10
1
2
3
4
5
6
7
8
9
ATG
TGA
2kb

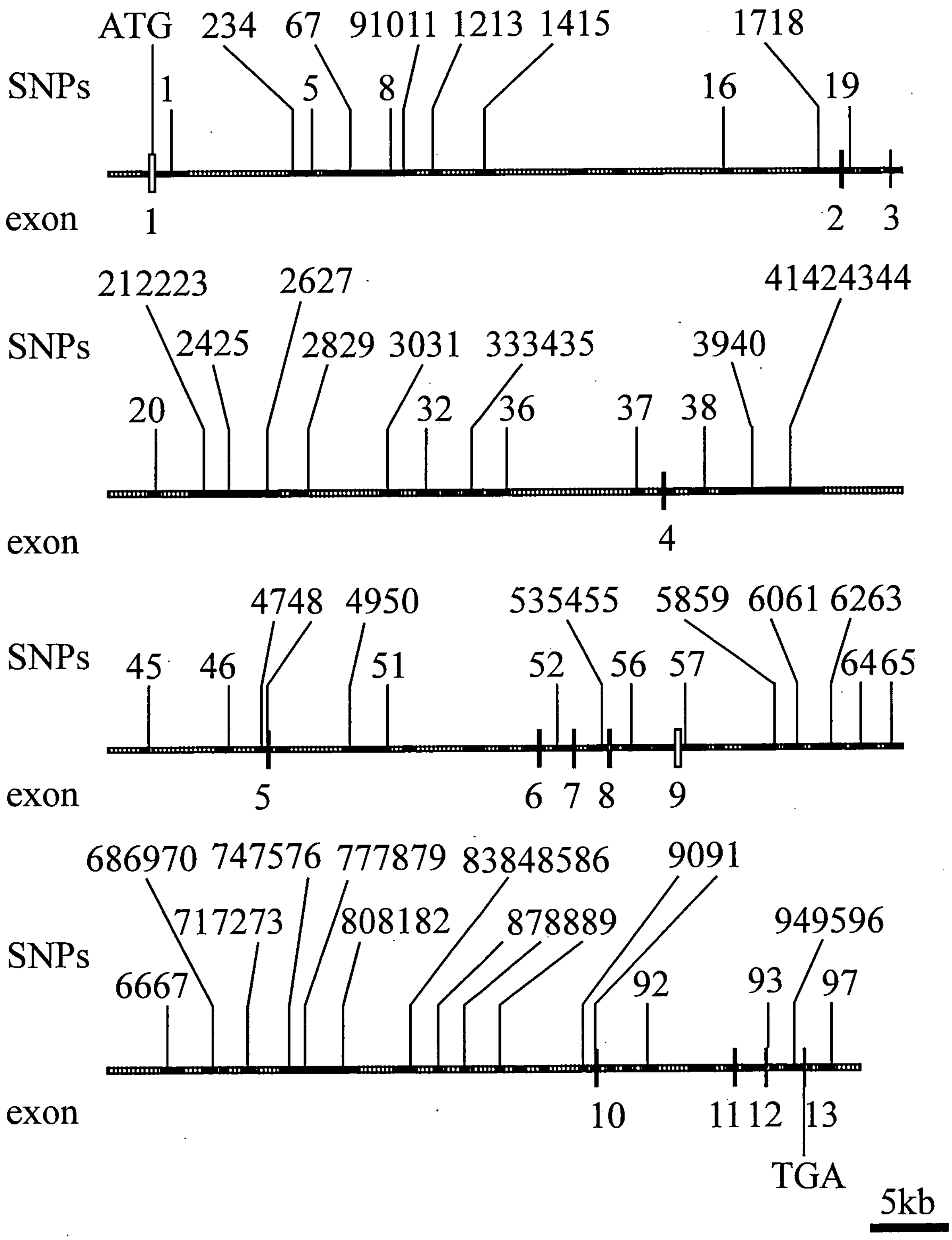
ATG
234
67
91011
1213
1415
1718
SNPs
1
5
8
16
19
exon
1
2
3
212223
2627
41424344
SNPs
2425
2829
3031
333435
3940
20
32
36
37
38
exon
4
4748
4950
535455
5859
6061
6263
SNPs
45
46
51
52
56
57
6465
exon
5
6
7
8
9
686970
747576
777879
83848586
9091
717273
808182
878889
949596
SNPs
6667
92
93
97
exon
10
11
12
13
TGA
5kb

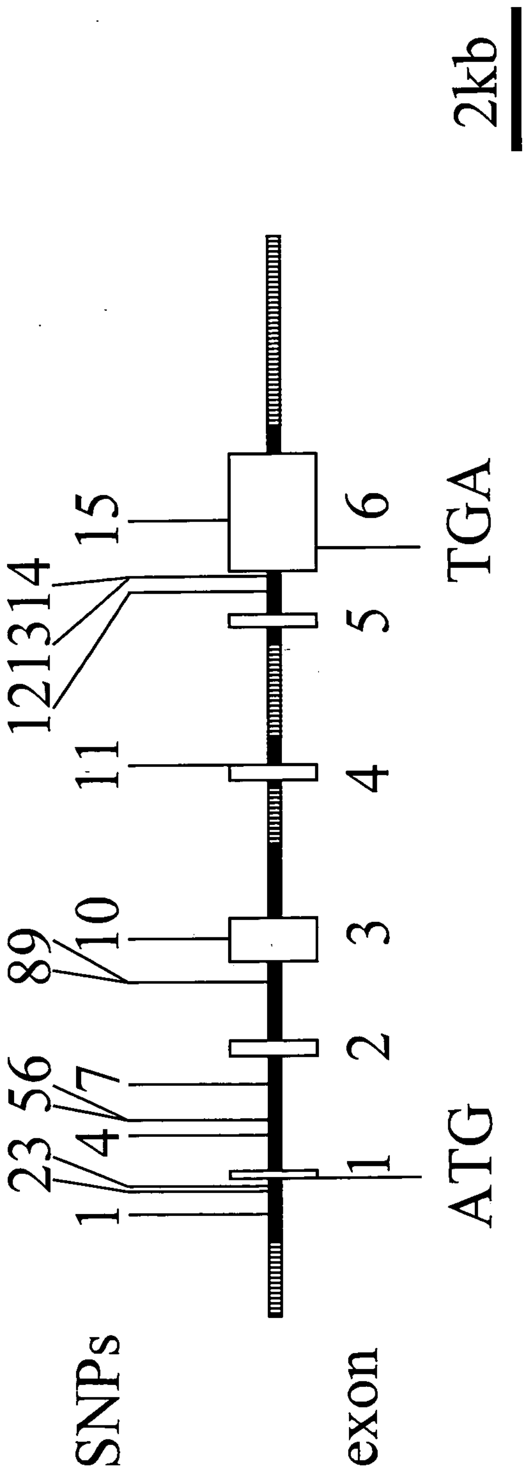
2kb
SNPs
exon
1
23
4
56
7
89
10
11
121314
15
ATG
TGA
1
2
3
4
5
6

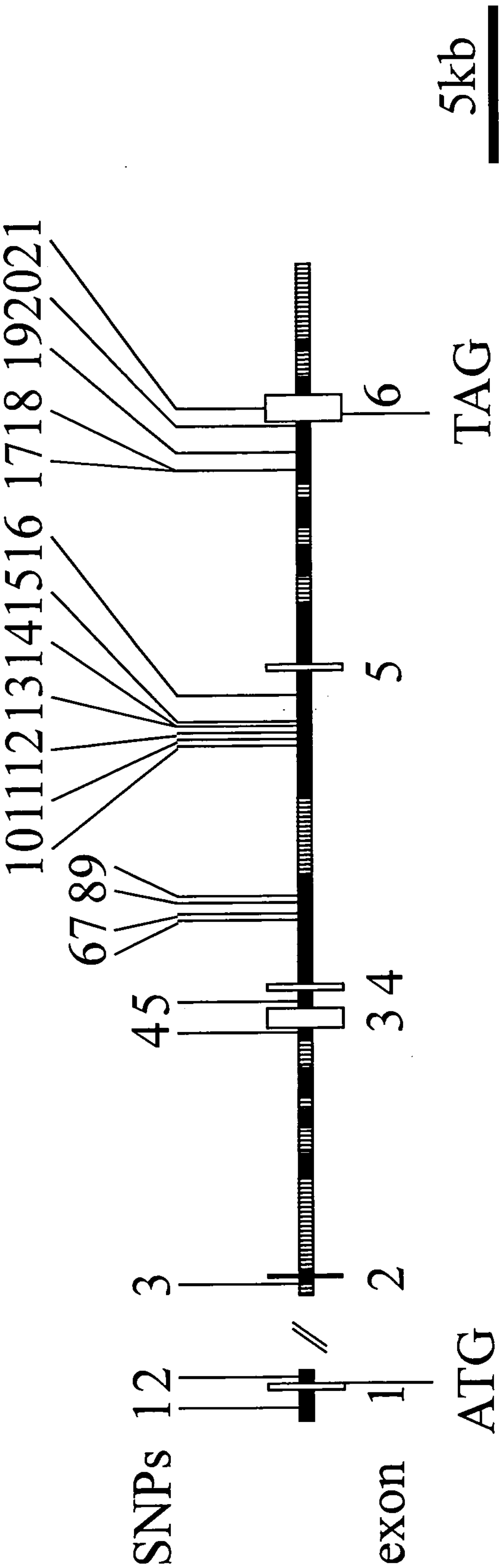
SNPs
exon
1 2
3
4 5
6 7 8 9
10 11 12 13 14 15 16
17 18 19 20 21
1
2
3 4
5
6
ATG
TAG
5kb

Fig. 17A

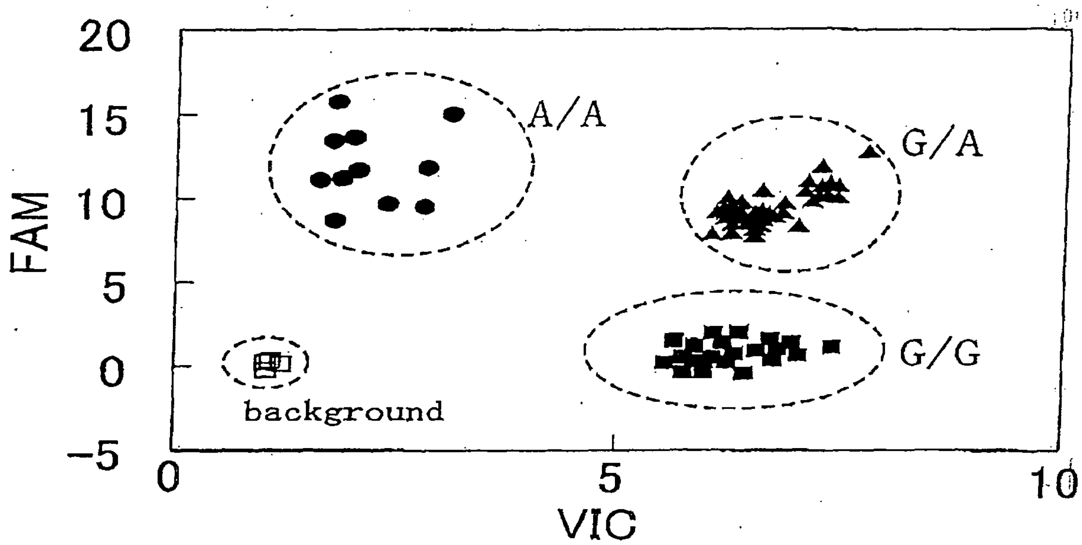


Fig. 17B

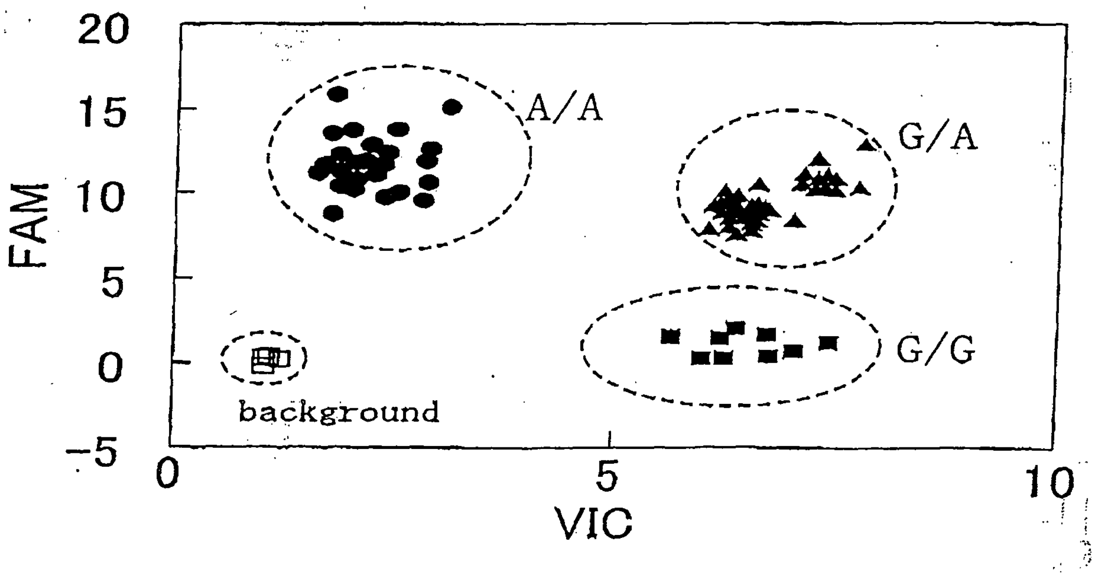

## INTERNATIONAL SEARCH REPORT

International application No. PCT/JP03/06750

A. CLASSIFICATION OF SUBJECT MATTER
Int.Cl[7] C12N15/09, C12M1/00, C12Q1/68, G01N33/15, G01N33/50, G01N33/53, G01N33/566

According to International Patent Classification (IPC) or to both national classification and IPC

B. FIELDS SEARCHED

Minimum documentation searched (classification system followed by classification symbols)
Int.Cl[7] C12N15/09, C12M1/00, C12Q1/68, G01N33/15, G01N33/50, G01N33/53, G01N33/566

Documentation searched other than minimum documentation to the extent that such documents are included in the fields searched

Electronic data base consulted during the international search (name of data base and, where practicable, search terms used)
SwissProt/PIR/GeneSeq, GeneBank/EMBL/DDBJ/GeneSeq, BIOSIS, MEDLINE, WPIDS

C. DOCUMENTS CONSIDERED TO BE RELEVANT

| Category* | Citation of document, with indication, where appropriate, of the relevant passages | Relevant to claim No. |
|---|---|---|
| Y | WO 00/39332 A (Janssen Pharmaceutica Naamloze Vennootschap), 06 July, 2000 (06.07.00), & AU 1878300 A & CA 2355658 A & EP 1141398 A & JP 2002-533136 A | 1-14 |
| Y | Meisel C. et al., Clin.Chem.Lab.Med., Vol.38(9), pages 869 to 876, (2000) | 1-14 |
| Y | KITAGAWA, K. et al., J.Biol.Chem., Vol.276(21), pages 17830 to 17835, (2001) | 1-14 |
| Y | NAKAJIMA, M. et al., Clin.Pharmacol.Ther., Vol.68, pages 72 to 78, (2001) | 1-14 |
| Y | Akiyoshi N. et al., Biochem.Biophys.Res.Commun., Vol.281, pages 810 to 814, (2001) | 1-14 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 September, 2003 (09.09.03) | 24 September, 2003 (24.09.03) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/06750

**Box I Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

Each of claims 1 to 14 has 215 inventions respectively having oligonucleotides selected from the group consisting of the base sequences represented by SEQ ID NOS:1 to 215 and base sequences complementary thereto as a technical feature.

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:
The parts relating to claim 1 in the inventions as set forth in claims 1 to 14.

**Remark on Protest** ☐ The additional search fees were accompanied by the applicant's protest.

☐ No protest accompanied the payment of additional search fees.

Form PCT/ISA/210 (continuation of first sheet (1)) (July 1998)

SEQUENCE LISTING

<110> RIKEN

<120> A method of detecting gene polymorphism

<130> PH-1793-PCT

<150> JP 2002-158237

<151> 2002-5-30

<160> 215

<170> PatentIn Ver. 2.1

<210> 1

<211> 41

<212> DNA

<213> Homo sapiens

<400> 1

tgctggcctc agggatgctt ytggtggcct tgctggtctg c 41

<210> 2

<211> 41

<212> DNA

<213> Homo sapiens

<400> 2

ttcattggaa actacctgca rctgaacaca gagcagatgt a 41

<210> 3

<211> 41

<212> DNA

<213> Homo sapiens

<400> 3

aggcagggag atgggtggca yggggtgggg gctgcctagt t 41

<210> 4

<211> 41

<212> DNA

<213> Homo sapiens

<400> 4

cagtgtggac cagagtctta rgaaatggag ttttggagtt t 41

<210> 5

<211> 41

<212> DNA

<213> Homo sapiens

<400> 5

caggatcttg ggatgtccag ytccctgact gtgagaacct g 41

<210> 6

<211> 41

<212> DNA

<213> Homo sapiens

<400> 6

agctgctccg ctcctgcccc ygccgccccc tggcctgtct c 41

<210> 7

<211> 41

<212> DNA

<213> Homo sapiens

<400> 7

gagcagggga ccccgagtgc kggggcagga gaaggaaaac a 41

<210> 8

<211> 41

<212> DNA

<213> Homo sapiens

<400> 8

acccgcgcgc gttctgcctg sggatgggga ctaggtgggg a 41

<210> 9

<211> 41

<212> DNA

<213> Homo sapiens

<400> 9
ttaacaggat cctactccaa maatgcgaat ggctgatgtc t 41

<210> 10
<211> 41
<212> DNA
<213> Homo sapiens

<400> 10
ctccagactc tttgtgtcag ragaatcaaa cacatgttcc c 41

<210> 11
<211> 41
<212> DNA
<213> Homo sapiens

<400> 11
cttcccctta cctggggcac mctagttccc cctccagccc c 41

<210> 12
<211> 41
<212> DNA
<213> Homo sapiens

<400> 12
caggacttca atccccagca yttcctgaat gagaaggggc a 41

<210> 13

<211> 41

<212> DNA

<213> Homo sapiens

<400> 13

ccccagcact tcctgaatga saaggggcag tttaagaaga g 41

<210> 14

<211> 41

<212> DNA

<213> Homo sapiens

<400> 14

gaccactgtt tgctgccagg ycacggctca caccagcagg g 41

<210> 15

<211> 41

<212> DNA

<213> Homo sapiens

<400> 15

ccacggctca caccagcagg sgcctccctc accctcctcc c 41

<210> 16

<211> 41

<212> DNA

<213> Homo sapiens

<400> 16

gggaagagaa gaaacagaag sggctcagtt caccttgata a 41

<210> 17

<211> 41

<212> DNA

<213> Homo sapiens

<400> 17

gagctgggat gagaggaagg raaccccttac attatgctat g 41

<210> 18

<211> 41

<212> DNA

<213> Homo sapiens

<400> 18

ctgggatgag aggaaggaaa mccttacatt atgctatgaa g 41

<210> 19

<211> 41

<212> DNA

<213> Homo sapiens

<400> 19

tccacagttt atctgttgcc ygctcctaaa tccacagccc t 41

<210> 20
<211> 41
<212> DNA
<213> Homo sapiens

<400> 20
agctgtgtgg gattcagggt yggggtgtag ttgggaggtg a 41

<210> 21
<211> 41
<212> DNA
<213> Homo sapiens

<400> 21
ggtcttgatg tcagtctggc rgcagaggaa gagcaggggg a 41

<210> 22
<211> 41
<212> DNA
<213> Homo sapiens

<400> 22
aggctgagga gttcagcggg ygaggcgagc aggccacctt c 41

<210> 23

<211> 41

<212> DNA

<213> Homo sapiens

<400> 23

cagtgtcccc tcaaaatcag yccccgatat tggacaactg g 41

<210> 24

<211> 41

<212> DNA

<213> Homo sapiens

<400> 24

tctacttcac ccacttaaat rcctgaaaac atggacaggt g 41

<210> 25

<211> 41

<212> DNA

<213> Homo sapiens

<400> 25

cacctcaaca ggtatcccct scacttcaac atcttcacca g 41

<210> 26

<211> 41

<212> DNA

<213> Homo sapiens

<400> 26
agccccactt taatacctga rcacctgaac aaaagccccc a 41

<210> 27
<211> 41
<212> DNA
<213> Homo sapiens

<400> 27
agaaggtgga gcacaaccag ygcacgctgg atcccaattc c 41

<210> 28
<211> 41
<212> DNA
<213> Homo sapiens

<400> 28
gggctaagaa tgggggcagt sggggaagga aggggagagg t 41
<210> 29
<211> 41
<212> DNA
<213> Homo sapiens

<400> 29
gaaagttgtc tctatctgat ktctcacaaa acgtaagtgc c 41

<210> 30

<211> 41

<212> DNA

<213> Homo sapiens

<400> 30

tatctgattt ctcacaaaac rtaagtgccc agaaaatctt t 41

<210> 31

<211> 41

<212> DNA

<213> Homo sapiens

<400> 31

caccagttct gcatctcttg stcctccttc tgtttctccg a 41

<210> 32

<211> 41

<212> DNA

<213> Homo sapiens

<400> 32

tcttgctact cctggttcag ygccacccta acacccatga c 41

<210> 33

<211> 41

<212> DNA

<213> Homo sapiens

<400> 33

aacagaaacc aacttatctt sgtcgatttt gaggttgata g 41

<210> 34
<211> 41
<212> DNA
<213> Homo sapiens

<400> 34
ttatcttcgt cgattttgag rttgatagta atttcagtta t 41

<210> 35
<211> 41
<212> DNA
<213> Homo sapiens

<400> 35
agagtaagca aacctcaaga yactcaaggt aggcactcgt g 41

<210> 36
<211> 41
<212> DNA
<213> Homo sapiens

<400> 36
tagatttgtt tacccataag yctgcatagc tcctgaacaa g 41

<210> 37

<211> 41

<212> DNA

<213> Homo sapiens

<400> 37

ccacaccccc ataggtagtc yccaggtctt gcatacggga t 41

<210> 38

<211> 41

<212> DNA

<213> Homo sapiens

<400> 38

ttcacggtac acctgggacc saggcccgtg gtcatgctgt g 41

<210> 39

<211> 41

<212> DNA

<213> Homo sapiens

<400> 39

ccaacttctt ctacaaccaa yccacacctc ccctgcaccc c 41

<210> 40

<211> 41

<212> DNA

<213> Homo sapiens

<400> 40

gggggcctgag aggaggtgca kagtgagaac cggctgcatg g 41

<210> 41

<211> 41

<212> DNA

<213> Homo sapiens

<400> 41

acttcagtct gtgtccttga yctgctgctt cttcctaggg g 41

<210> 42

<211> 41

<212> DNA

<213> Homo sapiens

<400> 42

tcctaggggc cctcatggac sccaccttcc tcttccagtc c 41

<210> 43

<211> 41

<212> DNA

<213> Homo sapiens

<400> 43

gaccccacct tcctcttcca ktccattacc gccaacatca t 41

<210> 44

<211> 41

<212> DNA

<213> Homo sapiens

<400> 44

agaaagacaa ataaacaggc ygaggtagac aatgggtgac a 41

<210> 45

<211> 41

<212> DNA

<213> Homo sapiens

<400> 45

gttcagaggc agaggggagt rgggaagtgg ggttcccatg g 41

<210> 46

<211> 41

<212> DNA

<213> Homo sapiens

<400> 46

agaaagatga ggtgaaagga rggagaaaat agggaggagg a 41

<210> 47

<211> 41

<212> DNA

<213> Homo sapiens

<400> 47
agaaaagcaa actgggccag rtagtgtcaa agacctttag g 41

<210> 48
<211> 41
<212> DNA
<213> Homo sapiens

<400> 48
tcaaagacct ttaggccaac rgagggcagc cagggagatg g 41

<210> 49
<211> 41
<212> DNA
<213> Homo sapiens

<400> 49
ccagagggca ggtactatcc ycaacttgag aaaaacaacg a 41

<210> 50
<211> 41
<212> DNA
<213> Homo sapiens

<400> 50

tacccccaac ataccagatc ygcttcctgc cccgctgaag g 41

<210> 51
<211> 41
<212> DNA
<213> Homo sapiens

<400> 51
tctcacccca ccaaagccaa sgcttcaatt tcagtctgtg g 41

<210> 52
<211> 41
<212> DNA
<213> Homo sapiens

<400> 52
cccacacagg acaacagggt kcaggggtct ggacagctgt t 41

<210> 53
<211> 41
<212> DNA
<213> Homo sapiens

<400> 53
acaggggtga gtccgcgtcc ytggcacgga gcgggggtg c 41

<210> 54

<211> 41

<212> DNA

<213> Homo sapiens

<400> 54

ttcccctcct gttcaaccgc mggggtacag gtggcttcgt c 41

<210> 55

<211> 41

<212> DNA

<213> Homo sapiens

<400> 55

ttcagggctt tgctcagctg yagctggtga cctccagaga g 41

<210> 56

<211> 41

<212> DNA

<213> Homo sapiens

<400> 56

ggggtggatg tcctgagacc rggaaggggg aagagaccca c 41

<210> 57

<211> 41

<212> DNA

<213> Homo sapiens

<400> 57

tgagctgata aagaacgccg kcagcacaga gcagacgctg g 41

<210> 58

<211> 41

<212> DNA

<213> Homo sapiens

<400> 58

cttttcaaga atgttgtcga yagataggaa agaggtggga g 41

<210> 59

<211> 41

<212> DNA

<213> Homo sapiens

<400> 59

ggggccccac gcacccccctt kcctaacgtc aggatgtgta t 41

<210> 60

<211> 41

<212> DNA

<213> Homo sapiens

<400> 60

gacagagaag ataatgtccc rgttcccccc aagtaagacc t 41

<210> 61

<211> 41

<212> DNA

<213> Homo sapiens

<400> 61

ctacagctgc cctggaccct ygttccttcc acagggctcc t 41

<210> 62

<211> 41

<212> DNA

<213> Homo sapiens

<400> 62

gaggtgatgt gagggcaccc rcatgcaaac aggccagtca g 41

<210> 63

<211> 41

<212> DNA

<213> Homo sapiens

<400> 63

agttcacagc ctgagtggtg ygtgccgccc tcctcctgaa g 41

<210> 64

<211> 41

<212> DNA

<213> Homo sapiens

<400> 64

ctttggcagg ggtcactgag kggaagggct ggccccactc c 41

<210> 65

<211> 41

<212> DNA

<213> Homo sapiens

<220>

<221> variation

<222> 21

<223> n represents tgt or deletion

<400> 65

tcctgggaat gaagtgttgt naggtggatt tttttttcc c 41

<210> 66

<211> 41

<212> DNA

<213> Homo sapiens

<220>

<221> variation

<222> 21

<223> n represents 9-11 repeats of t

<400> 66

gaagtgttgt tgtaggtgga ncccaaagac tagacatttt a 41

<210> 67

<211> 41

<212> DNA

<213> Homo sapiens

<400> 67

agttgacagc tttcccaaaa yggttcacgg agacttcatt t 41

<210> 68

<211> 41

<212> DNA

<213> Homo sapiens

<400> 68

gacagctttc ccaaaacggt kcacggagac ttcatttgac t 41

<210> 69

<211> 41

<212> DNA

<213> Homo sapiens

<400> 69

caaaccaaac atcctctaag ktctactgtg cagagtatag c 41

<210> 70

<211> 41

<212> DNA

<213> Homo sapiens

<400> 70

ttcccgacat caggcggcgg yggtggtccg ggagaaaccc g 41

<210> 71

<211> 41

<212> DNA

<213> Homo sapiens

<220>

<221> variation

<222> 21

<223> n represents agg or deletion

<400> 71

ccgcgcggag cgcctgggag nagaaggagc cgacctgccg a 41

<210> 72

<211> 41

<212> DNA

<213> Homo sapiens

<400> 72

tgggtggagc tcagttggga sttcctggga ctggggagga a 41

<210> 73

<211> 41

<212> DNA

<213> Homo sapiens

<220>

<221> variation

<222> 21

<223> n represents 10-11 repeats of a

<400> 73

acagagcgag attccgtctc ngaaagaaag gaagaagggg g 41

<210> 74

<211> 41

<212> DNA

<213> Homo sapiens

<400> 74

ctcctggttc ctgcggcccc ygccaggccc ccacaagcag c 41

<210> 75

<211> 41

<212> DNA

<213> Homo sapiens

<220>

<221> variation

<222> 21

<223> n represents tctc or deletion

<400> 75

catttgtgcc gggctgtctg ntccagcatc tctcctcttt c 41

<210> 76

<211> 41

<212> DNA

<213> Homo sapiens

<220>

<221> variation

<222> 21

<223> n represents 11-12 repeats of a

<400> 76

acagagcgag acactgtttc ngtgagaatt ctagagggaa g 41

<210> 77

<211> 41

<212> DNA

<213> Homo sapiens

<400> 77

gcgagacact gtttcaaaaa raaaaaagtg agaattctag a 41

<210> 78

<211> 41

<212> DNA

<213> Homo sapiens

<220>
<221> variation
<222> 21
<223> n represents 7-8 repeats of tc

<400> 78
gaggaatact gactcagccc nctcaccagg gaagcgtgtc t 41

<210> 79
<211> 41
<212> DNA
<213> Homo sapiens

<400> 79
ctctcccagc ctgtgaccac mgatgtccac acacccccaa c 41

<210> 80
<211> 41
<212> DNA
<213> Homo sapiens

<220>
<221> variation
<222> 21
<223> n represents 10-13 repeats of t

<400> 80

tttcttttta actggtttaa ngaacttttt tatctcattg t 41

<210> 81
<211> 41
<212> DNA
<213> Homo sapiens

<400> 81
tccacaacca tcagccccgc yccaggactc actgtgggtc t 41

<210> 82
<211> 41
<212> DNA
<213> Homo sapiens

<400> 82
ccttcagcgg gccccctgtc rctctctccc ccacaaaccc t 41

<210> 83
<211> 41
<212> DNA
<213> Homo sapiens

<400> 83
caaaggcact gaggagcccc stctcagcat tgcttttatc c 41

<210> 84
<211> 41
<212> DNA
<213> Homo sapiens

<400> 84
aactgctccc ttctagtggt raaggggaaa gcagtttcat g 41

<210> 85
<211> 41
<212> DNA
<213> Homo sapiens
<220>
<221> variation
<222> 21
<223> n represents 11-13 repeats of t

<400> 85
gagggataca tctatttccc natctttttg tatttttgtc t 41

<210> 86
<211> 41
<212> DNA
<213> Homo sapiens

<400> 86
atgattgaat gaactcttcc yctaacccta gtgccataat a 41

<210> 87

<211> 41

<212> DNA

<213> Homo sapiens

<400> 87

gaaattgaag agcaagactg yaaaaggagg ggatgatgga a 41

<210> 88

<211> 41

<212> DNA

<213> Homo sapiens

<400> 88

catccgtaca cagagagtga scaaaattgg tagcatcatc t 41

<210> 89

<211> 41

<212> DNA

<213> Homo sapiens

<400> 89

acagtgtagg gacagacaca yctctttccc tgggtgtcct t 41

<210> 90

<211> 41

<212> DNA

<213> Homo sapiens

<400> 90

cctaatgtta accctccaca ktctggccag tcttgactcc c 41

<210> 91

<211> 41

<212> DNA

<213> Homo sapiens

<400> 91

taagctgaag tgtcttcggg rgtgttgggg atggtcacca a 41

<210> 92

<211> 41

<212> DNA

<213> Homo sapiens

<400> 92

ggatggtcac caatggtgtg rtggggacag agccatagtg c 41

<210> 93

<211> 41

<212> DNA

<213> Homo sapiens

<400> 93

tcagtgtagg ggccaggttg stcctgggcc gaacctgggg a 41

<210> 94
<211> 41
<212> DNA
<213> Homo sapiens

<400> 94
ttgcaatcca gaatcagcca yaaaatccca tttccttttt t 41

<210> 95
<211> 41
<212> DNA
<213> Homo sapiens

<400> 95
aaatactcac tattataaat ytgtaaatga acagtctcgt t 41

<210> 96
<211> 41
<212> DNA
<213> Homo sapiens

<400> 96
gggaggccaa ggcaggagga ycgcttgagg ccaggagtta a 41

<210> 97

<211> 41

<212> DNA

<213> Homo sapiens

<400> 97

gctgatctct gcgtgatgct waacaaccag tttgactcat t 41

<210> 98

<211> 41

<212> DNA

<213> Homo sapiens

<400> 98

tggcaagaat ccatctctta ycactatcaa tatgtattga g 41

<210> 99

<211> 41

<212> DNA

<213> Homo sapiens

<400> 99

agctattagt cgtggtggta ygtgataaaa aactctcctg a 41

<210> 100

<211> 41

<212> DNA

<213> Homo sapiens

<220>

<221> variation

<222> 21

<223> n represents a or deletion

<400> 100

gtcccatact gaaaaaaaaa ntcaatttag caaataaata a 41

<210> 101

<211> 41

<212> DNA

<213> Homo sapiens

<220>

<221> variation

<222> 21

<223> n represents tg or deletion

<400> 101

tggctgagga tagggtaggg ngaaactatt gctgatttgt c 41

<210> 102

<211> 41

<212> DNA

<213> Homo sapiens

<400> 102

actttaggtt tttgctgctg rtaaaagtgc aagcctcagt a 41

<210> 103
<211> 41
<212> DNA
<213> Homo sapiens

<400> 103
tccctcttct ttgggtagaa rcatccaaca gggcatgata g 41

<210> 104
<211> 41
<212> DNA
<213> Homo sapiens

<400> 104
tggagaggca ccgagtctca ygatgtgtgg ctggaggttg t 41

<210> 105
<211> 41
<212> DNA
<213> Homo sapiens

<220>
<221> variation
<222> 21
<223> n represents 12-25 repeats of t

<400> 105

aggcactctt tgcagatgaa nggcctcggc ttaaagagga a 41

<210> 106
<211> 41
<212> DNA
<213> Homo sapiens

<400> 106
tccctggctt gtgccacctc yatcttttcc gtggctaatg t 41

<210> 107
<211> 41
<212> DNA
<213> Homo sapiens

<400> 107
tactgacttc tgcttccttg ytccctgggt ctacactgtt c 41

<210> 108
<211> 41
<212> DNA
<213> Homo sapiens

<400> 108
acaaccttgg cagtggcaga ygagagagaa cctcaggact g 41

<210> 109
<211> 41

<212> DNA

<213> Homo sapiens

<400> 109

caatagagta gggaggttta wcattacagg tttctttcag a 41

<210> 110

<211> 41

<212> DNA

<213> Homo sapiens

<400> 110

actaccgtgc caccacccac rcaccacacc attgctgcag a 41

<210> 111

<211> 41

<212> DNA

<213> Homo sapiens

<400> 111

acaaggagcg aagggtccat raagccttca caaagagggg c 41

<210> 112

<211> 41

<212> DNA

<213> Homo sapiens

<220>

<221> variation

<222> 21

<223> n represents 10-12 repeats of t

<400> 112

gtgacaaatt ctccctctcc ncattgcaga agtcacatgc g 41

<210> 113

<211> 41

<212> DNA

<213> Homo sapiens

<400> 113

aggacagtgc cccataattt yctgctccag atctatgccc t 41

<210> 114

<211> 41

<212> DNA

<213> Homo sapiens

<400> 114

tctacttaag ttcccagaag rcggctgtga actattttgg t 41

<210> 115

<211> 41

<212> DNA

<213> Homo sapiens

<220>

<221> variation

<222> 21

<223> n represents 12-16 repeats of a

<400> 115

agcattagca atattactac ngaagcagga agactcccaa g 41

<210> 116

<211> 41

<212> DNA

<213> Homo sapiens

<220>

<221> variation

<222> 21

<223> n represents gag or deletion

<400> 116

tttccatagt taatgagaag naagaattta aaacaattaa t 41

<210> 117

<211> 41

<212> DNA

<213> Homo sapiens

<400> 117

attttcgtcc tcttccactc rgcacaggat taagccttca g 41

<210> 118

<211> 41

<212> DNA

<213> Homo sapiens

<400> 118

gggggtgatg caacagggag ytccctgaca tacaacaaca t 41

<210> 119

<211> 41

<212> DNA

<213> Homo sapiens

<400> 119

tgaggagaga ggggcccttg cgcagacttg ccataaaacc g 41

<210> 120

<211> 40

<212> DNA

<213> Homo sapiens

<400> 120

tgaggagaga ggggcccttg gcagacttgc cataaaaccg 40

<210> 121

<211> 41

<212> DNA

<213> Homo sapiens

<400> 121

tgtttttagc caccacatgc ycctgagctg acccctcgag c 41

<210> 122

<211> 41

<212> DNA

<213> Homo sapiens

<220>

<221> variation

<222> 21

<223> n represents 9-10 repeats of t

<400> 122

tactgttttc atttttggta nacattttaa tttttttata c 41

<210> 123

<211> 41

<212> DNA

<213> Homo sapiens

<400> 123

ggttcaaatc tttgtacagc yagatagcaa tagatataaa t 41

<210> 124

<211> 41

<212> DNA

<213> Homo sapiens

<400> 124

tacagccaga tagcaataga yataaattga caatggcctc a 41

<210> 125

<211> 41

<212> DNA

<213> Homo sapiens

<400> 125

ccagtcaaca gggcttccct yaggagatgt tctctaaact g 41

<210> 126

<211> 41

<212> DNA

<213> Homo sapiens

<220>

<221> variation

<222> 21

<223> n represents ″ttggtcccccaaccgcctgtctccccacaccccgcaa″ or ″aggctgcag tcc″

<400> 126

acatgtggtt ctgagactgc ncctgcaggc tggggacact c 41

<210> 127
<211> 41
<212> DNA
<213> Homo sapiens

<400> 127
cctgctcagt agggggactg magaacacac agaagccatt g 41

<210> 128
<211> 41
<212> DNA
<213> Homo sapiens

<400> 128
tcaagtcggt agccgacagc rttctgtttt tacgtgacaa a 41

<210> 129
<211> 41
<212> DNA
<213> Homo sapiens

<220>
<221> variation
<222> 21
<223> n represents 9-12 repeats of t

<400> 129

ttgtatttaa attaaaattg nggcctatgt cataccaggg a 41

<210> 130

<211> 41

<212> DNA

<213> Homo sapiens

<400> 130

aaattgtttt tttttttgg tcctatgtca taccagggat a 41

<210> 131

<211> 40

<212> DNA

<213> Homo sapiens

<400> 131

aaattgtttt tttttttgg cctatgtcat accagggata 40

<210> 132

<211> 41

<212> DNA

<213> Homo sapiens

<400> 132

ggcattaatt ttttcatttt waaaaaaaac ccagaaatac g 41

<210> 133

<211> 41

<212> DNA

<213> Homo sapiens

<400> 133

ttttcactct agcagggaaa ygtgtaatca gttgtagaaa c 41

<210> 134

<211> 41

<212> DNA

<213> Homo sapiens

<400> 134

gggcaggtgg ctcagttgtg ycatcccttc tatttctcct c 41

<210> 135

<211> 41

<212> DNA

<213> Homo sapiens

<400> 135

attaattgca cagatgttta ytgagtgctt actatgtgcc a 41

<210> 136

<211> 41

<212> DNA

<213> Homo sapiens

<400> 136

gggagggcag gactccaaaa mggctccgat tccaggcccg c 41

<210> 137

<211> 41

<212> DNA

<213> Homo sapiens

<220>

<221> variation

<222> 21

<223> n represents 12-13 repeats of ac

<400> 137

gccacctttt gagagtgttc ngcatcccta gcctggctgt g 41

<210> 138

<211> 41

<212> DNA

<213> Homo sapiens

<400> 138

tgaaataggg tcattgcttg scttctcctg ctctccgggt t 41

<210> 139
<211> 41
<212> DNA
<213> Homo sapiens

<400> 139
caataattca gattctctac ygttagcagt tctaaacccc t 41

<210> 140
<211> 41
<212> DNA
<213> Homo sapiens
<400> 140
catctctcat cccatacagc yttgactgca ttctcctttc a 41

<210> 141
<211> 41
<212> DNA
<213> Homo sapiens

<220>
<221> variation
<222> 21
<223> n represents 9-10 repeats of a

<400> 141
caaatctatc ctcactatgg ntgtcttccc atgtggatct c 41

<210> 142
<211> 41
<212> DNA
<213> Homo sapiens

<400> 142
ttcagtcccc cagccacaca aggggtcaga ggtgctgctg a 41

<210> 143
<211> 40
<212> DNA
<213> Homo sapiens

<400> 143
ttcagtcccc cagccacaca ggggtcagag gtgctgctga 40

<210> 144
<211> 41
<212> DNA
<213> Homo sapiens

<400> 144
accctatctt ggaagttaag wtttgtggtc actgctacca a 41

<210> 145
<211> 41

<212> DNA

<213> Homo sapiens

<400> 145

gcccatgccc acagtaaccc ktagggaaac tgacccgctc t 41

<210> 146

<211> 41

<212> DNA

<213> Homo sapiens

<220>

<221> variation

<222> 21

<223> n represents 13-16 repeats of t

<400> 146

ggtttgtttg gtgagctttc naggttgtat tttataactg a 41

<210> 147

<211> 41

<212> DNA

<213> Homo sapiens

<220>

<221> variation

<222> 21

<223> n represents c or deletion

<400> 147

accaagtgga tcaatggtgg ntctggcatt tctataaagg g 41

<210> 148

<211> 41

<212> DNA

<213> Homo sapiens

<220>

<221> variation

<222> 21

<223> n represents 9-10 repeats of t

<400> 148

catgtaacat tgggttttgc ncctcacgta aaatccacat a 41

<210> 149

<211> 41

<212> DNA

<213> Homo sapiens

<220>

<221> variation

<222> 21

<223> n represents 10-12 repeats of a

<400> 149

caagcagtat tccacagatg nttataaaag cgaaaccaaa a 41

<210> 150

<211> 41

<212> DNA

<213> Homo sapiens

<400> 150

aaatggaaca aaaagtttta kgaaacatgt caccttctat g 41

<210> 151

<211> 41

<212> DNA

<213> Homo sapiens

<400> 151

tacaaacgag gaaatgtatc rggttcctta agagcctttc c 41

<210> 152

<211> 41

<212> DNA

<213> Homo sapiens

<220>

<221> variation

<222> 21

<223> n represents 8-9 repeats of t

<400> 152

agccgcccac tccgcacaca nctagttgtc ataaacaaat t 41

<210> 153
<211> 41
<212> DNA
<213> Homo sapiens

<400> 153
tgtccctctg gcttctgcag mttttcctga tgtggcaggg t 41

<210> 154
<211> 41
<212> DNA
<213> Homo sapiens

<400> 154
tgtccctcta gagggtgaca ygccttgaat ggcagcgaac c 41

<210> 155
<211> 41
<212> DNA
<213> Homo sapiens

<400> 155
ccccatagtg tgtggaccat ygggggccta gaactgaggt c 41

<210> 156
<211> 41
<212> DNA
<213> Homo sapiens
<400> 156
ggtttaactg cattttactc kttccagcct gtctggccat t 41

<210> 157
<211> 41
<212> DNA
<213> Homo sapiens

<400> 157
ttgcagtcca gcccaaagag rcctgtccca gcaggacgcc a 41

<210> 158
<211> 41
<212> DNA
<213> Homo sapiens

<400> 158
tgccaaactt ttaaactcag kggaattccg caaaaatcta g 41

<210> 159
<211> 41
<212> DNA
<213> Homo sapiens

<400> 159
gagaggcagg ttcacccagc ygtctgtgcc cgggcagggc a 41

<210> 160
<211> 41
<212> DNA
<213> Homo sapiens

<400> 160
ttcacccagc tgtctgtgcc ygggcagggc aactcccgaa g 41

<210> 161
<211> 41
<212> DNA
<213> Homo sapiens

<400> 161
ttggcctggg gacagttttt sagcgactgc tctttgagaa g 41

<210> 162
<211> 41
<212> DNA
<213> Homo sapiens

<400> 162
cacagagagc gtgcgctcca wtaaaacggc actaaatggc a 41

<210> 163

<211> 41

<212> DNA

<213> Homo sapiens

<400> 163

ccagggactg tgcatcaccc rtctccaccc caccccagaa c 41

<210> 164

<211> 41

<212> DNA

<213> Homo sapiens

<400> 164

tggaatggcc tttctctccc mcaacgccca gccaagggag g 41

<210> 165

<211> 41

<212> DNA

<213> Homo sapiens

<400> 165

tctgagccca gacaatggct yatttatttc agacaatatt c 41

<210> 166

<211> 41

<212> DNA

<213> Homo sapiens

<400> 166

ggccttatta ataaatccat yggtggtctg ctgaaaatgc t 41

<210> 167

<211> 41

<212> DNA

<213> Homo sapiens

<400> 167

tcctacagca aatcatcaac ragatgcaca cgaagatgag g 41

<210> 168

<211> 41

<212> DNA

<213> Homo sapiens

<400> 168

ccttttcaga tgctccataa mctcgtgtga aaagccccgg t 41

<210> 169

<211> 41

<212> DNA

<213> Homo sapiens

<400> 169

ggatctgtgg ggtgagtgaa yaaaagcatt ttctctgcaa a 41

<210> 170
<211> 41
<212> DNA
<213> Homo sapiens

<400> 170
gggttctgtc cccttctaaa rcaggtataa atagatgcca t 41

<210> 171
<211> 41
<212> DNA
<213> Homo sapiens

<400> 171
aggccaaggt caaggggagg sgctgcaggg ggggcttcct c 41

<210> 172
<211> 41
<212> DNA
<213> Homo sapiens
<400> 172
atcccagtgc gcccacaccc ygctcaagag ctcatgggca c 41

<210> 173

<211> 41

<212> DNA

<213> Homo sapiens

<400> 173

cccctgagtt ctgcagcctc raggaaggcc tgccctatct g 41

<210> 174

<211> 41

<212> DNA

<213> Homo sapiens

<400> 174

gctgacacaa cttggactta ytcataaaag aaaatcagaa t 41

<210> 175

<211> 41

<212> DNA

<213> Homo sapiens

<400> 175

ccacgtgctg cacaagttcc rgttccaagc ctgccctgag a 41

<210> 176

<211> 41

<212> DNA

<213> Homo sapiens

<400> 176

gaaacggggg tcctcaccca katcctgccc actcccaagc t 41

<210> 177
<211> 41
<212> DNA
<213> Homo sapiens

<400> 177
actcagctcc agaggaaacc saactcctcg agtaattctt t 41

<210> 178
<211> 41
<212> DNA
<213> Homo sapiens

<400> 178
tggggaggat cagcagggac rgctggagtc actgaaagat a 41
<210> 179
<211> 41
<212> DNA
<213> Homo sapiens

<400> 179
tcctgtggcc ctccccacac ycttgttgcc accacctgcc c 41

<210> 180
<211> 41

<212> DNA

<213> Homo sapiens

<400> 180

gaaacatgaa gcagcagaaa ygtttttccc agttctcttt c 41

<210> 181

<211> 41

<212> DNA

<213> Homo sapiens

<400> 181

gtcaacatat atttgagaga mcttcaactt atcaagtatt g 41

<210> 182

<211> 41

<212> DNA

<213> Homo sapiens

<220>

<221> variation

<222> 21

<223> n represents 9-10 repeats of t

<400> 182

tcaaggccag ttactaccac nctaatagaa tgaacaaatg g 41

<210> 183
<211> 41
<212> DNA
<213> Homo sapiens

<400> 183
ttctattact gtttttaaat maatgttaat caactgtggt g 41

<210> 184
<211> 41
<212> DNA
<213> Homo sapiens

<400> 184
gttcaagaat gcaagaaaaa yaaatacagt cagatccaga a 41
<210> 185
<211> 41
<212> DNA
<213> Homo sapiens

<400> 185
aagaatgcaa gaaaaacaaa yacagtcaga tccagaacca t 41

<210> 186
<211> 41
<212> DNA
<213> Homo sapiens

<400> 186
gaaaaatgat gcttagcaaa rtgataaaca ctagaatgta g 41

<210> 187
<211> 41
<212> DNA
<213> Homo sapiens

<400> 187
aaaacattat caattagttt rtgtgcaata gctgtaaata a 41

<210> 188
<211> 41
<212> DNA
<213> Homo sapiens

<400> 188
attagtttat gtgcaatagc ygtaaataag tgcagtagca t 41

<210> 189
<211> 41
<212> DNA
<213> Homo sapiens

<400> 189
catgttcagg actgcgcaca rtgcccggga gaaactggca g 41

<210> 190
<211> 41
<212> DNA
<213> Homo sapiens

<400> 190
tgaatgacct gccagtatta rgtgggtata cttgttatgt t 41

<210> 191
<211> 41
<212> DNA
<213> Homo sapiens

<220>
<221> variation
<222> 21
<223> n represents 8-9 repeats of t

<400> 191
catgataata aacctgccac ncttaaaaag cacctcctcc c 41

<210> 192
<211> 41
<212> DNA
<213> Homo sapiens

<400> 192

agtatttgct aaatcactaa yggggtagaa ttaaaaagaa a 41

<210> 193

<211> 41

<212> DNA

<213> Homo sapiens

<400> 193

ttaaaggcgg ttttctttgt rtttttattg cagactttta a 41

<210> 194

<211> 41

<212> DNA

<213> Homo sapiens

<400> 194

cttagtgaga tcccgtctcc raagaaaaga tatgtattct a 41

<210> 195

<211> 41

<212> DNA

<213> Homo sapiens

<400> 195

ccttattctt tctgagtaca rcctgtagta cttgaaccac t 41

<210> 196

<211> 41

<212> DNA

<213> Homo sapiens

<400> 196

aggtaagcgc ctcggccgcc wcgacgcatg cgccctgggc c 41

<210> 197

<211> 41

<212> DNA

<213> Homo sapiens

<220>

<221> variation

<222> 21

<223> n represents 9-12 repeats of t

<400> 197

ttttcttttg ttcttttatc nccttgtagg agacccggtg a 41

<210> 198

<211> 41

<212> DNA

<213> Homo sapiens

<220>

<221> variation

<222> 21

<223> n represents 10-11 repeats of t

<400> 198

gaactcatat tttctctctc nctaggaaag tacataacat t 41

<210> 199

<211> 41

<212> DNA

<213> Homo sapiens

<220>

<221> variation

<222> 21

<223> n represents 12-14 repeats of t

<400> 199

ataaccctct ataattgata ngctgaggtg gtaaaacaag a 41

<210> 200

<211> 41

<212> DNA

<213> Homo sapiens

<400> 200

gcatgaagta tatgtacaaa rgcaagactt acacagttaa a 41

<210> 201

<211> 41

<212> DNA

<213> Homo sapiens

<400> 201

aaaaagttta aacttttatc mttataagag gaaaaaagat t 41

<210> 202

<211> 41

<212> DNA

<213> Homo sapiens

<400> 202

tcttgttaag attctgctta ygacctcagt aaaacatttt a 41

<210> 203

<211> 41

<212> DNA

<213> Homo sapiens

<400> 203

tcagaaaaaa atctggggtg kaggtaggat ttagaactgc g 41

<210> 204

<211> 41

<212> DNA

<213> Homo sapiens

<220>

<221> variation

<222> 21

<223> n represents 16-19 repeats of a

<400> 204

atttataagt atatggaaag naattctaga acgaattgag a 41

<210> 205

<211> 41

<212> DNA

<213> Homo sapiens

<220>

<221> variation

<222> 21

<223> n represents 6-7 repeats of a

<400> 205

cctcatgtgt gagaaggggg ntgagggtct tgttcatttg t 41

<210> 206

<211> 41

<212> DNA

<213> Homo sapiens

<400> 206

ggaagtcctc ccagggccca kctggtctgt cagtggcact g 41

<210> 207

<211> 41

<212> DNA

<213> Homo sapiens

<400> 207

ccattgttga taataacgca rctcattact tttgtcgtct a 41

<210> 208

<211> 41

<212> DNA

<213> Homo sapiens

<400> 208

ggcatgatgg ctcagaacga sagccaacct catctcgcac a 41

<210> 209

<211> 41

<212> DNA

<213> Homo sapiens

<400> 209

aattgtcatt tgtgatactg yggtgggcgc aagctgccat g 41

<210> 210

<211> 41

<212> DNA

<213> Homo sapiens

<400> 210

ccatttgtta tcatgtgaag sgggagaaaa ctccatttgg g 41

<210> 211

<211> 41

<212> DNA

<213> Homo sapiens

<400> 211

aagctcactt agactgattt kggttaggta gatagaggac c 41

<210> 212

<211> 41

<212> DNA

<213> Homo sapiens

<400> 212

gactgatttg ggttaggtag rtagaggacc agtctcctca g 41

<210> 213

<211> 41

<212> DNA

<213> Homo sapiens

<400> 213

aaatgaggac aaacctaata sttttaaagg aaatggttca c 41

<210> 214

<211> 41

<212> DNA

<213> Homo sapiens

<400> 214

aagtcataca gcttttcaga yttcactttg atatgggaga a 41

<210> 215

<211> 41

<212> DNA

<213> Homo sapiens

<400> 215

atcaaaatta ccctaaacat yctaagctca tctatttctt t 41